# EUROPEAN PATENT APPLICATION

(11) **EP 4 603 145 A2**
(43) Date of publication of application: **20.08.2025**
(21) Application number: 25186722.2
(22) Date of filing: 21.11.2023
(51) Int. Cl.: A61P 25/04

(54) **TREATING PAIN ASSOCIATED WITH CENTRAL SENSITIZATION**

(30) Priority: 23.11.2022 US 202263384790 P; 23.11.2022 US 202263384792 P; 23.11.2022 US 202263384793 P; 23.11.2022 US 202263384794 P; 23.11.2022 US 202263384795 P; 23.11.2022 US 202263384796 P; 06.02.2023 US 202363483353 P
(62) Divisional of application: 23837005.0
(71) Applicant: Medicon Pharmaceuticals, Inc., Setauket, NY 11733 (US)
(72) Inventor: RIGAS, Basil, Setauket, 11733 (US)
(74) Representative: Carpmaels & Ransford LLP

(57) **Abstract**

The invention features methods of treating pain, for example pain associated with central sensitization, with phosphosulindac and compositions thereof.

## Description

This application claims the benefit of United States provisional applications 63/384790, 63/384792, 63/384793, 63/384794, 63/384795, 63/384796, all filed 23rd November 2022, and 63/483353, filed 6th February 2023. The complete contents of these provisionals are incorporated herein by reference for all purposes.

### FIELD OF THE INVENTION

The invention relates to compounds and their use in the treatment of pain associated with central sensitization. The invention also relates to compounds and their use in the treatment of neuropathic pain associated with post-traumatic peripheral neuropathy (PTPN), neuropathic pain associated with post-herpetic neuralgia (PHN), migraine pain, and corneal neuropathic pain.

### BACKGROUND OF THE INVENTION

According to recent studies, pain associated with central sensitization is relatively common, present in up to 1 in 5 patients with chronic pain from any cause. In fact, about 20 % of the adult patient population report widespread generalized pain associated with central sensitization.

Pain associated with central sensitization occurs when a subject's nervous system is persistently in a state of high activity, resulting in decreased thresholds for firing action potentials. Therefore, in this setting, even though the peripheral nervous system is providing limited input, the central nervous system responds as if there has been peripheral input (*i.e.,* the central nervous system is hyperexcitable). This state of hypersensitivity is known as "wind-up", and manifests as pain sensation in response to innocuous stimuli (allodynia) and over-exagerrated response to painful stimuli (hyperalgesia). As central sensitization results from changes in the properties of the neurons in the CNS (*i.e.,* central neuronal plasticity), the perception of pain is no longer coupled to the presence, intensity, or duration of a particular peripheral stimuli (noxious or otherwise). Accordingly, central sensitization is implicated in the generation and maintenance of pain in which the pain signalling is generated centrally (*i.e.,* due to the hypersensitivity of central pain signalling neurons), even absent a peripheral stimulus.

The dynamic changes in central neurons (*i.e.,* plasticity) that occurs in the development and maintenance of pain associated with central sensitization are considered a major contributor of many clinical pain syndromes. Pain associated with central sensitization, sometimes referred to as centralised pain or central pain has both genetic and environmental influences that predispose patients, and occurs in patients with, for example, fibromyalgia, chronic pain syndromes, as well as neurological injuries such as stroke or a spinal cord injury. Although the pain may be experienced as originating from the periphery, the pain generation occurs at central sites of action, resulting in the symptoms of allodynia and hyperalgesia.

Central sensitization may be a component of neuropathic pain associated with neuropathy. Neuropathies are diseases or abnormalities of the nervous system, which afflict more than 20 million in the USA alone. Indeed, according to recent studies, it is observed that neuropathic pain affects about 1 in every 10 adults and the economic burden for treating this pain is increasing.

Neuropathies are associated with the development of neuropathic pain. Neuropathic pain can occur as a result of damage to the peripheral or central nervous system. Peripheral neuropathic pain is caused by damage to nerve structures such as peripheral nerve endings or nociceptors which become extremely sensitive to stimulation and which can generate pulses in the absence of stimulation. The damage can occur for many reasons, such as a traumatic injury (such as nerve compression, spinal cord injury and nerve damage following surgery), chemotherapy treatments, diseases such as diabetes, as well as advanced-stage cancers, viruses (*e.g*., herpes zoster or HIV).

The lesion of the peripheral nerve can result in pathological states characterized by the presence of continuous spontaneous pain often associated with hyperalgesia (increased response to harmful stimuli) and allodynia (pain induced by a non-painful stimulus). Hyperalgesia and allodynia have been linked to central sensitization, in which CNS nociceptive neurons display increased excitability due to a reduced stimulation threshold, triggered by persistent input or peripheral injury. As noted herein, central sensitization is implicated in the generation and maintenance of neuropathic pain associated with peripheral neuropathies.

From a symptomatic perspective, pain associated with central sensitization may cause sharp pains, dull aches, a sensation of painful burning or cold, paraesthesia, a loss of proprioception, numbness, or even a loss of the sensation of pain. Similarly, peripheral neuropathies may cause sharp pains, dull aches, a sensation of painful burning or cold, paraesthesia, a loss of proprioception, numbness, or even a loss of the sensation of pain.

There is currently a worldwide need for additional pain therapy, and pain associated with central sensitization has developed into a major health problem in broad areas of the population. From a therapeutic perspective, pain associated with central sensitization often responds to neuromodulators, anti-epileptics, or antidepressants and is not responsive to nonsteroidal anti-inflammatory drugs (NSAIDs). Recommended therapies include tricyclic antidepressants (TCAs), such as amitriptyline, serotonin and norepinephrine reuptake inhibitors (SNRIs), such as duloxetine or venlafaxine, and anti-convulsants, such as pregabalin and gabapentin.

Neuropathic pain has also developed into a major health problem in broad areas of the population. Treatment of neuropathic pain is often attempted using so-called unconventional analgesics such as antidepressants like duloxetine and amitriptyline, or anti-epileptics like gabapentin or pregabalin. Additionally, topical anaesthetics, including lidocaine, have been used for the treatment and management of neuropathic pain.

Despite evidence to the contrary, NSAIDs are widely used in the management of neuropathic pain. However, NSAIDs have been shown to be ineffective in the treatment of neuropathic pain, which can be associated with central sensitization. Indeed, upon a review of recent clinical trials, there was no indication of any significant pain reduction with NSAIDs in neuropathic pain patients (Moore et al. (2015) Cochrane Database of Systematic Reviews 10:1-25), with no clinical outcome showing a statistically significant difference between NSAIDs and placebo. The Cochrane Library concluded that NSAIDs should not be recommended for the treatment of neuropathic pain. Therefore, the anti-inflammatory activity of typical NSAIDs fails to generate an analgesic effect, certainly when the pain is generated via central sensitization or is associated with peripheral neuropathies.

Therefore, there is a strong need for compounds that treat pain associated central sensitization. Furthermore, there is a strong need for compounds that treat and/or prevent pain associated with peripheral neuropathies, for example PTPN and PHN, as well as migraine pain.

### SUMMARY OF THE INVENTION

The inventor has surprisingly found that phosphosulindac (PS) is effective in the treatment of pain associated with central sensitization. Indeed, data from multiple distinct animal models generating central sensitization demonstrates an ability of PS to treat allodynia, a manifestation of central sensitization generated by amplification of signals in centrally located neurons in response to typically innocuous stimuli. The observation of treatment of allodynia in multiple distinct models, along with observations that PS is able to reach key sites of action by traversing peripheral neurons towards the CNS, support a role for PS acting directly on neuronal signalling implicated in central sensitization. Accordingly, the combination of observations herein provide a broadly applicable role for PS as an analgesic, with central effects, in the treatment of pain associated with central sensitization (*i.e.,* generated in central sites of action and manifested, for example, as allodynia).

PS is a non-steroidal compound with anti-inflammatory activity. However, unlike its parent compound (the NSAID sulindac) PS does not inhibit COX-1 and COX-2 or prostaglandin synthesis, and so is not a typical NSAID. PS has previously been shown to have anti-cancer and anti-inflammatory properties via its inhibition of activation of NF-κB and changes in MAPK signalling branches, as well as an activity in treating rheumatoid arthritis in inflammatory mouse models via suppression of key pro-inflammatory signalling pathways (Mackenzie et al. (2010) Gastroenterology 139(4): 1320-32 and Mattheolabakis et al. (2013) Pharm Res 30(6): 1471-82). WO 2019/067919 suggests an anti-inflammatory activity of PS in an acute model of dry eye disease (DED) and suggests PS decreases corneal sensitivity in an acute model. Observations of reduced sensitivity to an acute stimulus, which does not generate persistent pain, do not demonstrate efficacy in treating pain associated with central sensitization (*i.e.*, generated in central sites of action). Furthermore, in this acute model, the effect of PS was observed immediately, suggesting a local action of this atypical NSAID akin to the activity observed for typical NSAIDs (*e.g*. ketorolac) in the same model. Again, such peripheral activity does not demonstrate an efficacy of PS in treating pain generated at central sites of action. Indeed, clinical guidance in the field recommends avoiding the use of NSAIDs for the treatment of all types of

neuropathic pain, generally associated with central sensitization, and ketorolac has been shown to have limited analgesic activity in models of such pain. Furthermore, in the DED model, PS is seen to restore suppressed ocular sensitivity, suggesting a role of PS in increasing rather than reducing nociception. Therefore, these observations fail to suggest a role of PS in treating pain associated with central sensitization, and the observations herein demonstrate an unprecedented activity of PS in reducing pain generated at central sites of action.

The present inventor considered the activity of PS in specific animal models in which central sensitization, manifested as allodynia, has been established and demonstrated a surprising therapeutic efficacy, equivalent to direct acting nerve blocking anaesthetics that are able to reduce pain associated with central sensitization. Specific animal models are important during the development of therapies for treating pain associated with central sensitization. Indeed, given the pathogenesis of such pain, involving alterations in the sensitivity of centrally located neurons, observations of efficacy of a particular compound (for example, in an acute pain model) cannot indicate the utility of that compound in treating pain associated with central sensitization. Therefore, the efficacy of a compound in treating pain associated with central sensitization is demonstrated by observations indicating the ability of the compound to reverse manifestations of central sensitization (*e.g*., allodynia) in a model system in which chronic pain has been established. Accordingly, the animal models used in early testing before further clinical development are crucial. Based on the specific animal models of pain generated via sensitization of central neurons (*e.g*., allodynia), the observations herein demonstrate an unprecedented efficacy of PS in the treatment of pain associated with central sensitization.

Therefore, in a first aspect, the invention provides a method of treating pain associated with central sensitization comprising administering a therapeutically effective amount of PS to a subject in need thereof such that pain associated with central sensitization is treated. In some embodiments, the pain associated with central sensitization is not pain associated with central sensitization caused by chemotherapy-induced peripheral neuropathy (CIPN) or diabetic peripheral neuropathy (DPN).

In some embodiments, the PS is the sulfoxide form of PS. Therefore, the PS may have the formula I (PS-I):

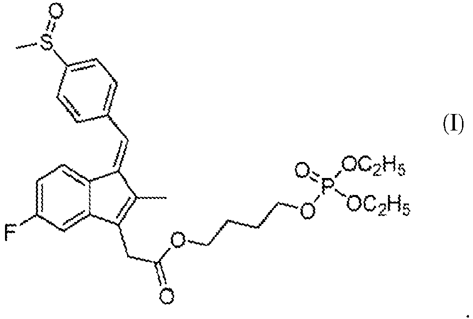

In other embodiments, the PS is the sulfide form of PS. Therefore, the PS may have the formula II (PS-II):

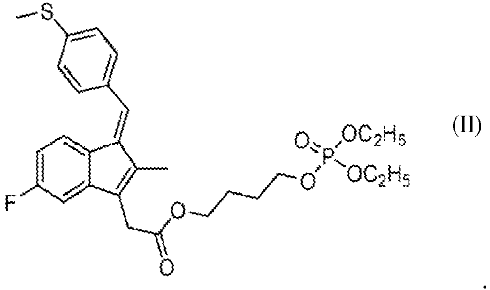

Herein, references to 'phosphosulindac' or to 'PS' encompass both PS-I and PS-II. The sulfoxide form of the compound is preferred. The compounds of formulae I & II are described in US patent 8,236,820, which is hereby incorporated by reference in its entirety.

As noted above, pain associated with central sensitization is generated as a result of over-activity of centrally located neurons, which display reduced stimulation thresholds and can depolarise even in the absence of a peripheral stimulus. Indeed, the perception of pain is no longer coupled to the presence, intensity, or duration of a particular peripheral stimulus (noxious or otherwise). Accordingly, subjects having pain associated with central sensitization, may experience pain induced by a non-painful stimulus (allodynia) or experience heightened pain in response to a harmful stimulus (hyperalgesia). On the basis of the observations herein, PS may have a direct analgesic effect, for example by reducing the neuronal signalling involved in the sensation of pain. Accordingly, PS may reduce pain signalling occurring centrally. The PS may reduce pain signalling occurring in the dorsal root ganglion. The PS may reduce pain signalling occurring in the spinal cord dorsal horn. Given that PS is shown to ascend peripheral neurons towards the spinal cord, PS may reduce pain signalling occurring in the CNS. In some embodiments, the pain associated with central sensitization is allodynia. The allodynia may be in response to mechanical and/or thermal stimuli. In addition, in some embodiments, the pain associated with central sensitization is hyperalgesia.

In some embodiments, the pain associated with central sensitization is pain associated with post-traumatic peripheral neuropathy. In some embodiments, the pain associated with central sensitization is pain associated with post-herpetic neuralgia. In some embodiments, the pain associated with central sensitization is migraine pain (or pain of other headache disorders). In some embodiments, the pain associated with central sensitization is corneal neuropathic pain.

Furthermore, *in vivo* evidence indicates efficacy of PS in the treatment of neuropathic pain associated with PTPN, migraine pain, neuropathic pain associated with PHN, and corneal neuropathic pain. Further experiments in specific animal models confirm these preliminary observations.

Accordingly, in another aspect, the invention provides a method of treating and/or preventing neuropathic pain associated with post-traumatic peripheral neuropathy (PTPN) comprising administering a therapeutically effective amount of PS to a subject in need thereof such that neuropathic pain associated with PTPN is treated and/or prevented.

In another aspect, the invention provides a method of treating and/or preventing neuropathic pain associated with post-herpetic neuralgia (PHN) comprising administering a therapeutically effective amount of PS to a subject in need thereof such that neuropathic pain associated with PHN is treated and/or prevented.

In another aspect, the invention provides a method of treating and/or preventing migraine pain comprising administering a therapeutically effective amount of PS to a subject in need thereof such that migraine pain is treated and/or prevented.

In a further aspect, the invention provides a method of treating and/or preventing pain comprising administering a therapeutically effective amount of PS to a subject in need thereof such that the pain is treated and/or prevented.

PS may be formulated into a pharmaceutical composition for use in the invention. In some embodiments, the pharmaceutical composition comprises PS and one or more pharmaceutically acceptable excipients. PS is particularly preferred as a formulation for topical administration. PS can be administered, for example topically, to an area in which the sensation of pain manifests. In some embodiments, PS is formulated for oral administration. Accordingly, PS can be administered orally. Therefore, in some embodiments, the invention provides a method of treating pain associated with central sensitization comprising administering a therapeutically effective amount of PS to a subject in need thereof such that pain associated with central sensitization is treated, wherein the PS is administered orally. In some embodiments, PS is administered both orally and topically.

### BRIEF DESCRIPTION OF THE FIGURES

**Figure 1** **-** schematic outline of the treatment study for pain associated with central sensitization.
**Figure 2** - effect of PS on treating pain associated with central sensitization.
**Figure 3** - effect of PS on treating pain associated with central sensitization caused by paclitaxel. The effect of PS compared to its vehicle control was significant from day 5 and increased thereafter (^{†}, p<0.002, ^{‡}, p=4.9x10⁻⁵, ^{&}, p=1.7x10⁻⁷, *, p=2.2x10⁻⁷).
**Figure 4** - effect of PS on treating pain associated with central sensitization caused by vincristine. The effect of PS compared to its vehicle control was significant on day 16 (* p=8.6x10⁻⁶).
**Figure 5** - effect of PS on treating pain associated with central sensitization caused by oxaliplatin. The effect of PS compared to its vehicle control was significant on day 22 (* p=0.004).
**Figure 6** - effect of PS on treating pain associated with central sensitization compared to sulindac, lidocaine and pregabalin. Figure 6A concerns mechanical allodynia (*, statistically significant differences; NS, not statistically significant). Figure 6B concerns cold allodynia (values: Mean ± SEM; *, p<0.0001).
**Figure 7** - schematic outline of the treatment study for pain associated with central sensitization. STZ is streptozotocin. PWT is paw withdrawal threshold test.
**Figure 8** - effect of PS on pain associated with central sensitization caused by STZ, compared to vehicle.
**Figure 9** - effect of sulindac, lidocaine, and pregabalin on pain associated with central sensitization.
Figure 10 - A) confirmation of efficacy of model system; B) effect of PS on treating pain associated with PTPN compared to sulindac and pregabalin. The effect of PS compared to its vehicle control was significant on day 14 (p < 0.005).
**Figure 11** **-** schematic outline of the metabolism of PS.
**Figure 12** - biodistribution of PS in different tissues upon topical administration. SN=sciatic nerve. DRG=dorsal root ganglia.
**Figure 13** - biodistribution of the metabolites of PS in different tissues upon topical administration of PS. SN=sciatic nerve. DRG=dorsal root ganglia.
**Figure 14- A** ) biodistribution of PS in ocular tissues upon topical administration of PS to the outer surface of the eyelid; B) levels of sulindac detected in ocular tissues as a result of PS hydrolysis upon administration to the outer surface of the eyelid.

### DETAILED DESCRIPTION OF THE INVENTION

### Definitions

The following definitions of types of pain are according to the International Association for the Study of Pain (IASP). "Pain" is an unpleasant sensory and emotional experience associated with, or resembling that associated with, actual or potential tissue damage. "Central sensitization" refers to increased responsiveness of nociceptive neurons in the central nervous system to their normal or subthreshold afferent input. "Peripheral sensitization" refers to increased responsiveness and reduced threshold of nociceptive neurons in the periphery to the stimulation of their receptive fields. "Allodynia" is pain due to a stimulus that does not normally provoke pain. "Hyperalgesia" is increased pain from a stimulus that normally provokes pain. Pain associated with central sensitization can be either generalized or in multiple locations in the body. Given the related nervous system involvement, pain associated with central sensitization displays symptoms corresponding to those observed with neuropathic pain. Accordingly, patients with pain associated with central sensitization may experience one or more sensations described as heat, burning, throbbing, shooting, stabbing, sharpness, cramping, aching, tingling, numbness, or pins and needles. Pain associated with central sensitization is also associated with mood changes, fatigue, cognitive disturbances, sleep changes, and pain catastrophizing. Additionally, patients with pain associated with central sensitization may have multifocal pain, memory complaints, and comorbidities including major depressive disorder or generalized anxiety disorder.

"Neuropathic pain" is caused by a lesion or disease of the somatosensory nervous system. Neuropathic pain is a clinical description (and not a diagnosis) which requires a demonstrable lesion or a disease that satisfies established neurological diagnostic criteria. Patients with neuropathic pain may experience one or more sensations described as heat, burning, throbbing, shooting, stabbing, sharpness, cramping, aching, tingling, numbness, or pins and needles. The term "lesion of the somatosensory nervous system" is commonly used when diagnostic investigations (*e.g*., imaging, neurophysiology, biopsies, lab tests) reveal an abnormality or when there was obvious trauma. The term "disease of the somatosensory nervous system" is commonly used when the underlying cause of the lesion is known (*e.g*., stroke, vasculitis, diabetes mellitus, genetic abnormality). "Peripheral neuropathic pain" is pain caused by a lesion or disease of the peripheral somatosensory nervous system. "Central neuropathic pain" is pain caused by a lesion or disease of the central somatosensory nervous system.

The following definitions of types of headache are according to the International Classification of Headache Disorders (ICHD) 3rd Edition (ICHD-3). Migraine has two major types: "migraine without aura", a clinical syndrome characterized by headache with specific features and associated symptoms; and "migraine with aura", primarily characterized by the transient focal neurological symptoms that usually precede or sometimes accompany the headache. "Migraine without aura" (*i.e*., common migraine; hemicrania simplex) is a recurrent headache disorder manifesting in attacks lasting 4-72 hours. The headache typically has a unilateral location, pulsating quality, moderate or severe intensity, aggravation by routine physical activity and association with nausea and/or photophobia and phonophobia. "Migraine with aura" (*i.e.,* classic or classical migraine) involves recurrent attacks, lasting miniutes, of unilateral fully reversible visual, sensory or other CNS symptoms that usually develop gradually and are usually followed by headache and associated migraine symptoms. "Episodic migraine" commonly involves about 1-2 migraine/headaches per month. "Chronic migraine" is a headache occuring on 15 or more days/month for more than three months, which, on at least 8 days/month, has features of migraine headache.

In general, the term "disease" refers to a state of being or health status of a patient or subject capable of being treated using the methods provided herein.

The term "therapeutically effective amount" refers to that amount of a compound or combination of compounds as described herein that is sufficient to effect the intended application. Accordingly, when the intended application is treating the disease, the "therapeutically effective

amount" refers to that amount of a compound or combination of compounds as described herein that is sufficient to treat the disease. When the intended application is treating and/or preventing the disease, the "therapeutically effective amount" refers to that amount of a compound or combination of compounds as described herein that is sufficient to treat and/or prevent the disease.

"Pharmaceutically acceptable excipient" is intended to include any and all solvents, dispersion media, coatings, antibacterial and antifungal agents, isotonic and absorption delaying agents, and inert ingredients included in pharmaceutical compositions. The use of such pharmaceutically acceptable excipients for formulating active pharmaceutical ingredients is well known in the art. Except insofar as any conventional pharmaceutically acceptable excipient is incompatible with PS, its use in the therapeutic compositions of the invention is contemplated.

Use of the term "about" when referring to a number is optional, and means that the number referred to is an approximation within typical experimental variability (or within statistical experimental error), and thus the number may vary accordingly.

The term "comprising" encompasses "including" as well as "consisting", *e.g.,* a composition "comprising" X may consist exclusively of X or may include something additional (*e.g.,* X + Y).

### Pain associated with central sensitization

As outlined above, pain associated with central sensitization occurs when centrally located neurons show a decreased sensitivity to fire action potentials (*i.e.,* the neurons are sensitized). This occurs in light of persistent nociceptive signalling from the periphery resulting in peripheral sensitization which ultimately leads to hyperexcitability of central neurons, manifested in the sensation of pain even in the absence of on-going peripheral input. Indeed, central sensitization is associated with spontaneous pain, but is typically manifested as allodynia (the sensation of pain induced by a non-painful stimulus) or hyperalgesia (a heightened sensation of pain in response to a harmful stimulus). Central sensitization is associated with chronic pain states in which the pain is generated or amplified by hyperexcitability of higher order neurons. The pain can manifest as a widespread or diffuse pain, sometimes localised in the vicinity of the site of the original trigger of nociception. In some embodiments, the invention provides a method of treating pain associated with central sensitization, comprising administering a therapeutically effective amount of PS to a subject in need thereof such that pain associated with central sensitization is treated. A subject experiencing pain associated with central sensitization would benefit from an analgesic which can both reduce the pain generated by central sensitization and prevent generation of further pain associated with central sensitization. Therefore, in some embodiments, PS can be used in the treatment and prevention of pain associated with central sensitization. In line with the above, the invention provides PS for use in the treatment of pain associated with central sensitization. Furthermore, the invention provides the use of PS for the manufacture of a medicament for the treatment of pain associated with central sensitization.

The pain associated with central sensitization is not acute nociceptive pain (*i.e.,* that relieves when the harmful stimulus is removed). Accordingly, in some embodiments, the pain associated with central sensitization is chronic pain (*i.e.,* pain that persists or recurs for more than three months). In particular embodiments, the pain is sensed in the absence of peripheral nociceptor input, for example to noxious or innocuous stimuli. The pathogenesis of the central sensitization may vary, depending on the initial pathology triggering peripheral input and contributing to the central sensitization. For example, the central sensitization may be as result of inflammatory pain mechanisms - that is to say, the initial trigger was an inflammatory response, but the central sensitization that is generated causes pain even in the absence of on-going inflammation. In particular instances, the central sensitization is a result of neuropathic pain mechanisms. Central sensitization is a feature of a number of chronic pain conditions. The pain associated with central sensitiation may be the pain associated with one or more of the following: inflammatory pain; neuropathic pain; fibromyalgia; chronic pain; chronic regional pain syndrome; rheumatoid arthritis; psoriatic arthritis; osteoarthritis; spondyloarthritis; lupus; temporomandibular disorders; and/or idiopathic low back pain. The pain associated with central sensitization resulting from inflammatory pain is not due to on-going peripheral inflammatory responses. In particular embodiments, the pain associated with central sensitization may be pain associated with one or more of the following: neuropathic pain; fibromyalgia; chronic pain; chronic regional pain syndrome; osteoarthritis; temporomandibular disorders; and/or idiopathic low back pain. In some embodiments, pain associated with central sensitization occurs following a stroke or spinal cord injury, or in subjects with multiple sclerosis. In some embodiments, the pain associated with central sensitization may be pain associated with post-traumatic peripheral neuropathy. In some embodiments, the pain associated with central sensitization may be pain associated with post-herpetic neuralgia. In some embodiments, the pain associated with central sensitization may be migraine pain. In some embodiments, the pain associated with central sensitization may be pain of other headache disorders. In some embodiments, the pain associated with central sensitization may be pain associated with corneal neuropathic pain.

On the basis of the observations herein, PS has a direct analgesic effect on pain associated with central sensitization. In treating pain associated with central sensitization, PS may reduce the pain. In some instances, the reduction is complete such that the pain is eliminated. In treating the pain associated with central sensitization, the PS may also reduce one or more of the symptoms associated with central sensitization. In treating and preventing pain associated with central sensitization, PS may decrease the incidence of the pain. In treating and preventing pain associated with central sensitization, the PS may also decrease the incidence of one or more of the symptoms associated with central sensitization.

The pain associated with central sensitization may be a diffuse pain. The pain may be widespread. In some embodiments, the pain may be diffuse around the region of initial injury. In some embodiments, the pain associated with central sensitization may have features of neuropathic pain, and therefore may result in one or more sensations described as heat, burning, throbbing, shooting, stabbing, sharpness, cramping, aching, tingling, numbness, or pins and needles. Patients suffering from pain associated with central sensitization may experience a range of symptoms. The symptoms include mood changes, fatigue, cognitive disturbances, sleep changes, pain catastrophizing, memory complaints, depression, anxiety, photophobia, and/or phonophobia. Even if the symptoms experienced by a subject experiencing pain associated with central sensitization are not considered painful (or do not reach a threshold necessary to be considered pain *per se*)*,* PS may reduce any one or more of the symptoms experienced by the subject. In some instances, the reduction is complete such that one or more of the symptoms experienced by the subject are eliminated. Indeed, the resolution of the underlying pain associated with central sensitization would remedy many of the symptoms associated therewith.

As noted above, the pain associated with central sensitization may be allodynia and/or hyperalgesia. In particular embodiments, the pain associated with central sensitization is allodynia (*e.g*., mechanical or thermal allodynia). PS may reduce the neuronal signalling involved in the sensation of pain generated via central sensitization. In some instances, the reduction may be complete such that the pain generation is eliminated. Thus, the PS may reduce pain signalling occurring centrally. The PS may reduce pain signalling occurring in the dorsal root ganglion. The PS may reduce pain signalling occurring in the spinal cord dorsal horn. Given that PS is shown to ascend peripheral neurons towards the spinal cord, PS may reduce pain signalling occurring in the CNS. In some instances, the reduction may be complete such that pain signalling is eliminated. The pain associated with central sensitization in a subject may be neuropathic pain. In some embodiments, the pain associated with central sensitization, for example neuropathic pain, is not pain associated with central sensitization caused by CIPN or DPN. In some embodiments, PS does not prevent the development of central sensitization.

Pain associated with central sensitization in a patient can be measured on a visual analogue pain scale or using any other appropriate method in the art.

### Pain associated with post-traumatic peripheral neuropathy

Post-traumatic peripheral neuropathy (PTPN) can arise from a range of traumatic peripheral nerve injuries and is associated with neuropathic pain that can cause mild discomfort to life-long impairment. Traumatic nerve injuries may be classified into categories (Seddon and Sunderland's Grade I-VI) based on the presence of demyelination and the extent of damage to the axons and the connective tissues of the nerve (see Menorca et al., Hand Clin. (2013); 29(3):317-330). The main classifications, in order of severity, are neurapraxia (Grade I), axonotmesis (encompassing Grades II-IV), and neurotmesis (Grade V). Grade VI (a later addition to the Seddon and Sunderland classification) involves different levels of damage (Grade III-V) along the nerve.

Of course, such peripheral nerve trauma can be associated with chronic neuropathic pain. The trauma may result in a tangle of neural fibers and connective tissue that develops following the nerve injury (a traumatic neuroma), with the area associated with paresthesia. The damaged nerve and any surrounding nerves may show altered gene expression that renders them hypersensitive, with development of spontaneous discharges. Therefore, the trauma results in painful hypersensitivity to non-noxious stimuli (allodynia) or an exaggerated pain response to noxious stimuli (hyperalgesia), reflective of central sensitization. Traumatic neuropathic pain can cause the patient to feel burning, stabbing, raw, gnawing or sickening sensations as well as numbness, and tingling and prickling sensations.

The neuropathic pain associated with PTPN is particularly difficult to treat. It is currently managed by secondary amine tricyclic antidepressants (e.g., nortriptyline, desipramine), calcium channel α-2-δ ligand anticonvulsants (e.g., pregabalin, gabapentin), opioids, ketamine, and topical lidocaine. In addition, procedures can be employed including nerve blocks, ablation, and neurostimulation, designed to interfere with, interrupt, or modulate pain pathways. Unfortunately, pain control is not very satisfactory and many of the systemic treatments induce major side effects leading to poor treatment adherence.

Therefore, there is a strong need for compounds that treat and/or prevent pain associated with peripheral neuropathies, in particular PTPN.

The inventor has surprisingly found that PS is effective in the treatment and prevention of pain associated with PTPN.

As noted herein, PS is a non-steroidal compound with anti-inflammatory activity. However, unlike its parent compound, the NSAID sulindac, PS does not inhibit COX-1 and COX-2 prostaglandin synthesis, and so is not a typical NSAID. PS has previously been shown to have anti-cancer and anti-inflammatory properties via its inhibition of activation of NF-κB and changes in MAPK signalling branches, as well as an activity in treating rheumatoid arthritis in inflammatory mouse models via suppression of key pro-inflammatory signalling pathways (Mackenzie et al. (2010) Gastroenterology 139(4): 1320-32 and Mattheolabakis et al. (2013) Pharm Res 30(6): 1471-82). WO 2019/067919 suggests an anti-inflammatory activity of PS in an acute model of dry eye disease (DED). Furthermore, in this model, PS is seen to restore suppressed ocular sensitivity in DED, suggesting a role of PS in increasing rather than reducing nociception. Although PS is not a typical NSAID as noted above, it demonstrated similar activity to NSAIDs when administered to normal eyes in the DED model. However, these observations fail to suggest a role for PS in the treatment of neuropathic pain associated with PTPN. Furthermore, clinical guidance in the field recommends avoiding the use of NSAIDs for the treatment of all types of neuropathic pain, and so anti-inflammatory activity alone is considered not sufficient therapeutically.

Nevertheless, preliminary in vivo evidence indicates the efficacy of PS in the treatment of neuropathic pain associated with PTPN. Further experiments in the specific animal model of neuropathic pain associated with PTPN confirm these preliminary observations.

Therefore, the invention provides a method of treating and/or preventing neuropathic pain associated with PTPN comprising administering a therapeutically effective amount of PS to a subject in need thereof such that neuropathic pain associated with PTPN is treated and/or prevented.

In some embodiments, the PS is the sulfoxide form of PS. Therefore, the PS may have the formula I (PS-I):

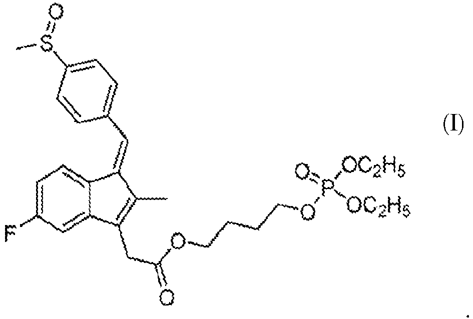

In other embodiments, the PS is the sulfide form of PS. Therefore, the PS may have the formula II (PS-II):

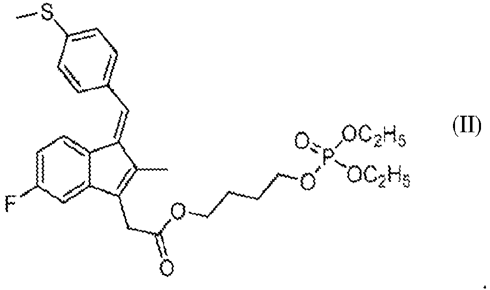

Herein, references to 'phosphosulindac' or to 'PS' encompass both PS-I and PS-II. The sulfoxide form of the compound is preferred. The compounds of formulae I and II are described in U.S. Patent No. 8,236,820, which is hereby incorporated by reference in its entirety.

The neuropathic pain associated with PTPN may be due to traumatic nerve injuries falling into any one or more of Seddon and Sunderland's classes (*i.e.,* Grade I-VI). For example, the traumatic nerve injury may be neurapraxia (Grade I, defined by focal demyelination (causing asynchronous conduction or even conduction block) without damage to the axons or the connective tissues). The neurapraxis may be caused by mild compression or traction of the nerve. The compression injury may occur in locations where nerves pass through narrow anatomical openings, for example those in the upper extremities including the carpal tunnel and cubital tunnel. Nerves may also be compressed by displaced fragments of fractures, disclocation of joints, or expanding hematoma.

The traumatic nerve injury may be axonotmesis (Grade II-IV, with increasing severity, involving axon damage with intact endoneurium (Grade II); involving axon and endoneurium damage with intact perineurium (Grade III); and involving axon, endoneurium, and perineurium damage with intact epineurium (Grade IV)). The axonotmesis may be caused by crush injuries that do not result in a complete transection of the nerve. Such crush injuries, with varying degrees of neural damage, may occur from an acute traumatic compression of the nerve from a blunt object, such as a bat, surgical clamp or other crushing object.

In some instances, the traumatic nerve injury may be neurotmesis (Grade V, defined by full transection of the axons and connective tissue layers wherein complete discontinuity of the nerve is observed). Injuries involving complete discontinuation of the nerve may occur due to a laceration from a knife, gunshot, glass shard or as a consequence of a car accident or surgical complication.

Finally, the traumatic nerve injury may be a combination of any one of these classes (Grade VI).

In some embodiments, the traumatic nerve injury is a compression injury. In particular embodiments, the traumatic nerve injury is a crush injury. In certain embodiments, the neuropathic pain associated with PTPN is pain caused by a nerve compression injury and/or a nerve crush injury. The nerve compression injury may be caused by accidents and trauma; joint sprains (*e.g.,* ankle, knee or wrist); arthritis; broken bones; bone spurs; dislocated joints (*e.g.,* elbow or shoulder); herniated disc; hypothyroidism; surgical complications; tumours and/or cysts.

The traumatic nerve injury may affect one or more of the following nerves: median nerve; radial nerve; suprascapular nerve; ulnar nerve; lateral femoral cutaneous nerve; peroneal nerve; pudendal nerve; sciatic nerve; tibial nerve; and/or the spinal nerve. The spinal nerve may be one or more of the following: cervical nerve; thoracic nerve; lumbar nerve; sacral nerve and/or coccygeal nerve. Accordingly, the neuropathic pain associated with PTPN may be pain caused by one or more of the following: carpal tunnel syndrome; pronator teres syndrome; radial tunnel syndrome; suprascapular nerve entrapment; thoracic outlet syndrome; ulnar nerve entrapment (cubital tunnel syndrome or Guyon's canal syndrome); meralgia paresthetica; peroneal nerve compression; pudendal nerve entrapment syndrome; sciatica; tarsal tunnel syndrome; herniated cervical disc; herniated thoracic disc; and/or herniated lumbar disc. In certain embodiments, the neuropathic pain associated with PTPN may be pain caused by a herniated spinal disc.

The neuropathic pain associated with PTPN may be caused by a herniated disc in the vertebrae of the spinal column. A herniated disc (prolapsed disc or slipped disc) occurs when the fibrous outer portion of the disc ruptures or tears, resulting in the disc bulging out of the spinal vertebrae. Such a herniated disc may result in the compression of nerves located between adjacent vertebrae or even of the spinal cord itself. This can cause pain, numbness, tingling or weakness in the arms or legs. Long-term compression of the disc can result in symptoms associated with neuropathic pain (*e.g*., allodynia and hyperalgesia). A bulging disc is less severe than a herniated disc but is also a cause of neuropathic pain associated with PTPN. The herniated (or bulging) disc may be a herniated (or bulging) cervical disc, for example causing pain in the neck, shoulders or arms. The herniated (or bulging) disc may be a herniated (or bulging) thoracic disc, for example causing pain in the mid-back around the level of the disc herniation (or bulge). The herniated (or bulging) disc may be a lumbar herniated (or bulging) disc, for example causing intermittent or continuous back pain and/or sciatica.

The neuropathic pain associated with post-traumatic peripheral neuropathy, for example due to traumatic injury to peripheral neurons, can arise immediately after injury and can manifest for example as burning, stabbing, raw, gnawing, sickening pain, poorly localized and sometimes diffuse, and is associated with other sensory symptoms. Accordingly, in some embodiments, the invention provides a method of treating neuropathic pain associated with PTPN comprising administering a therapeutically effective amount of PS to a subject in need thereof such that neuropathic pain associated with PTPN is treated. The pain can also arise with delayed onset after injury. Accordingly, the invention provides a method of preventing neuropathic pain associated with PTPN, comprising administering a therapeutically effective amount of PS to a subject in need thereof such that neuropathic pain associated with PTPN is prevented. Given that the pain can be experienced both immediately and with delayed onset, a subject may experience pain immediately on injury which develops into a different pain sensation occurring with delayed onset. Therefore, in some embodiments, PS can be used in the treatment and prevention of neuropathic pain associated with PTPN. In line with the above, the invention provides PS for use in the treatment and/or prevention of neuropathic pain associated with PTPN. Furthermore, the invention provides the use of PS for the manufacture of a medicament for the treatment and/or prevention of neuropathic pain associated with PTPN.

On the basis of the observations herein, PS has a direct analgesic effect on neuropathic pain associated with PTPN. The neuropathic pain associated with PTPN may be a stabbing or burning pain. In treating neuropathic pain associated with PTPN, PS may reduce the neuropathic pain. In some instances, the reduction may be complete such that the neuropathic pain associated with PTPN is eliminated. In treating the neuropathic pain associated with PTPN, the PS may also reduce one or more of the sensory symptoms associated with PTPN. In preventing neuropathic pain associated with PTPN, PS may decrease the incidence of the neuropathic pain. In preventing neuropathic pain associated with PTPN, the PS may also decrease the incidence of one or more of the sensory symptoms associated with PTPN.

Patients suffering from PTPN describe a range of sensory symptoms. The sensory symptoms include paresthesia (*e.g.*, numbness, tingling, pricking, and/or formication), burning sensations, or stabbing sensations. Even if the sensory symptoms experienced by a subject with traumatic nerve injury are not considered painful (or do not reach a threshold necessary to be considered pain *per se),* PS may reduce any one or more of the sensory symptoms experienced by the subject, including those listed above. In some instances, the reduction may be complete such that the one or more of the sensory symptoms associated with PTPN are eliminated.

As noted above, nerve damage associated with PTPN may result in over-activation of pain signalling pathways resulting in sensitization of peripheral and/or central neurons, which display reduced stimulation thresholds. Accordingly, subjects having PTPN may experience pain as a consequence of this sensitization, for example, experiencing pain induced by a non-painful stimulus (allodynia) or experiencing heightened pain in response to a harmful stimulus (hyperalgesia). On the basis of observations herein, PS may have a direct analgesic effect, for example by reducing the neuronal signalling involved in the sensation of pain. Accordingly, the neuropathic pain associated with PTPN may be a consequence of central sensitization resulting in allodynia and/or hyperalgesia. PS may reduce the neuronal signalling involved in the sensation of pain in a subject with traumatic peripheral nerve injury. The PS may reduce pain generated via peripheral sensitization or via central sensitization. In particular, given its ability to traverse towards key sites of pain generation, PS may reduce pain generated via central sensitization. In some instances, the reduction may be complete such that the pain generation is eliminated. Thus, the PS may reduce pain signalling occurring centrally. The PS may reduce pain signalling occurring in peripheral nerves. The PS may reduce pain signalling occurring in the dorsal root ganglion. The PS may reduce pain signalling occurring in the spinal cord dorsal horn. Given that PS is shown to ascend peripheral neurons towards the spinal cord, PS may reduce pain signalling occurring in the CNS. In some instances, the reduction may be complete such that pain signalling is eliminated. The neuropathic pain in a subject with PTPN may be allodynia (*e.g*., mechanical or thermal allodynia). Additionally or alternatively, the neuropathic pain in a subject with PTPN may be hyperalgesia.

Neuropathic pain in a patient can be measured on a visual analogue pain scale or using any other appropriate method in the art.

PS may be formulated into a pharmaceutical composition for use in the invention. In some embodiments, the pharmaceutical composition comprises PS and one or more pharmaceutically acceptable excipients. PS may be formulated for topical administration, in particular for topical administration in the vicinity of or to the region of trauma on the subject's body.

In particular embodiments of treating and/or preventing pain associated with PTPN, PS may be administered orally.

### Pain associated with post-herpetic neuralgia

PHN is a common complication of herpes zoster, which results from the reactivation of varicella zoster virus (VZV). VZV is a highly virulent neurotropic virus, which can cause varicella (chickenpox) as the primary infection in susceptible individuals. The virus can be retrogradely transported along axons of sensory neurons from the skin to establish a latent infection within sensory ganglia in the peripheral nervous system. In the even of the previously infected subject becoming immune suppressed, the virus can reactivate, presenting as acute herpes zoster (AHZ, "shingles"). Recovery from AHZ is frequently complicated by the development of post-herpetic neuralgia, a neuropathic pain syndrome characterized by persisting pain arising in areas affected by herpes zoster. PHN is typically defined as pain lasting 90 days or more after the initial presentation of the rash or at least three months after the healing of skin lesions. AHZ is diagnosed more than 1 million times a year in the US alone. Approximately 20% of patients with AHZ will experience PHN and will continue to suffer intermittent neuropathic symptoms, including itchiness and pain. The pain is characterized as sharp, stabbing, throbbing or burning, often localized to the site of the original rash. The long-pasting pain is associated with painful hypersensitivity to non-noxious stimuli (allodynia) or an exaggerated pain response to noxious stimuli (hyperalgesia), reflective of central sensitization. Without treatment, the incidence of pain persisting three months after the development of the rash is reported to be about 8-15%, a figure which increases rapidly in the elderly.

The pain associated with PHN is linked to peripheral and particularly central sensitization (Hadley et al., Curr Pain Headache Rep. (2016); 20:17). During reactivation of VZV, the virus replicates and spreads from the dorsal root ganglion to the periphery. The propagation of the virus causes nerve damage (*e.g.,* due to an immune reaction against the neurons) which results in more frequent depolarization of nociceptors. The reduction in the threshold for nociceptor signalling results in peripheral sensitization. On-going peripheral signalling results in central sensitization, characterized by a heightened state of activation of centrally located neurons (*e.g.* in the dorsal root horn and higher order neurons). Further pathological mechanisms resulting in altered gene expression in centrally located neurons, loss of co-inhibitory signalling or altered neuronal signalling networks (*e.g*., deafferentiation) all contribute to the hypersensitivity experienced in subjects with pain associated with PHN. The persistent pain, linked to central sensitization, manifests as_hyperalgesia (increased response to harmful stimuli) and/or allodynia (pain induced by a non-painful stimulus).

The neuropathic pain associated with PHN is particularly difficult to treat. Current treatments for neuropathic pain associated with PHN include systemic tricyclic antidepressants, anticonvulsants and opiods, as well as topical lidocaine and capsaicin. Additionally, there are interventional therapies including subcutaneous botulinum toxin injections, nerve blocks and nerve stimulations. However, these therapies are not aways effective. Indeed, even with the most effective drugs, only 30-50% of patients obtain more than 50% pain relief, often with significant side-effects. The neuropathic pain associated with PHN causes significant suffering and a financial burden, manifested in both healthcare costs and lost of quality-adjusted life years, and given the lack of efficacy of current treatments, pain associated with PHN represents an area of largely unmet medical need.

Therefore, there is a strong need for compounds that treat and/or prevent pain associated with peripheral neuropathies, in particular PHN.

The inventor has surprisingly found that PS is effective in the treatment and prevention of pain associated with PHN.

As noted herein, PS is a non-steroidal compound with anti-inflammatory activity. However, unlike its parent compound, the NSAID sulindac, PS does not inhibit COX-1 and COX-2 prostaglandin synthesis, and so is not a typical NSAID. PS has previously been shown to have anti-cancer and anti-inflammatory properties via its inhibition of activation of NF-κB and changes in MAPK signalling branches, as well as an activity in treating rheumatoid arthritis in inflammatory mouse models via suppression of key pro-inflammatory signalling pathways (Mackenzie et al. (2010) Gastroenterology 139(4): 1320-32 and Mattheolabakis et al. (2013) Pharm Res 30(6): 1471-82). WO 2019/067919 suggests an anti-inflammatory activity of PS in an acute model of dry eye disease (DED). Furthermore, in this model, PS is seen to restore suppressed ocular sensitivity in DED, suggesting a role of PS in increasing rather than reducing nociception. Although PS is not a typical NSAID as noted above, it demonstrated similar activity to NSAIDs when administered to normal eyes in the DED model. However, these observations fail to suggest a role for PS in the treatment of neuropathic pain associated with PHN. Furthermore, clinical guidance in the field recommends avoiding the use of NSAIDs for the treatment of all types of neuropathic pain, and so anti-inflammatory activity alone is considered not sufficient therapeutically. Indeed, Moore et al. (Cochrane Database of Systematic Reviews (2015); 10: 1-25) concluded that NSAIDs failed to achieve pain reduction in PHN.

Nevertheless, preliminary in vivo evidence indicates the efficacy of PS in the treatment of neuropathic pain associated with PHN.

Therefore, the invention provides a method of treating and/or preventing neuropathic pain associated with PHN comprising administering a therapeutically effective amount of PS to a subject in need thereof such that neuropathic pain associated with PHN is treated and/or prevented.

In some embodiments, the PS is the sulfoxide form of PS. Therefore, the PS may have the formula I (PS-I):

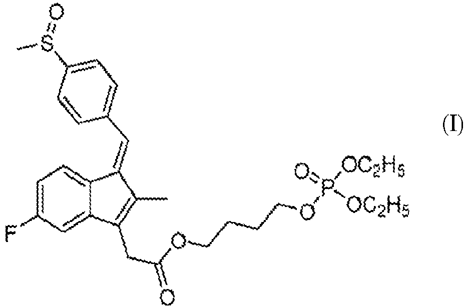

In other embodiments, the PS is the sulfide form of PS. Therefore, the PS may have the formula II (PS-II):

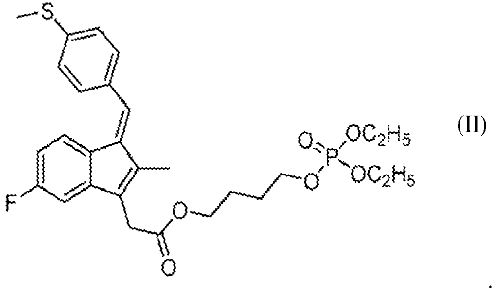

Herein, references to 'phosphosulindac' or to 'PS' encompass both PS-I and PS-II. The sulfoxide form of the compound is preferred. The compounds of formulae I and II are described in U.S. Patent No. 8,236,820, which is hereby incorporated by reference in its entirety.

As outlined above, reactivation of varicella zoster virus can cause acute herpes zoster, manifesting as a rash, and subsequently neuropathic pain associated with post-herpetic neuralgia (PHN). As noted above, neuropathic pain associated with PHN persists for 90 days or more after the initial presentation of the rash or at least three months after the resolution of skin lesions. The pain may manifest for example as sharp, burning, throbbing or stabbing pain. The neuropathic pain associated with PHN typically arises after herpes zoster reactivation and resolution of the rash, allowing the subject to take preventative measures to avoid generation of neuropathic pain after resolution of the rash and skin lesions . Accordingly, in some embodiments, the invention provides a method of preventing neuropathic pain associated with PHN comprising administering a therapeutically effective amount of PS to a subject in need thereof such that neuropathic pain associated with PHN is prevented. In other embodiments, the invention provides a method of treating neuropathic pain associated with PHN, comprising administering a therapeutically effective amount of PS to a subject in need thereof such that neuropathic pain associated with PHN is treated. As the pain associated with PHN develops during manifestation of the rash and persists after the rash has resolved, a subject would benefit from an analgesic which can both treat existing neuropathic pain and prevent generation of further neuropathic pain associated with PHN. Therefore, in some embodiments, PS can be used in the treatment and prevention of neuropathic pain associated with PHN. In line with the above, the invention provides PS for use in the treatment and/or prevention of neuropathic pain associated with PHN. Furthermore, the invention provides the use of PS for the manufacture of a medicament for the treatment and/or prevention of neuropathic pain associated with PHN.

As explained herein, the pain associated with PHN is typically localized to the site of the rash. The rash (*e.g.,* a maculopapular rash) may manifest in one or more adjacent dermatomes (an area of skin that is mainly supplied by a single spinal nerve). The spinal nerve may be a cervical nerve, a thoracic nerve, a lumbar nerve, and/or a sacral nerve. Accordingly, the neuropathic pain associated with PHN may be experienced in one or more adjacent dermatomes. The neuropathic pain associated with PHN may be experienced in one or more dermatomes, wherein each dermatome is supplied by a cervical nerve, a thoracic nerve, a lumbar nerve, or a sacral nerve. Typically, the neuropathic pain associated with PHN manifests on the subject's trunk, along a thoracic dermatome. In certain instances, for example in immune compromised individuals, the rash, and thus the associated neuropathic pain, may be more widespread, affecting three or more dermatomes (*i.e.,* due to disseminated zoster).

On the basis of the observations herein, PS has a direct analgesic effect on neuropathic pain associated with PHN. The neuropathic pain associated with PHN may be a sharp, throbbing, stabbing or burning pain. In treating neuropathic pain associated with PHN, PS may reduce the neuropathic pain. In some instances, the reduction is complete such that the neuropathic pain is eliminated. In treating the neuropathic pain associated with PHN, the PS may also reduce one or more of the sensory symptoms associated with PHN. In preventing neuropathic pain associated with PHN, PS may decrease the incidence of the neuropathic pain. In preventing neuropathic pain associated with PHN, the PS may also decrease the incidence of one or more of the sensory symptoms associated with PHN.

Patients suffering from PHN describe a range of sensory symptoms. The sensory symptoms include itching and numbness. Even if the sensory symptoms experienced by a subject with PHN are not considered painful (or do not reach a threshold necessary to be considered pain *per se*), PS may reduce any one or more of the sensory symptoms experienced by the subject. In some instances, the reduction is complete such that the one or more sensory symptoms experienced by the subject are eliminated.

As noted above, nerve damage associated with PHN may result in over-activation of pain signalling pathways resulting in sensitization of peripheral and/or central neurons, which display reduced stimulation thresholds. Accordingly, subjects having PHN may experience pain as a consequence of this sensitization, for example, experiencing pain induced by a non-painful stimulus (allodynia) or experiencing heightened pain in response to a harmful stimulus (hyperalgesia). On the basis of observations herein, PS may have a direct analgesic effect, for example by reducing the neuronal signalling involved in the sensation of pain. Accordingly, the neuropathic pain associated with PHN may be a consequence of central sensitization resulting in allodynia and/or hyperalgesia. PS may reduce the neuronal signalling involved in the sensation of pain in a subject with PHN. In some instances, the reduction may be complete such that the pain is eliminated. The PS may reduce pain generated via peripheral sensitization or via central sensitization. In some instances, the reduction may be complete such that the pain generation is eliminated. In particular, given its ability to traverse towards central sites of pain generation, the PS may reduce pain generated via central sensitization. Thus, the PS may reduce pain signalling occurring centrally. The PS may reduce pain signalling occurring in peripheral nerves, for example nerves innervating one or more dermatomes. The PS may reduce pain signalling occurring in one or more spinal nerves, for example one or more cervical nerves, one or more thoracic nerves, one or more lumbar nerves, and/or one or more sacral nerves. In particular, PS may reduce pain signalling occurring in one or more thoracic nerves. The PS may reduce pain signalling occurring in the dorsal root ganglion. The PS may reduce pain signalling occurring in the spinal cord dorsal horn. Given that PS is shown to ascend peripheral neurons towards the spinal cord, PS may reduce pain signalling occurring in the CNS. In some instances, the reduction may be complete such that pain signalling is eliminated. The neuropathic pain in a subject with PHN may be allodynia (*e.g*., mechanical or thermal allodynia). Additionally or alternatively, the neuropathic pain in a subject with PHN may be hyperalgesia.

Neuropathic pain in a patient can be measured on a visual analogue pain scale or using any other appropriate method in the art.

In another aspect, PS may also be useful for treating other herpes zoster associated pain, for example prodomal pain (in advance of the manifestation of the rash) or acute herpes zoster pain (coinciding with the manifestation of the rash). The damage incurred to peripheral neurons from the translocation of the virus causes increased signalling from centrally located neurons. This may drive neuronal sensitization in advance of the onset of persistent pain characteristic of neuropathic pain associated with PHN. Therefore, PS may be useful in treating pain at these stages of the pathology of the infection. The invention thus also provides a method of treating pain experienced by subjects as a result of herpes zoster comprising administering a therapeutically effective amount of PS to a subject in need thereof such that the pain is treated. The invention also provides a method of treating acute herpes zoster pain comprising administering a therapeutically effective amount of PS to a subject in need thereof such that the acute herpes zoster pain is treated. The invention also provides a method of treating herpes zoster prodromal pain comprising administering a therapeutically effective amount of PS to a subject in need thereof such that the herpes zoster prodromal pain is treated.

PS may be formulated into a pharmaceutical composition for use in the invention. In some embodiments, the pharmaceutical composition comprises PS and one or more pharmaceutically acceptable excipients. PS may be formulated for topical administration, in particular for topical administration to the area of skin (*e.g*., dermatome) affected by the rash, for example the subject's trunk.

In particular embodiments of treating and/or preventing pain associated with PHN, PS may be administered orally.

### Migraine pain

Migraine is a disabling neurological disorder affecting over 1 billion people worldwide, with a one-year prevalence of 15%. Its prevalence peaks in those aged 35-39, and it is the leading cause of disability in people younger than 50, and thus has a significant socioeconomic burden. Migraine is typically characterized by a recurrent unilateral, pulsating headache, with moderate to severe intensity, accompanied by nausea, vomiting, and sensory hypersensitivity symptoms.

The pathophysiology of migraine has been a matter of debate and is now classified as a neuronal disorder (Goadsby et al., Physiol Rev (2017); 97:553-622). Previous theories of pain generation via dilation of cranial arteries lost traction given the failure of effective therapies (*e.g*., sumatriptan) to reverse the slight dilation of these arteries observed during a migraine attack. Furthermore, the suggestion that migraine is triggered by so-called neurogenic inflammation (local dural release of endogenous inflammatory mediators) has also been rendered implausible, not least in light of the clinical failure of compounds designed to inhibit this process. Indeed, evidence of inflammatory pathology in migraineurs is lacking. Accordingly, migraine is considered a purely neuronal disorder resulting from changes or dysfunction in brain stem and hypothalamic regions, which contribute to changes in cellular and vascular function in many regions of the brain. These changes cause neurons to fail to normally modulate or gate sensory inputs. Dysfunction of these regions can lead to the perception of head pain through normal vessels throbbing, and continued dysfunction can lead to central sensitization of trigeminovascular neurons and the exacerbation of pain to normal physical activity as well as cutaneous allodynia. Indeed, peripheral and central sensitization of trigeminal neurons is considered a fundamental component of the pathophysiology, being clinically observed in migraine patients. This neuronal mechanism would explain the longevity of migraine attack, and the transition to chronic migraine as well as specific related symptoms (*e.g*., cutaneous allodynia).

Central sensitization occurs when the function of neuronal circuits in sensory pathways are enhanced or inappropriately modulated, and results in abnormal sensitivity, manifested as, for example, the presence of continuous spontaneous pain often associated with hyperalgesia (increased response to harmful stimuli) and allodynia (pain induced by a non-painful stimulus). As central sensitisation results from changes in the properties of the neurons in the CNS, the perception of pain is no longer coupled to the presence, intensity, or duration of a particular peripheral stimuli (noxious or otherwise). Accordingly, central sensitisation is implicated in the generation and maintenance of pain in which the pain signalling is generated centrally (*i.e.,* due to the hypersensitivity of central pain signalling neurons), even absent a peripheral stimulus.

As suggested above, sensitization in migraine is the pain that develops as a consequence of failure of integration and filtering of sensory signalling, ultimately resulting in the perception of activation of sensory systems under normal conditions, for example the cutaneous allodynia experienced by migraineurs. Such symptoms are experienced in both episodic and chronic migraine, although pain amplification is thought to be more involved in chronic migraine.

There is currently a worldwide need for additional pain therapy for the treatment of migraine and other headache disorders.

A variety of pharmacologic interventions have been postulated for treating migraine reflecting the diverse nature of this disorder. Indeed, relatively non-selective drugs such as ergot alkaloids have been used for decades. Other treatments include opiates (*e.g*., oxycodone), beta-blockers (*e.g.,* propranolol), anticonvulsants (*e.g.,* topiramate), or serotonin receptor agonists (*e.g*., sumatriptan). Patients with milder symptoms may be able to control those symptoms with non-steroidal anti-inflammatory agents (NSAIDs), although, as noted above, inflammatory responses are considered of limited relevance when considering migraine pathophysiology. Indeed, studies have demonstrated that particular NSAIDs, for example naproxen, are not clinically useful for treating migraine (see, for example, Law et al. Cochrane Database of Systematic Reviews (2013); 10: 1-45).

Therefore, there is a strong need for compounds that treat and/or prevent migraine pain and pain of other headache disorders, in particular pain associated with central sensitization.

The inventor has surprisingly found that PS is effective in the treatment and prevention of migraine pain.

As noted here, PS is a non-steroidal compound with anti-inflammatory activity. However, unlike its parent compound, the NSAID sulindac, PS does not inhibit COX-1 and COX-2 prostaglandin synthesis, and so is not a typical NSAID. PS has previously been shown to have anti-cancer and anti-inflammatory properties via its inhibition of activation of NF-κB and changes in MAPK signalling branches, as well as an activity in treating rheumatoid arthritis in inflammatory mouse models via suppression of key pro-inflammatory signalling pathways (Mackenzie et al. (2010) Gastroenterology 139(4): 1320-32 and Mattheolabakis et al. (2013) Pharm Res 30(6): 1471-82). WO 2019/067919 suggests an anti-inflammatory activity of PS in an acute model of dry eye disease (DED). Furthermore, in this model, PS is seen to restore suppressed ocular sensitivity in DED, suggesting a role of PS in increasing rather than reducing nociception. Although PS is not a typical NSAID as noted above, it demonstrated similar activity to NSAIDs when administered to normal eyes in the DED model. However, these observations fail to suggest a role for PS in the treatment of migraine pain, which manifests not as a consequence of inflammatory responses, but rather dysfunctional sensory neuronal signalling. Indeed, certain typical NSAIDs (*e.g*., naproxen) have been shown to be not clinically useful as analgesics for migraine and so anti-inflammatory activity alone is not sufficient to treat migraine pain.

Nevertheless, preliminary *in vivo* evidence indicates the efficacy of PS in the treatment of migraine pain. Further experiments in the specific animal model of migraine pain confirm these preliminary observations.

Therefore, the invention provides a method of treating and/or preventing migraine pain comprising administering a therapeutically effective amount of PS to a subject in need thereof such that migraine pain is treated and/or prevented.

In some embodiments, the PS is the sulfoxide form of PS. Therefore, the PS may have the formula I (PS-I):

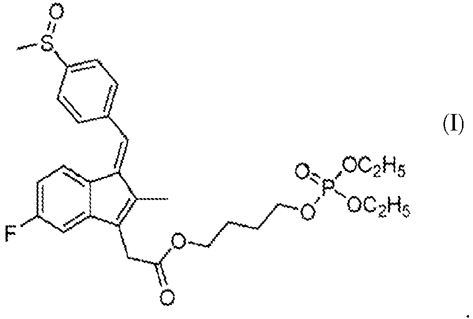

In other embodiments, the PS is the sulfide form of PS. Therefore, the PS may have the formula II (PS-II):

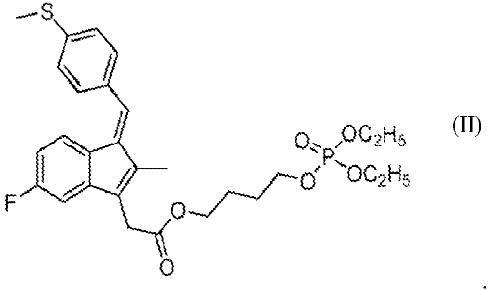

Herein, references to 'phosphosulindac' or to 'PS' encompass both PS-I and PS-II. The sulfoxide form of the compound is preferred. The compounds of formulae I and II are described in U.S. Patent No. 8,236,820, which is hereby incorporated by reference in its entirety.

As outlined above, migraine is characterized by moderate to severe attacks of unilateral pulsating head pain, associated with photophobia, phonophobia, nausea and/or vomiting. Accordingly, in some embodiments, the invention provides a method of treating migraine pain comprising administering a therapeutically effective amount of PS to a subject in need thereof such that migraine pain is treated. Typically, a migraine attack is comprised of three phases: a premonitory phase, the migraine headache itself, and a postdrome phase. The premonitory phase occurs about 24-48 hours prior to the headache phase and is typically characterized by symptoms such as mood alterations, fatigue and neck discomfort, and in some individuals experiences of aura, a transient focal neurological symptom of visual, sensory or motor disturbances. A subject within the premonitory phase, and in advance of the onset of the headache phase, may be able to administer a therapeutic that prevents the onset of the headache phase. Accordingly, in other embodiments, the invention provides a method of preventing migraine pain, comprising administering a therapeutically effective amount of PS to a subject in need thereof such that migraine pain is prevented. A subject may experience repeated migraine attacks, in particular if they have a short interictal period between attacks, and so administration of the therapeutic may treat the pain of an ongoing attack and prevent pain of subsequent attacks. Therefore, in some embodiments, PS can be used in the treatment and prevention of migraine pain. In line with the above, the invention provides PS for use in the treatment and/or prevention of migraine pain. Furthermore, the invention provides the use of PS for the manufacture of a medicament for the treatment and/or prevention of migraine pain.

In some embodiments, when treating and/or preventing migraine pain, the PS may be administered during the premonitory phase, the headache phase and/or the postdrome phase. In particular, for preventing migraine pain, PS may be administered during the premonitory phase. For treating migraine pain, PS may be administered during the headache phase.

The migraine may be episodic migraine. In certain instances, the migraine may be chronic migraine. The migraine may be migraine without aura or migraine with aura.

On the basis of the observations herein, PS has a direct analgesic effect on migraine pain. The migraine pain may be a pulsating head pain. A subject with migraine, in particular chronic migraine, may experience a persistent migraine headache and/or premonitory-like phase, with limited neurological recovery and baseline restoration between attacks. Subjects suffering from migraine may experience a number of associated symptoms, including aura, nausea, vomiting, photophobia and/or phonophobia. The sensory disturbances associated with aura may include visual symptoms, pins and needles (tingling), and/or numbness. Furthermore, subjects may experience cranial autonomic symptoms such as eye redness or tearing. Additionally, subjects may experience cutaneous allodynia.

The migraine with aura may be migraine with typical aura or migraine with brainstem aura. The migraine may be hemiplegic migraine (*e.g*., familial hemiplegic migraine or sporadic hemiplegic migraine); retinal migraine; chronic migraine; or probable migraine (with and without aura).

In treating migraine pain, PS may reduce the pain. In some instances, the reduction may be complete such that the migraine pain is eliminated. In treating the migraine pain, the PS may also reduce one or more of the symptoms associated with migraine. In preventing migraine pain, PS may decrease the incidence of the pain. In preventing migraine pain, the PS may also decrease the incidence of one or more of the symptoms associated with migraine. The PS may reduce cutaneous allodynia. In certain embodiments, PS may reduce chronic migraine pain. As noted above, the reduction may be complete such that the pain is eliminated.

Even if the symptoms experienced by a subject with migraine, for example aura or cranial autonomic symptoms, are not considered painful (or do not reach a threshold necessary to be considered pain *per se*), in treating and/or preventing migraine pain, PS may reduce any one or more of the symptoms experienced by a subject with migraine. In some instances, the reduction may be complete such that the one or more of the symptoms experienced by a subject with migraine are eliminated.

Peripheral and central sensitization are features of migraine pain. Migraine pain may be a consequence of central sensitisation resulting in allodynia, for example cutaneous allodynia, and/or hyperalgesia. Accordingly, subjects having migraine may experience pain as a consequence of this sensitisation, for example, experiencing pain induced by a non-painful stimulus (allodynia) or experiencing heightened pain in response to a harmful stimulus (hyperalgesia). On the basis of observations herein, PS may have a direct analgesic effect, for example by reducing the neuronal signalling involved in the sensation of pain. Accordingly, PS may reduce the neuronal signalling involved in the sensation of pain in a subject with migraine. In some instances, the reduction may be complete such that the pain is eliminated. Furthermore, PS may reduce pain generated via peripheral sensitisation or, in particular, via central sensitisation. In particular, given its ability to traverse towards central sites of pain generation, the PS may reduce pain generated via central sensitisation. The reduction may be complete such that the pain generation is eliminated. Accordingly, PS may reduce pain signalling occurring centrally. The PS may reduce pain signalling occurring in the trigeminal nerve. The PS may reduce pain signalling occurring in the trigeminal ganglion. The PS may reduce pain signalling occurring in the trigeminal nucleus caudalis, within the trigeminocervical complex (TCC). Given that PS is shown herein to ascend peripheral neurons towards the CNS, the preliminary observations of an analgesic activity of PS in migraine indicate that PS may reduce pain signalling occurring in higher order neurons and/or pain sensing regions of the brain (*e.g*., trigeminothalamic neurons). The reduction may be complete such that the pain signalling is eliminated. In some embodiments, the pain is allodynia, for example, cutaneous allodynia. The allodynia may be in response to mechanical and/or thermal stimuli. In addition, in some embodiments, the pain is hyperalgesia. The migraine pain may be neuropathic pain.

A patient with migraine can be diagnosed using the well-known ICHD-3 guidelines. Migraine pain can be measured on a visual analogue pain scale or using any other appropriate method in the art.

PS may be formulated into a pharmaceutical composition for use in the invention. In some embodiments, the pharmaceutical composition comprises PS and one or more pharmaceutically acceptable excipients. PS is preferred as a formulation for topical administration. PS can be administered in the vicinity of the sensory branches of the trigeminal nerve (*i.e.,* the ophthalmic nerve, the maxillary nerve and/or the mandibular nerve). PS may be administered topically to the face and/or neck of the subject. PS may be administered topically to one or both temples of the subject. In particular instances, PS may be administered topically behind one or both ears of the the subject.

In particular embodiments, for treating and/or preventing migraine pain, PS may be administered orally.

### Pain of other headache disorders

In light of the observations herein of the efficacy of PS in treating migraine pain, PS may treat and/or prevent pain of other headache disorders, in particular headache disorders with pathophysiology manifesting in dysfunction of the trigeminal system.

For example, PS may treat and/or prevent chronic headache pain, tension-type headache pain and/or trigeminal autonomic cephalalgia pain. The trigeminal autonomic cephalalgia may be cluster headache, hemicrania continua, paroxysmal hemicrania, short-lasting unilateral neuralgiform headache with conjunctival injection and tearing, and short-lasting unilateral neuralgiform headache with cranial autonomic symptoms. In some embodiments, PS may treat and/or prevent trigeminal neuralgia pain, for example head and facial pain.

PS may be formulated into a pharmaceutical composition for use in the invention. In some embodiments, the pharmaceutical composition comprises PS and one or more pharmaceutically acceptable excipients. PS is preferred as a formulation for topical administration. PS can be administered in the vicinity of the sensory branches of the trigeminal nerve (*i.e.,* the ophthalmic nerve, the maxillary nerve and/or the mandibular nerve). PS may be administered topically to the face and/or neck of the subject. PS may be administered topically to one or both temples of the subject. In particular instances, PS may be administered topically behind one or both ears of the the subject.

In particular embodiments, for treating and/or preventing pain of other headache disorders, PS may be administered orally.

### Corneal neuropathic pain

Corneal discomfort affects 5-30% of the population aged over 50 years. Corneal neuropathic pain is a condition in which corneal pain is experienced in response to normally non-painful stimuli (*e.g.,* a wind or draught). This is a reflection of the sensitization of centrally acting neurons after prolonged and repeated nociceptive signalling from peripheral neurons in response to direct damage to corneal nerves. Any mechanical or chemical injury to the corneal nerve endings can result in ectopic sprouts and neuroma formation which show spontaneous activity. The central sensitization, manifesting as painful hypersensitivity to non-noxious stimuli (allodynia) or an exaggerated pain response to noxious stimuli (hyperalgesia), or indeed spontaneous signalling, results in sustained chronic corneal pain even in the absence of peripheral stimuli or clinical signs. In this way, corneal neuropathic pain is distinct in its mechanism of pain generation compared to, for example, inflammatory eye disorders which trigger acute peripheral pain signalling from the cornea in response to ongoing inflammation.

Patients with corneal neuropathic pain experience severe corneal pain, as well as irritation such as burning, photophobia and grittiness, even without peripheral signs. Accordingly, this indication inevitably negatively impacts the quality of life of patients. The chronic pain sensation, accompanied by light sensitivity and irritation results in impaired functioning and inability to perform routine daily activities.

Corneal neuropathic pain is particularly difficult to treat. For corneal neuropathic pain patients, experiencing corneal pain in the absence of any ongoing corneal pathology, anti-inflammatory drugs are ineffective. Instead, centrally acting neuromodulators are often recommended. For example, anticonvulsants (*e.g*., gabapentin and pregabalin) can be considered as the first line treatment; serotonin-norepinephrine reuptake inhibitors (*e.g*., duloxetine and venlafaxine) as the second line treatment; and tricyclic antidepressants (*e.g*., nortriptyline, amitriptyline) as third-line agents. Combination therapy or weak opioids (tramadol) can also be used in the case of resistance to treatment in the setting of a broad neurologic pain.

Unfortunately, pain control is not very satisfactory and many of the systemic treatments induce major side effects leading to poor treatment adherence.

Therefore, there is a strong need for compounds that treat and/or prevent corneal neuropathic pain.

The inventor has surprisingly found that phosphosulindac (PS) is effective in the treatment of corneal neuropathic pain.

As noted herein, PS is a non-steroidal compound with anti-inflammatory activity. However, unlike its parent compound, the NSAID sulindac, PS does not inhibit COX-1 and COX-2 prostaglandin synthesis, and so is not a typical NSAID. PS has previously been shown to have anti-cancer and anti-inflammatory properties via its inhibition of activation of NF-κB and changes in MAPK signalling branches, as well as an activity in treating rheumatoid arthritis in inflammatory mouse models via suppression of key pro-inflammatory signalling pathways (Mackenzie et al. (2010) Gastroenterology 139(4): 1320-32 and Mattheolabakis et al. (2013) Pharm Res 30(6): 1471-82). WO 2019/067919 suggests an anti-inflammatory activity of PS in an acute model of dry eye disease (DED) and suggests PS decreases corneal sensitivity in an acute model of pain generation in normal eyes. Observations of reduced sensitivity to an acute stimulus in healthy eyes, which does not generate persistent pain, do not demonstrate efficacy in treating corneal neuropathic pain, which is associated with central sensitization and thus can be generated in central sites of action. Furthermore, in this acute model, the effect of PS was observed immediately, suggesting a local action of the atypical NSAID, PS, akin to the activity observed for typical NSAIDs (e.g. ketorolac) in the same model. Again, such peripheral activity does not demonstrate an efficacy of PS in treating pain generated at central sites of action.

Indeed, clinical guidance in the field recommends avoiding the use of NSAIDs for the treatment of all types of neuropathic pain, and ketorolac has been shown to have limited analgesic activity in models of such pain - accordingly the observations in an acute pain model do not extrapolate to treatment of corneal neuropathic pain. Furthermore, in the DED model, PS is seen to restore suppressed corneal sensitivity, suggesting a role of PS in increasing rather than reducing nociception. Therefore, these observations fail to suggest a role of PS in treating corneal neuropathic pain, while the observations herein indicate an unprecedented activity of PS in reducing pain generated at central sites of action.

Indeed, preliminary in vivo evidence indicates the efficacy of PS in the treatment of corneal neuropathic pain.

Therefore, the invention provides a method of treating corneal neuropathic pain comprising administering a therapeutically effective amount of PS to a subject in need thereof such that corneal neuropathic pain is treated.

In some embodiments, the PS is the sulfoxide form of PS. Therefore, the PS may have the formula I (PS-I):

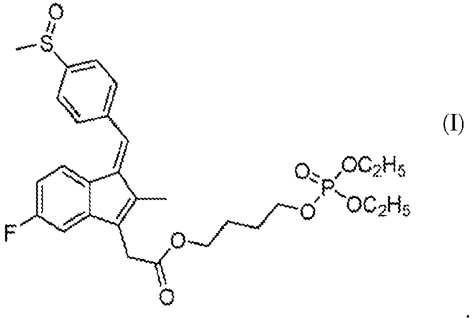

In other embodiments, the PS is the sulfide form of PS. Therefore, the PS may have the formula II (PS-II):

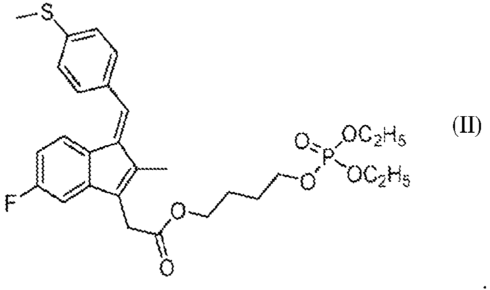

Herein, references to 'phosphosulindac' or to 'PS' encompass both PS-I and PS-II. The sulfoxide form of the compound is preferred. The compounds of formulae I and II are described in U.S. Patent No. 8,236,820, which is hereby incorporated by reference in its entirety.

Corneal neuropathic pain is also known as ocular neuropathic pain. Accordingly, herein the terms 'corneal neuropathic pain' and 'ocular neuropathic pain' may be used interchangeably. As explained above, corneal neuropathic pain is a persistent chronic pain which occurs in light of neuroplastic changes in centrally located neurons due to persistent nociceptive signalling from the periphery. Such changes result in hyperexcitability of nociceptors in the CNS, so-called central sensitization, which manifests, for example as allodynia. The pain persists even in the absence of an on-going peripheral trigger, rendering corneal neuropathic pain, like other neuropathic pains, particularly difficult to treat as resolving peripheral pathology does not affect the sensation of pain. In this way, the corneal neuropathic pain may be chronic corneal pain.

Corneal neuropathic pain can develop as a result of a number of peripheral drivers of nociception. Typically, corneal neuropathic pain develops in light of consistent pain signalling generated from the cornea (one of the most densely innervated tissues). As noted above, persistent peripheral signalling through corneal nerves ultimately results in central sensitization, a key feature of corneal neuropathic pain, which can result in the sensation of pain even in the absence of peripheral triggers. Therefore, corneal neuropathic pain may be caused by any peripheral stimulus that causes chronic stimulation of corneal nerves. For example, the corneal neuropathic pain may be caused by chronic corneal surface diseases or conditions, for example recurrent corneal erosions, corneal surface neoplasia, and/or inflammatory eye conditions. The corneal neuropathic pain may be caused by surgical interventions, for example kerato-refractive surgery (*e.g*., photorefractive keratectomy (PRK), laser in-situ keratomileusis (LASIK), Small Incision Lenticule Extraction (SMILE) and corneal inlay procedures), cataract surgery (*e.g.,* laser-assisted cataract surgery), corneal transplant surgeries, and/or laser retinopexy. The corneal neuropathic pain may be caused by laser procedures for the treatment of retinal conditions (*e.g.,* diabetic macular oedema; proliferative diabetic retinopathy; macular oedema due to retinal vein occlusions; neovascularisation secondary to retinal vein occlusions; peripheral retinal degenerations, holes, and/or tears; Eales' disease and other retinal vasculitis; central serious retinopathy; retinopathy of prematurity; extrafoveal polyps of polypoidal choroidal vasculopathy (PCV)). The corneal neuropathic pain may be caused by an infection, for example herpes simplex keratitis and/or herpes zoster keratitis. The corneal neuropathic pain may be caused by toxic keratopathy, for example due to topical or systemic agents (*e.g.*, preservatives containing benzalokium chloride or isotretinoin, respectively). The corneal neuropathic pain may be caused by radiation or ultraviolet light exposure. The corneal neuropathic pain may be a result of systemic neuropathies, for example small fiber neuropathy or multiple sclerosis. Furthermore, the corneal neuropathic pain may be caused by trauma, for example causing damage to the corneal nerves, for example chemical burns. The initial trigger of the pain may be air pollution or dry weather, which triggers persistent corneal inflammation and ultimately corneal neuropathic pain. Accordingly, the corneal neuropathic pain may be caused by allergens, for example causing allergic conjunctivitis. The corneal neuropathic pain may be caused by one or more chalazions, for example due to the persistent irritation of the cornea caused by the chalazion during blinking. Persistent peripheral nociceptor signalling in response to these peripheral triggers results in increased sensitivity of centrally located neurons resulting in the generation of pain even once the initial clinical presentation has resolved. Indeed, even local anaesthesia cannot relieve the pain given the dysregulation of centralised neuronal signalling.

Additionally, in some embodiments, the corneal neuropathic pain is caused by direct damage to centrally located neurons, for example by ischemia, hemorrhage, mechanical compression, infection, and/or degenerative processes. Furthermore, the corneal neuropathic pain may be a result of injury to adjacent tissues or nerves, for example the conjunctiva, eye muscles, globe, optic nerve, and or autonomic or sympathetic nerves.

Corneal neuropathic pain arises as a result of perisitent peripheral nociceptive signalling resulting in neuronal sensitization, manifested as hypersensitivity to innocuous peripheral triggers and associated long-lived pain. Therefore, central sensitization is a feature of corneal neuropathic pain. The pain can manifest for example as shooting, burning, or stabbing pain associated with other sensory symptoms. Accordingly, in some embodiments, the invention provides a method of treating corneal neuropathic pain comprising administering a therapeutically effective amount of PS to a subject in need thereof such that corneal neuropathic pain is treated. A subject experiencing corneal neuropathic pain would benefit from an analgesic which can both treat existing corneal neuropathic pain and prevent generation of further corneal neuropathic pain (*i.e.,* prevent further central sensitization). Therefore, in some embodiments, PS can be used in the treatment and prevention of corneal neuropathic pain. In line with the above, the invention provides PS for use in the treatment of corneal neuropathic pain. Furthermore, the invention provides the use of PS for the manufacture of a medicament for the treatment of corneal neuropathic pain.

On the basis of the observations herein, PS has a direct analgesic effect on corneal neuropathic pain. The corneal neuropathic pain may be a stabbing or burning pain. In treating corneal neuropathic pain, PS may reduce the corneal neuropathic pain. In some instances, the reduction may be complete such that the corneal neuropathic pain is eliminated. In treating the corneal neuropathic pain, the PS may also reduce one or more of the associated sensory symptoms.

Patients suffering from corneal neuropathic pain describe a range of sensory symptoms. The sensory symptoms include paresthesia (*e.g*., numbness, tingling, pricking, and/or formication), photosensitivity, or photoallodynia. Even if the sensory symptoms experienced by the subject are not considered painful (or do not reach a threshold necessary to be considered pain *per se*), PS may reduce any one or more of the sensory symptoms experienced by the subject, including those listed above. In some instances, the reduction may be complete such that the one or more of the associated sensory symptoms are eliminated. Furthermore, in treating corneal neuropathic pain PS may improve other related symptoms in the subject, for example anxiety, depression, and/or apathy.

As noted above, corneal neuropathic pain is generated as a result of over-activity of centrally located neurons, which display reduced stimulation thresholds and can depolarise even in the absence of a peripheral stimulus. Indeed, the perception of pain is no longer coupled to the presence, intensity, or duration of a particular peripheral stimulus (noxious or otherwise). Thus, the corneal neuropathic pain may be a consequence of central sensitization. Accordingly, subjects having corneal neuropathic pain may experience pain induced by a non-painful stimulus (allodynia) and/or may experience heightened pain in response to a harmful stimulus (hyperalgesia). On the basis of preliminary observations, PS may have a direct analgesic effect, for example by reducing the neuronal signalling involved in the sensation of pain. Thus, PS may reduce the neuronal signalling involved in the sensation of pain in a subject with corneal neuropathic pain. Given its ability to traverse towards central sites of pain generation, PS may reduce pain generated via central sensitization. Accordingly, PS may reduce pain signalling occurring centrally. The corneal nociceptors make the first division of the trigeminal nerve and travel to the trigeminal ganglion and trigeminal nucleus caudalis. The PS may reduce pain signalling occurring in the trigeminal ganglion. The PS may reduce pain signalling occurring in the trigeminal nucleus caudalis, within the trigeminocervical complex (TCC). Given that PS is shown herein to ascend peripheral neurons towards the CNS, PS may reduce pain signalling occurring in higher order neurons and/or pain sensing regions of the brain (*e.g*., trigeminothalamic neurons). In some instances, the reduction may be complete such that the pain signalling is eliminated. In some embodiments, the corneal neuropathic pain is allodynia. The allodynia may be in response to mechanical and/or thermal stimuli. Additionally or alternatively, the corneal neuropathic pain in a subject may be hyperalgesia. In some embodiments, the corneal neuropathic pain is chronic corneal neuropathic pain. In some embodiments, the corneal neuropathic pain is not acute corneal pain.

Corneal neuropathic pain in a patient can be measured on a visual analogue pain scale or using any other appropriate method in the art.

Given the observations herein, PS shows direct activity on neurons associated with the generation of pain caused by central sensitization. Therefore, PS may also be useful for treating particular forms of corneal pain. Corneal pain is also known as ocular pain, and so these terms may be used interchangeably herein. For example, PS may treat corneal pain caused by central sensitization. Therefore, the invention also provides a method of treating corneal pain caused by central sensitization comprising administering a therapeutically effective amount of PS to a subject in need thereof such that corneal pain caused by central sensitization is treated. Indeed, PS may treat corneal pain manifesting as allodynia. PS may treat corneal pain manifesting as hyperalgesia. As the pain is caused by central sensitization, it is experienced in the absence of ongoing noxious peripheral triggers (*e.g.,* corneal inflammation). The corneal pain may be chronic pain (*i.e.,* pain persisting for 3 months or more). In certain embodiments, the corneal pain is not acute pain, for example acute pain associated with DED (*i.e.,* pain experienced as a result of ongoing corneal inflammation).

PS may be useful for treating corneal pain generated at central sites of action (*i.e.,* those sites responsible for central pain signalling in the absence of ongoing peripheral triggers). Therefore, the invention provides a method of treating corneal pain generated at central sites of action comprising administering a therapeutically effective amount of PS to a subject in need thereof such that corneal pain generated at central sites of action is treated. Indeed, given the observations herein, PS may treat corneal pain in light of its ability to traverse towards central sites of action. For example, PS may reduce corneal pain by accumulating within the trigeminal nucleus caudalis, or within higher order neurons within the CNS. Accordingly, the PS may be acting directly on the pain generating centers within the trigeminal nucleus caudalis, or within higher order neurons within the CNS. Therefore, the invention provides a method of treating corneal pain comprising administering a therapeutically effective amount of PS to a subject in need thereof such that corneal pain is treated, wherein the PS reduces pain signalling occurring within the trigeminal nucleus caudalis, or within higher order neurons within the CNS. In some instances, the reduction may be complete such that the pain signalling is eliminated.

The observations herein demonstrate an ability of PS, administered topically to the outer surface of the eyelid, to penetrate the tissues of the eyelid and reach the cornea in therapeutically relevant amounts. Administration to the outer surface of the eyelid avoids the need for using eyedrops which may generate a stinging sensation that reduces patient compliance and thus therapeutic outcomes. Furthermore, administration to the outer surface of the eyelid is preferred for those patients with reduced motor functionality or loss of fine motor skills who may struggle to administer eyedrops. Of course, such administration to the outer surface of the eyelid requires the therapeutic agent to effectively penetrate the eyelid such that it reaches the ocular surface in therapeutically relevant amounts. Accordingly, the observations herein validate administration of PS to the outer surface of the eyelid as an appropriate topical administration route for treating various diseases and conditions of the eye, in particular those in which the therapeutic agent must reach the ocular surface in therapeutically relevant amounts to achieve therapeutic efficacy. Without wishing to be bound by theory, the ability of PS to reach the corneal nerves in therapeutically relevant amounts may allow PS to traverse along the peripheral corneal neurons towards central sites of action, thus providing a route by which PS can reach pain generating centers associated with corneal pain, even when administered topically to the outer surface of the eyelid.

Therefore, in a further aspect, the invention provides PS for use in therapy, wherein the PS is topically administered to the outer surface of one or more eyelids. The invention also provides a method of treating and/or preventing an eye disease or condition, comprising administering a therapeutically effective amount of PS to a subject in need thereof such that the eye disease or condition is treated and/or prevented, wherein the PS is topically administered to the outer surface of one or more eyelids. The eye disease or condition may be corneal pain, for example corneal pain as described herein, for example corneal neuropathic pain. Therefore, the invention also provides a method of treating and/or preventing corneal pain, comprising administering a therapeutically effective amount of PS to a subject in need thereof such that corneal pain is treated and/or prevented, wherein the PS is topically administered to the outer surface of one or more eyelids. The one or more eyelids may be one or both of the upper eyelids and/or one or both of the lower eyelids.

The corneal pain may be caused by central sensitization. The corneal pain may be generated at central sites of action. As provided herein, the corneal pain may be caused by one or more surgical interventions, for example kerato-refractive surgery (including PRK, LASIK, SMILE and/or corneal inlay procedures); laser retinopexy; cataract surgery (*e.g.,* laser-assisted cataract surgery); and/or corneal transplant surgeries. Also as discussed herein, the corneal pain may be caused by laser procedures for the treatment of retinal conditions (*e.g*., diabetic macular oedema; proliferative diabetic retinopathy; macular oedema due to retinal vein occlusions; neovascularisation secondary to retinal vein occlusions; peripheral retinal degenerations, holes, and/or tears; Eales' disease and other retinal vasculitis; central serious retinopathy; retinopathy of prematurity; extrafoveal polyps of polypoidal choroidal vasculopathy (PCV)). The corneal pain may be caused by intravitreal injection (*e.g.,* for the treatment of wet age-related macular degeneration (AMD)). The corneal pain may be experienced during or after the surgical intervention or procedure. In this way, the PS may be administered before, during, or after the surgical intervention or procedure. In particular instances, the corneal pain is experienced during the the surgical intervention or procedure. In this way, the PS may be administered before or during the surgical intervention or procedure. As explained herein, the corneal pain may be caused by an inflammatory eye disease or condition. The inflammatory eye disease or condition may be dry eye disease (DED). The inflammatory eye disease or condition may be allergic conjunctivitis. The corneal pain may be caused by one or more chalazions, for example due to the persistent irritation of the cornea caused by the chalazion during blinking. The corneal pain may be pain caused by one or more of the following: chronic corneal surface diseases or conditions (*e.g*., recurrent corneal erosions, corneal surface neoplasia, and/or inflammatory eye conditions, for example dry eye disease (DED) or inflammatory eye conditions caused by allergens, such as allergic conjunctivitis); infection (*e.g*., herpes simplex keratitis and/or herpes zoster keratitis); toxic keratopathy (*e.g*., to topical or systemic agents); radiation or ultraviolet light exposure; trauma (*e.g*., chemical burns).

The eye disease or condition may be an inflammatory eye disease or condition. The inflammatory eye disease or condition may be dry eye disease (DED), conjunctivitis (*e.g.,* allergic conjunctivitis), keratitis, scleritis or uveitis. In particular embodiments, the eye disease or condition is dry eye disease (DED).

In line with the above, the invention provides PS for use in treating and/or preventing an eye disease or condition, wherein the PS is topically administered to the outer surface of one or more eyelids. Additionally, the invention provides the use of PS for the manufacture of a medicament for treating and/or preventing an eye disease or condition, wherein the PS is topically administered to the outer surface of one or more eyelids.

PS may be formulated into a pharmaceutical composition for use in the invention. In some embodiments, the pharmaceutical composition comprises PS and one or more pharmaceutically acceptable excipients. PS may be formulated for topical administration.

The formulations disclosed herein for topical administration of PS to the outer surface of the eyelid are applicable for use in the methods disclosed herein. The eyelid may be the upper eyelid or the lower eyelid. For topical administration to the outer surface of the eyelid the PS is substantially all applied to the outer surface of the eyelid.

### Pharmaceutical compositions

The PS for use in the methods of the invention can be formulated into an appropriate pharmaceutical composition for administering to subjects in need thereof, for example subjects with pain associated with central sensitization, subjects with PTPN, subjects with PHN, subjects with migraine pain (or pain of other headache disorders), or subjects with corneal neuropathic pain. Pharmaceutical compositions are typically formulated to provide a therapeutically effective amount of PS and may further comprise a pharmaceutically acceptable excipient. In particular embodiments, the pharmaceutical composition comprising PS may be formulated for topical formulation for use in the invention.

Pain associated with central sensitization can be generalized or can occur at various sites on the body. The pain may be diffuse and widespread, or may be localised to an area from which initial pain signalling was generated. The pain typically manifests at peripheral sites. Therefore, a particularly useful pharmaceutical composition comprising PS is one which can be applied directly to peripheral locations experiencing pain. Accordingly, the pharmaceutical composition comprising PS may be formulated for topical administration. In particular, the pharmaceutical composition comprising PS may be formulated for dermal administration.

Neuropathic pain associated with PTPN is sensed within the tissue of injury but pain may also be diffuse in proximity of the site of injury. Irrespective, the pain is sensed from peripheral regions. Therefore, a particularly useful pharmaceutical composition comprising PS is one which can be applied directly to peripheral locations experiencing neuropathic pain. In addition, the pharmaceutical composition comprising PS may be applied to those locations experiencing one or more sensory symptoms of PTPN. Accordingly, the pharmaceutical composition comprising PS may be formulated for topical administration. In particular, the pharmaceutical composition comprising PS may be formulated for dermal administration. A single application to the affected area may require less than about 5 ml of the pharmaceutical composition, for example about 3 ml of the pharmaceutical composition.

Neuropathic pain associated with PHN most commonly appears on a subject's trunk.As outlined above, PHN tends to affect one or two adjacent dermatomes and generally does not cross the midline of the body. Less commonly, PHN can be more widespread, affecting three or more dermatomes. Typically, the neuropathic pain associated with PHN manifests in one or more thoracic dermatomes (*i.e*., the subject's trunk). Therefore, a particularly useful pharmaceutical composition comprising PS is one which can be applied directly to peripheral locations experiencing neuropathic pain, for example the thoracic dermatomes on the trunk of the subject. In addition, the pharmaceutical composition comprising PS may be applied to those locations experiencing one or more sensory symptoms of PHN. Accordingly, the pharmaceutical composition comprising PS may be formulated for topical administration. In particular, the pharmaceutical composition comprising PS may be formulated for dermal administration, in particular to the skin of the thoracic dermatomes of the subject. A single application to one or more thoracic dermatome may require up to about 15 ml of the pharmaceutical composition. A single application to both an area affected by neuropathic pain associated with a disseminated zoster (*i.e.,* three or more dermatomes) may require up to about 30 ml of the pharmaceutical composition.

Migraine pain (and pain of other headache disorders) is sensed as head and face pain. Therefore, a particularly useful pharmaceutical composition comprising PS is one which can be applied directly to peripheral locations experiencing the pain, for example the head and/or neck of the subject. Accordingly, the pharmaceutical composition comprising PS may be formulated for topical administration. In particular, the pharmaceutical composition comprising PS may be formulated for dermal administration, in particular to the skin in the vicinity of the sensory branches of the trigeminal nerve. PS may be administered to the skin of the head and/or neck of the subject. PS may be topically administered where the neck meets the base of the skull. PS may be topically administered to one or both of the subject's temples. PS may be topically administered behind one or both of the subject's ears. A single application, for example to the temples or behind the ears, may require less than about 2 ml of the pharmaceutical composition, for example about 1 ml of the pharmaceutical composition (*i.e.,* about 0.5 ml of the pharmaceutical composition per temple or behind each ear).

Corneal neuropathic pain, like corneal pain, is sensed in the eye. Therefore, a particularly useful pharmaceutical composition comprising PS is one which can be applied such that it reaches the eyeball of the subject experiencing corneal neuropathic pain in therapeutically relevant amounts. Accordingly, the pharmaceutical composition comprising PS may be applied directly to one or both eyeballs of the subject experiencing corneal neuropathic pain. Accordingly, the pharmaceutical composition comprising PS may be formulated for topical administration to the ocular surface (*i.e.,* to the eyeball), for example onto the cornea or to a canthus. In preferred embodiments, the pharmaceutical composition comprising PS may be applied topically to the outer surface of one or more eyelids of the subject. Accordingly, the pharmaceutical composition comprising PS may be formulated for dermal administration, in particular to the skin of one or more eyelids of the subject (*i.e.,* the outer surface of one or more eyelids of the subject). The one or more eyelids may be one or both upper eyelids. The one or more eyelids may be one or both lower eyelids. For topical administration to the outer surface of the eyelid, the PS is substantially all applied to the outer surface of the eyelid. In certain instances, the lower eyelid may be preferred because the outer surface of the lower eyelid is less compromised, compared to the outer surface of the upper eyelid, by the process of blinking (*i.e.,* when the eye is open, the outer surface of the upper eyelid is juxtaposed to the skin of the orbit). The upper eyelid may be preferred in certain instances given the typically larger surface area of its outer surface for topical administration. A single application to one eyelid may require less than about 1 ml of the pharmaceutical composition, for example about 0.5 ml of the pharmaceutical composition.

In some embodiments, the pharmaceutical composition comprising PS may be formulated as a semi-solid or liquid. Therefore, the pharmaceutical composition comprising PS may be formulated as a cream, gel (*e.g.,* a hydrogel), lotion, ointment, foam, and/or spray. These compositions differ in their relative concentrations of oils and water, which causes the compositions to have different densities. Altering the density of the formulation is a way in which exposure of the affected area to the pharmaceutical composition can be controlled. For example, a less dense formulation, which requires rubbing in until it has been absorbed, may result in a shorter exposure time. Alternatively, a more dense formulation, which is not readily absorbed, may allow prolonged exposure of the area to the pharmaceutical composition. The skilled person is aware of formulating topical pharmaceutical compositions so as to modify the relative exposure of the area to the active pharmaceutical ingredient.

In treating corneal neuropathic pain, the pharmaceutical composition comprising PS for topical administration to the ocular surface and/or the outer surface of the eyelid may be formulated as a gel (*e.g.,* a hydrogel) or ointment. For topical administration to the ocular surface, the pharmaceutical composition comprising PS may be formulated as a gel (*e.g.,* a hydrogel), ointment, and/or eye drops. For topical administration to the outer surface of the eyelid, the pharmaceutical composition comprising PS may be formulated as a cream, gel (*e.g.,* a hydrogel), lotion, ointment, foam, and/or spray.

In other embodiments, the pharmaceutical composition comprising PS may be formulated as a patch which can be applied to the skin. The patch may be manufactured in such a way as to ensure controlled release of PS to the affected area.

In some embodiments of treating corneal neuropathic pain, the pharmaceutical composition comprising PS may be formulated as a patch which can be applied to the skin of one or more eyelids (*e.g.,* one or both lower eyelids) of the subject. The patch may be manufactured in such a way as to ensure controlled release of PS through the outer surface of the eyelid such that therapeutically appropriate amounts of PS reach the ocular surface.

In particular embodiments of treating corneal neuropathic pain, the pharmaceutical composition comprising PS may be formulated as eye drops. Such eye drop formulations may include a liquid or semi-solid pharmaceutical composition adapted to administration to the eye. A typical example of an eye drop composition is an ophthalmic solution administered dropwise to the eye. In some embodiments, an eye drop composition is an ophthalmic emulsion administered dropwise to the eye. In some embodiments, the size of the drop is between about 10 and about 100 µL. The drop size may be greater than about 10 µL, greater than about 20 µL, greater than about 30 µL, greater than about 40 µL, greater than about 50 µL, greater than about 60 µL, greater than about 70 µL, greater than about 80 µL, greater than about 90 µL, or greater than about 100 µL. The drop size may be less than about 10 µL, less than about 20 µL, less than about 30 µL, less than about 40 µL, less than about 50 µL, less than about 60 µL, less than about 70 µL, less than about 80 µL,less than about 90 µL, or less than about 100 µL.

Formulations suitable for topical administration and appropriate pharmaceutically acceptable excipients are well-known in the art. Exemplary formulations for topical administration are provided in WO 2019/067919, which is hereby incorporated by reference in its entirety.

In some embodiments, the formulation of PS suitable for topical administration, may comprise PS at a concentration of about 0.5% w/w to about 15% w/w of the pharmaceutical composition. Accordingly, the PS may be at a concentration of 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition. As an illustrative example, when formulated as a topical cream, the PS may be at a concentration of less than or equal to 8% w/w of the pharmaceutical composition, for example about 5% w/w of the pharmaceutical composition, in particular about 3% w/w of the pharmaceutical composition. As a further illustrative example, when formulated as a gel, the PS may be at a concentration of less than or equal to 8% w/w of the pharmaceutical composition, for example less than or equal to 5% w/w of the pharmaceutical composition, in particular less than or equal to 3% w/w of the pharmaceutical composition, for example about 2% or about 1% w/w of the pharmaceutical composition. In particular formulations, for example when formulated as a hydrogel or an ointment, the PS may be at a concentration of 5% w/w of the pharmaceutical composition.

In some embodiments of treating corneal neuropathic pain, the formulation of PS suitable for topical administration to the ocular surface and/or the outer surface of the eyelid, may comprise PS at a concentration of about 0.5% w/w to about 15% w/w of the pharmaceutical composition. Accordingly, the PS may be at a concentration of 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition. As an illustrative example, when formulated as a topical cream, the PS may be at a concentration of less than or equal to 8% w/w of the pharmaceutical composition, for example about 5% w/w of the pharmaceutical composition, in particular about 3% w/w of the pharmaceutical composition. As a further illustrative example, when formulated as a gel, the PS may be at a concentration of less than or equal to 8% w/w of the pharmaceutical composition, for example less than or equal to 5% w/w of the pharmaceutical composition, in particular less than or equal to 3% w/w of the pharmaceutical composition, for example about 2% or about 1% w/w of the pharmaceutical composition. In particular formulations, for example when formulated as a hydrogel or an ointment, the PS may be at a concentration of 5% w/w of the pharmaceutical composition.

The pharmaceutical composition comprising PS may alternatively be formulated for any other form of administration suitable for treating pain associated with central sensitization. The pharmaceutical composition comprising PS may alternatively be formulated for any other form of administration suitable for treating and/or preventing neuropathic pain associated with PTPN. The pharmaceutical composition comprising PS may alternatively be formulated for any other form of administration suitable for treating and/or preventing migraine pain (or pain of other headache disorders). The pharmaceutical composition comprising PS may alternatively be formulated for any other form of administration suitable for treating and/or preventing neuropathic pain associated with PHN. For example, the composition may be formulated for transdermal administration or injection, for example subcutaneous injection.

In certain embodiments of treating corneal neuropathic pain, the composition may be formulated for intravitreal injection.

The formulations disclosed herein are also suitable for treating corneal pain, for example corneal pain caused by central sensitization and/or corneal pain generated at central sites of action.

As already disclosed herein, in a further aspect, the invention provides PS for use in therapy, wherein the PS is topically administered to the outer surface of one or more eyelids. The invention also provides a method of treating and/or preventing an eye disease or condition, comprising administering a therapeutically effective amount of PS to a subject in need thereof such that the eye disease or condition is treated and/or prevented, wherein the PS is topically administered to the outer surface of one or more eyelids.

In some embodiments of topical administration of PS to the outer surface of one or more eyelids, the pharmaceutical composition comprising PS may be formulated as a gel (*e.g.,* a hydrogel) or ointment. For topical administration to the outer surface of the eyelid, the pharmaceutical composition comprising PS may be formulated as a cream, gel (*e.g.,* a hydrogel), lotion, ointment, foam, and/or spray.

In some embodiments of topical administration of PS to the outer surface of one or more eyelids, the pharmaceutical composition comprising PS may be formulated as a patch which can be applied to the skin of one or more eyelids (*e.g.,* one or both lower eyelids) of the subject. The patch may be manufactured in such a way as to ensure controlled release of PS through the outer surface of the eyelid such that therapeutically appropriate amounts of PS reach the ocular surface.

In some embodiments of topical administration of PS to the outer surface of one or more eyelids, the formulation of PS may comprise PS at a concentration of about 0.5% w/w to about 15% w/w of the pharmaceutical composition. Accordingly, the PS may be at a concentration of 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition. As an illustrative example, when formulated as a topical cream, the PS may be at a concentration of less than or equal to 8% w/w of the pharmaceutical composition, for example about 5% w/w of the pharmaceutical composition, in particular about 3% w/w of the pharmaceutical composition. As a further illustrative example, when formulated as a gel, the PS may be at a concentration of less than or equal to 8% w/w of the pharmaceutical composition, for example less than or equal to 5% w/w of the pharmaceutical composition, in particular less than or equal to 3% w/w of the pharmaceutical composition, for example about 2% or about 1% w/w of the pharmaceutical composition. In particular formulations, for example when formulated as a hydrogel or an ointment, the PS may be at a concentration of 5% w/w of the pharmaceutical composition.

A single application to one eyelid may require less than about 1 ml of the pharmaceutical composition, for example about 0.5 ml of the pharmaceutical composition.

The observations herein surprisingly demonstrate that PS has a direct effect on neuronal pain signalling generated centrally (*e.g*., via central sensitization) even upon oral administration. Previous observations have shown that, upon topical administration, PS traverses along peripheral neurons towards central sites, thus permitting PS to act directly on neurons involved in pain generation. With topical administration, PS is administered to areas with a high concentration of sensory neurons in the periphery, permitting its uptake and movement to central sites of action in high enough quantities to achieve an analgesic effect. It could not have been expected that oral administration of PS would also achieve an analgesic effect for indications known to have a central site of pain generation, for example pain associated with central sensitization, pain associated with PTPN, pain associated with post-herpetic neuralgia (PHN), and migraine pain. Indeed, typical NSAIDs have been shown to be ineffective in the treatment of neuropathic pain, irrespective of route of administration (Moore et al. Cochrane Database of Systematic Reviews (2015); 10: 1-25). The Cochrane Library concluded that NSAIDs should not be recommended for the treatment of neuropathic pain.

The observations herein demonstrate that upon oral administration, PS is found in therapeutically relevant amounts in pain sensing regions of the brain (*e.g.,* the medulla and cerebellum). Evidence herein demonstrates that PS is taken up by the neurons innervating the stomach lining and traverses along the vagus nerve to reach central sites in the brain. These observations are supported by observations demonstrating the translocation of PS along the sciatic nerve and the absence of therapeutic levels of PS in the blood. Therefore, without wishing to be bound by theory, PS can impart its direct analgesic effects on the neurons within the centrally located pain sensing regions of the brain providing an elegant mechanism by which centrally generated pain can be resolved irrespective of its original aetiology.

Therefore, PS may be administered orally. In some embodiments, the pharmaceutical composition comprising PS for use in the invention may be formulated for oral administration.

Therefore, the invention provides a method of treating pain associated with central sensitization comprising administering a therapeutically effective amount of PS to a subject in need thereof such that pain associated with central sensitization is treated, wherein the PS is administered orally. The invention provides a method of treating and/or preventing neuropathic pain associated with CIPN comprising administering a therapeutically effective amount of PS to a subject in need thereof such that neuropathic pain associated with CIPN is treated and/or prevented, wherein the PS is administered orally. The invention provides a method of treating and/or preventing neuropathic pain associated with DPN comprising administering a therapeutically effective amount of PS to a subject in need thereof such that neuropathic pain associated with DPN is treated and/or prevented, wherein the PS is administered orally. The invention provides a method of treating and/or preventing neuropathic pain associated with PTPN comprising administering a therapeutically effective amount of PS to a subject in need thereof such that neuropathic pain associated with PTPN is treated and/or prevented, wherein the PS is administered orally. The invention provides a method of treating and/or preventing neuropathic pain associated with PHN comprising administering a therapeutically effective amount of PS to a subject in need thereof such that neuropathic pain associated with PHN is treated and/or prevented, wherein the PS is administered orally. The invention provides a method of treating and/or preventing migraine pain (or pain associated with other headache disorders) comprising administering a therapeutically effective amount of PS to a subject in need thereof such that migraine pain (or pain associated with other headache disorders) is treated and/or prevented, wherein the PS is administered orally. The invention provides a method of treating and/or preventing corneal neuropathic pain comprising administering a therapeutically effective amount of PS to a subject in need thereof such that corneal neuropathic pain is treated and/or prevented, wherein the PS is administered orally.

The PS for oral administration may be formulated as a liquid or solid dosage form.

Liquid dosage forms for oral administration include, but are not limited to, pharmaceutically acceptable emulsions, microemulsions, solutions, suspensions, syrups and elixirs.

Solid dosage forms for oral administration include but are not limited to capsules, tablets, pills, powders, and granules. The solid dosage forms of tablets, dragees, capsules, pills, and granules can be prepared with coatings and shells such as enteric coatings, release controlling coatings and other coatings well known in the pharmaceutical formulating art. The solid dosage forms of capsules, tablers and pills, may be such that they release PS only, or preferentially, in a certain part of the intestinal tract, for example the stomach, optionally, in a delayed manner.

In some embodiments, the formulation for oral administration comprises one or more fillers, disintigrants, lubricants, glidants, anti-adherents and/or anti-statics.

The formulations suitable for oral adminsitration may comprise PS at a concentration of 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%*,* 5%, 4.5%, 4%, 3.5%, 3%, 2.5%, 2%, 1.5%, 1%, or 0.5% w/w of the pharmaceutical composition.

Even upon oral administration, PS reduces pain signalling occurring in the CNS, in particular, in pain sensing regions of the brain. In some embodiments, oral administration of PS reduces pain signalling occurring in the brain. Upon oral administration, PS may accumulate (via the vagus nerve) at therapeutically relevant levels in the primary somatosensory cortex, secondary somatosensory cortex, anterior cingulate cortex (ACC), prefrontal cortex (PFC), insular cortex, amygdala, thalamus, cerebellum, and periaqueductal gray matter (PAG). In particular embodiments, upon oral administration, PS accumulates at therapeutically relevant levels in the medulla and/or cerebellum. PS, for example upon oral administration, may reduce pain signalling in the somatosensory cortex, for example the primary somatosensory cortex. Orally administered PS may reduce pain signalling in one or more of the following areas of the brain: primary somatosensory cortex, secondary somatosensory cortex, anterior cingulate cortex, prefrontal cortex, insular cortex, amygdala, thalamus, cerebellum, and periaqueductal gray matter. In particular embodiments, orally administered PS may reduce pain signalling in the medulla and/or cerebellum.

### Dosing Regimens

The appropriate dosage regimen for PS for treating the indications described herein (*e.g.,* pain associated with central sensitization, neuropathic pain associated with PTPN, neuropathic pain associated with PHN, migraine pain (and pain of other headache disorders), or corneal neuropathic pain) will depend on such variables as the type and extent of progression of the pain *(e.g.,* as determined by the "Pain Ladder" guideline from the World Health Organization), the severity of the pain *(e.g.,* acute, subacute, or chronic), the age, weight, and general condition of the particular patient, formulation of the excipient, the route of administration, and the judgment of the attending clinician.

For topical administration, the PS can be administered to cover the one or more affected areas of the subject *(i.e.,* those peripheral areas experiencing pain). In some embodiments, about 0.01 to about 5 g of the PS may be administered to the affected area. With respect to the size of the affected area, the PS may be administered at about 0.005-0.25 g/10cm² of affected area. Therefore, the PS may be administered at about 0.005 g/10cm², 0.01 g/10cm², 0.05 g/10cm², 0.1 g/10 cm², 0.15 g/10cm², 0.2 g/10cm² or 0.25 g/10cm² of affected area.

For topical adminstration for treating and/or preventing neuropathic pain associated with PHN, the PS can be administered to cover the one or more affected areas, for example one or more thoracic dermatomes of the subject. The PS may be administered, for example topically, to the site of the rash or at a site experiencing sensory symptoms in advance of rash manifestation. Therefore, PS may be topically administered (*e.g*., to one or more thoracic dermatomes) in the prodromal phase or during the rash phase. Accordingly, PS may be administered prophylactically to prevent occurrence of neuropathic pain associated with PHN after resolution of the rash. In particular embodiments, the PS is administered, for example topically, to the site of the original rash after the rash and/or skin lesions have resolved. Therefore, PS may be topically administered (*e.g*., to one or more thoracic dermatomes) after resolution of the rash and/or skin lesions. In these instances, the PS may either prevent neuropathic pain associated with PHN from arising or treat neuropathic pain associated with PHN which is already established at the site of the original rash.

For topical adminstration for treating and/or preventing migraine pain (or pain associated with other headache disorders), the PS can be administered to cover the one or more affected areas, for example the temples or behind each ear of the subject.

For topical administration to the ocular surface, the PS can be administered to cover the affected area (*i.e.,* the ocular surface). For topical administration to the outer surface of the eyelid, the PS can be administered to ensure a therapeutically appropriate amount of PS reaches the ocular surface upon topical administration to the outer surface of the eyelid. As noted above, for topical administration to the outer surface of the eyelid, the PS is substantially all applied to the outer surface of the eyelid.

In embodiments of topical administration to the ocular surface, about 0.001 to about 1 mg of the PS may be administered to the ocular surface. With respect to the size of the ocular surface, the PS may be administered at about 0.005-0.25 mg/cm² of the ocular surface. Therefore, the PS may be administered at about 0.005 mg/cm², 0.01 mg/cm², 0.05 mg/cm², 0.1 mg/cm², 0.15 mg/cm², 0.2 mg/cm² or 0.25 mg/cm² of ocular surface. The PS may be administered at about 0.005 mg, 0.01 mg, 0.05 mg, 0.1 mg, 0.15 mg, 0.2 mg or 0.25 mg per eyedrop.

In embodiments of topical administration to the outer surface of the eyelid, about 0.1 to about 250 mg of the PS may be administered to the outer surface of the eyelid. With respect to the size of the outer surface of the eyelid, the PS may be administered at about 0.5-100 mg/cm² of the outer surface of the eyelid. Therefore, the PS may be administered at about 0.5 mg/cm², 5 mg/cm², 10 mg/cm², 25 mg/cm², 50 mg/cm², 75 mg/cm² or 100 mg/cm² of the outer surface of the eyelid.

The PS for use in topical administration for the methods of the invention in some instances may be applied and then removed from the affected area (*e.g.,* by washing off) before reapplication. In some instances the PS is washed off after a certain period of time. Alternatively, as the analgesic effect may reduce over time and reapplication may be necessary, in some instances the PS is not washed off and instead PS is simply reapplied to the affected area after passing of the appropriate dosing period. For example, PS may be applied to the affected area and left on the affected area (before removal or reapplication) for between about 0.5 hours and about 5 hours. Accordingly, PS may be applied topically and left on the affected area (before removal or reapplication) for about 0.5 hours, for about 1 hour, for about 2 hours, for about 3 hours, for about 4 hours, or for about 5 hours.

As the analgesic effect may reduce over time, the PS for use in topical administration to the ocular surface and/or the outer surface of the eyelid, in some instances may be reapplied to the ocular surface and/or the outer surface of the eyelid after passing of the appropriate dosing period. For those embodiments involving topical adminstration to the outer surface of the eyelid, the PS may be removed from the outer surface of the eyelid (*e.g.,* by washing off) before reapplication. In some instances the PS is washed off after a certain period of time. In some instances the PS is not washed off and instead PS is simply reapplied. For example, PS may be applied to the to the ocular surface and/or the outer surface of the eyelid and left on the the ocular surface and/or the outer surface of the eyelid (before removal or reapplication) for between about 0.5 hours and about 5 hours. Accordingly, PS may be applied topically to the ocular surface and/or the outer surface of the eyelid and left on the the ocular surface and/or the outer surface of the eyelid (before removal or reapplication) for about 0.5 hours, for about 1 hour, for about 2 hours, for about 3 hours, for about 4 hours, or for about 5 hours.

As pain associated with the indications described herein (*e.g.,* pain associated with central sensitization, neuropathic pain associated with PTPN and neuropathic pain associated with PHN) is chronic, it is necessary to repeat topical administration of PS. Similarly, as migraine pain (and pain of other headache disorders) may be long-term, for example up to 72 hours, and even longer for chronic migraine, it is necessary to repeat topical administration of PS. Accordingly, the PS may be applied topically 1 to 4 times a day. Therefore, the PS may be applied once a day, twice a day, three times a day, or four times a day. With particular formulations of PS, for example a hydrogel or an ointment with a PS concentration of about 5% w/w of the pharmaceutical composition, the formulation may be applied topically three times a day. In more severe cases, a further application of PS may be applied about 0.5 hours after each application.

As corneal neuropathic pain is chronic, it is necessary to repeat topical administration of PS. Accordingly, the PS may be applied topically to the ocular surface and/or the outer surface of the eyelid 1 to 4 times a day. Therefore, the PS may be applied once a day, twice a day, three times a day, or four times a day. With particular formulations of PS, for example a hydrogel or an ointment with a PS concentration of about 5% w/w of the pharmaceutical composition, the formulation may be applied topically to the ocular surface and/or the outer surface of the eyelid three times a day. In more severe cases, a further application of PS may be applied about 0.5 hours after each application. The PS may have a long-lasting analgesic effect and thus can be administered less frequently. For example, the PS can be administered topically less than once a day, for example once every other day. Indeed, for those patients experiencing long-term analgesia with a single administration, the PS may be administered topically less than once a week, for example once a fortnight.

In some embodiments of topical administration to the ocular surface and/or the outer surface of the eyelid, the PS may have a long-lasting analgesic effect and thus can be administered less frequently. For example, the PS can be administered topically to the ocular surface and/or the outer surface of the eyelid less than once a day, for example once every other day. Indeed, for those patients experiencing long-term analgesia with a single administration, the PS may be administered topically to the ocular surface and/or the outer surface of the eyelid less than once a week, for example once a fortnight.

For topical administration of some pharmaceutical compositions, it is useful to cover the affected area, for example with a dressing (*e.g.,* a plastic wrap or film), after the pharmaceutical composition has been applied, for example to ensure appropriate amount of the composition can be applied for an appropriate time. Therefore, after topical application of the PS, the affected area may be dressed.

In some embodiments, the PS may be administered topically in the form of a patch, for example a medicated plaster. The use of a patch may allow the dosing interval and/or dosing frequency to be reduced, for example due to the patch ensuring controlled release of the PS. Accordingly, the patch may be applied to the affected area once a day.

In some embodiments of topical administration to the outer surface of the eyelid, the PS may be administered topically to the outer surface of the eyelid in the form of a patch, for example a medicated plaster. The use of a patch may allow the dosing interval and/or dosing frequency to be reduced, for example due to the patch ensuring controlled release of the PS. Accordingly, the patch may be applied to the outer surface of the eyelid once a day.

PS may be administered orally at dosage levels of about 0.01 mg/kg to about 100 mg/kg, about 0.05 mg/kg to about 50 mg/kg, or about 0.1 mg/kg to about 10 mg/kg of subject body weight. In particular embodiments, the PS may be administered at dosage levels of about 1 mg/kg to about 5 mg/kg, for example about 3 mg/kg of subject body weight.

PS may be administered orally at a dosage of about 1 mg to about 2000 mg. In some embodiments, the PS may be administered orally at a dosage of about 100 mg to 1500 mg, for example about 200 mg to about 1000 mg. In some embodiments, the PS may be administered orally at a dosage of about 50 mg to about 400 mg, for example about 100 mg to about 350 mg, for example about 150 mg to about 300 mg, for example about 150mg to about 250 mg. In particular embodiments, PS is administered orally at a dosage of about 250 mg to about 300 mg, preferably about 250 mg. In some embodiments of multiple dosing, equal amount of PS may be administered in each dose. In other embodiments, a higher initial dose may be administered, followed by low maintenance doses.

In some embodiments, PS may be administered orally once a day, or more frequently. For example, PS may be administered twice a day, three times a day, four times a day, or more often as necessary. In particular embodiments, PS may be administered orally two or three times a day.

In particular embodiments, PS is administered orally at a dosage of about 150 mg to about 200 mg twice a day. Accordingly, a subject may be administered PS orally at a daily dosage of about 300 mg to about 400 mg.

In particular embodiments, PS is administered orally at a dosage of about 250 mg to about 300 mg (for example, about 250 mg) two or three times a day. Accordingly, a subject may be administered PS orally at a daily dosage of about 500 mg to up to about 900 mg a day.

The administration of the PS may continue as long as necessary. For example, the PS may be administered for more than 1, 2, 3, 4, 5, 6, 7, 14, 28, 56, or 84 days. As noted above, the PS can be administered chronically on an ongoing basis for the treatment of chronic effects, for example for at least 3 months. Accordingly, in some cases, continuous dosing is achieved and maintained as long as necessary. The PS may be administered intermittently according to the recurrence of the pain associated with the indications described herein and/or associated sensory symptoms.

The PS can be used for the treatment (and prevention) of pain associated with the indications described herein in mammals. For example the subject may be a human.

As noted above, PS can be formulated into an appropriate pharmaceutical composition for administering to subjects with any one of the indications described herein (*e.g.,* pain associated with central sensitization, PTPN, PHN, migraine (other other headache disorders), or corneal neuropathic pain). Accordingly, the PS may be administered according to the dosing regimens above in an appropriate pharmaceutical composition. In particular embodiments, PS or the appropriate pharmaceutical composition of PS is administered as a monotherapy.

The dosing regimens disclosed herein are suitable for treating corneal pain, for example corneal pain caused by central sensitization and/or corneal pain generated at central sites of action. Indeed, the dosing regimens disclosed herein are applicable for use in any of the methods of treatment disclosed herein.

A person having ordinary skill in the art understands that, in certain embodiments, dosages of such compounds may be adjusted depending upon the mammal to be treated. For example, the treatment of mice is described herein and such dosages may or may not be revised upon the administration of PS to a human. However, a person having ordinary skill in the art may, if necessary, convert the dosages provided herein as set forth in Guidance for Industry: Estimating the Maximum Safe Starting Dose in Initial Clinical Trials for Therapeutics in Adult Healthy Volunteers, U.S. Department of Health and Human Services, Food and Drug Administration, Center for Drug Evaluation and Research (CDER), July 2005. A human equivalent dose (HED) may be determined from an animal dose, the animal dose may be multiplied by the following conversion factors, to provide units in mg/kg: mouse = 0.08, hamster = 0.13, rat = 0.16, ferret = 0.19, guinea pig = 0.22, rabbit = 0.32, dog = 0.54, monkey = 0.32, marmoset = 0.16, squirrel monkey = 0.19, baboon = 0.54, micro-pig = 0.73, and mini-pig = 0.95.

### Pharmaceutically acceptable forms of PS

The pharmaceutical composition comprising PS can contain a pharmaceutically acceptable form of PS. The pharmaceutically acceptable form may be a solvate, derivative, and/or prodrug.

### Solvates

As used herein, the term "solvate" refers to a compound that further includes a stoichiometric or non-stoichiometric amount of solvent bound by non-covalent intermolecular forces. Where the solvent is water, the solvate is a hydrate. The pharmaceutically acceptable form of PS may include a solvate of PS, for example a solvate of PS-I and/or PS-II. In some embodiments, the solvate includes at least 1 molecule of solvent. In some embodiments, the solvate includes less than 1 molecule of solvent. In some embodiments, the solvate is a hydrate.

### Isotopes

The pharmaceutically acceptable form of PS may include an isotopically labelled derivative of PS-I. The pharmaceutically acceptable form of PS may include an isotopically labelled derivative of PS-II. An isotopically labelled derivative is a compound that is identical to PS, except that one or more atoms are replaced by an atom having an atomic mass or mass number different from the atomic mass or mass number usually found in nature. In some embodiments, the isotopically labelled derivative of PS includes one or more isotopes of hydrogen, carbon, oxygen, phosphorus, and fluorine. In some embodiments, the isotopically labelled derivative of PS includes one or more isotopes of ²H, ³H, ¹³C, ¹⁴C, ¹⁸O, ¹⁷O, ³¹P, ³²P, ³⁵S, and ¹⁸F, respectively. In some embodiments, the isotopically labelled derivative of PS includes one or more isotopes of ²H (e.g., deuterium). In some embodiments, the isotopically labelled derivative of PS includes one or more isotopes of ³H (e.g., tritium). In some embodiments, the isotopically labelled derivative of PS includes one or more isotopes of ¹⁴C.

### Derivatives and prodrugs

The pharmaceutically acceptable form of PS may include a derivative of PS-I. The pharmaceutically acceptable form of PS may include a derivative of PS-II. In some embodiments, the derivative of PS (e.g., PS-I or PS-II) is a metabolite. In other embodiments, the pharmaceutically acceptable form of PS is a prodrug of PS, for example a prodrug of PS-I or a prodrug of PS-II.

A sulfone group can be structurally expressed as: R-S(=O)₂-R'. In some embodiments, the derivative of PS is a sulfone form of PS.

PS contains an organophosphate functional group. An organophosphate functional group can be structurally expressed as O=P(OR)₃, O=P(OR)₂(OR'), or O=P(OR)(OR')(OR"). For example, O=P(OR)₂(OR') can represent PS if R = CH₂CH₃ and R' = the remainder of the molecule is as per PS in formula I or II (e.g., PS-I, PS-II, or a derivative thereof).

In some embodiments, the derivative of PS is PS wherein one of the ethoxy (e.g. -OCH₂CH₃) groups is an OH group, or a pharmaceutically acceptable salt thereof. In some embodiments, the derivative of PS is PS wherein both ethoxy (e.g. -OCH₂CH₃) groups are OH groups, or a pharmaceutically acceptable salt thereof.

The activity of PS demonstrated herein would be shared by pharmaceutically acceptable forms thereof. Therefore, the present invention provides pharmaceutically acceptable forms of PS for use in the methods of the invention.

While preferred embodiments of the invention are shown and described herein, such embodiments are provided by way of example only and are not intended to otherwise limit the scope of the invention. Various alternatives to the described embodiments of the invention may be employed in practicing the invention.

### NUMBERED EMBODIMENTS

The invention further provides the following numbered embodiments.
1. A method of treating pain associated with central sensitization comprising administering a therapeutically effective amount of phosphosulindac (PS) to a subject in need thereof such that pain associated with central sensitization is treated.
2. The method of embodiment 1, wherein treating the pain comprises reducing the pain.
3. The method of any one of the preceding embodiments, wherein treating the pain includes reducing one or more of the symptoms associated with central sensitization.
4. The method of embodiment 3, wherein the one or more symptom is selected from mood changes, fatigue, cognitive disturbances, sleep changes, pain catastrophizing, memory complaints, depression, anxiety, photophobia, and/or phonophobia.
5. The method of any one of the preceding embodiments, wherein PS reduces the neuronal signalling involved in the sensation of pain.
6. The method of any one of the preceding embodiments, wherein PS reduces pain signalling occurring centrally.
7. The method of any one of the preceding embodiments, wherein the PS reduces pain signalling occurring in the spinal cord dorsal horn.
8. The method of any one of the preceding embodiments, wherein PS reduces pain signalling occurring in the CNS.
9. The method of any one of the preceding embodiments, wherein the pain is allodynia.
10. The method of embodiment 9, wherein the allodynia is mechanical allodynia and/or thermal allodynia.
11. The method of any one of the preceding embodiments, wherein the pain is hyperalgesia.
12. A method of treating and/or preventing neuropathic pain associated with post-traumatic peripheral neuropathy (PTPN) comprising administering a therapeutically effective amount of phosphosulindac (PS) to a subject in need thereof such that neuropathic pain associated with PTPN is treated and/or prevented.
13. The method of embodiment 12, wherein treating the neuropathic pain comprises reducing the neuropathic pain.
14. The method of embodiment 12 or 13, wherein preventing the neuropathic pain comprises decreasing the incidence of the neuropathic pain.
15. The method of any one of embodiments 12-14, wherein treating the neuropathic pain includes reducing one or more of the sensory symptoms associated with PTPN.
16. The method of any one of embodiments 12-15, wherein preventing the neuropathic pain includes decreasing the incidence of one or more of the sensory symptoms associated with PTPN.
17. The method of embodiment 15 or 16, wherein the one or more sensory symptom is selected from paresthesia, burning sensations and stabbing sensations.
18. The method of embodiment 17, wherein the paresthesia includes one or more of numbness, tingling, pricking, or formication.
19. The method of any one of embodiments 12-18, wherein PS reduces the neuronal signalling involved in the sensation of pain.
20. The method of any one of embodiments 12-19, wherein PS reduces pain generated via peripheral sensitization.
21. The method of any one of embodiments 12-20, wherein PS reduces pain generated via central sensitization.
22. The method of any one of embodiments 12-21, wherein PS reduces pain signalling occurring centrally.
23. The method of any one of embodiments 12-22, wherein the PS reduces pain signalling occurring in peripheral nerves.
24. The method of any one of embodiments 12-23, wherein PS reduces pain signalling occurring in the dorsal root ganglion.
25. The method of any one of embodiments 12-24, wherein PS reduces pain signalling occurring in the spinal cord dorsal horn.
26. The method of any one of embodiments 12-25, wherein the neuropathic pain is allodynia.
27. The method of embodiment 26, wherein the allodynia is mechanical allodynia and/or thermal allodynia.
28. The method of any one of embodiments 12-27, wherein the neuropathic pain is hyperalgesia.
29. The method of any one of embodiments 12-28, wherein the neuropathic pain associated with PTPN is caused by neurapraxia, for example a nerve compression injury.
30. The method of any one of embodiments 12-29, wherein the neuropathic pain associated with PTPN is caused by axonotmesis, for example a nerve crush injury.
31. The method of any one of embodiments 12-30, wherein the neuropathic pain associated with PTPN is caused by one or more of the following: carpal tunnel syndrome; pronator teres syndrome; radial tunnel syndrome; suprascapular nerve entrapment; thoracic outlet syndrome; ulnar nerve entrapment (cubital tunnel syndrome or Guyon's canal syndrome); meralgia paresthetica; peroneal nerve compression; pudendal nerve entrapment syndrome; sciatica; tarsal tunnel syndrome; herniated cervical disc; herniated thoracic disc; and/or herniated lumbar disc.
32. A method of treating and/or preventing migraine pain comprising administering a therapeutically effective amount of phosphosulindac (PS) to a subject in need thereof such that migraine pain is treated and/or prevented.
33. The method of embodiment 32, wherein treating the pain comprises reducing the pain, for example pulsating head pain.
34. The method of embodiment 32 or 33, wherein preventing the pain comprises decreasing the incidence of the pain, for example pulsating head pain.
35. The method of any one of embodiments 32-34, wherein treating the pain includes reducing one or more of the symptoms associated with migraine.
36. The method of any one of embodiments 32-35, wherein preventing the pain includes decreasing the incidence of one or more of the symptoms associated with migraine.
37. The method of embodiment 35 or 36, wherein the one or more symptom is selected from aura, nausea, vomiting, photophobia, phonophobia and/or cranial autonomic symptoms.
38. The method of embodiment 37, wherein the aura includes one or more sensory disturbances, for example visual symptoms, pins and needles (tingling), and/or numbness.
39. The method of embodiment 37 or 38, wherein the cranial autonomic symptom eye redness or tearing.
40. The method of any one of embodiments 32-39, wherein the subject experiences cutaneous allodynia.
41. The method of any one of embodiments 32-40, wherein PS reduces the neuronal signalling involved in the sensation of pain.
42. The method of any one of embodiments 32-41, wherein PS reduces pain generated via peripheral sensitisation.
43. The method of any one of embodiments 32-42, wherein PS reduces pain generated via central sensitisation.
44. The method of any one of embodiments 32-43, wherein PS reduces pain signalling occurring centrally.
45. The method of any one of embodiments 32-44, wherein the PS reduces pain signalling occurring in the trigeminal nerve.
46. The method of any one of embodiments 32-45, wherein PS reduces pain signalling occurring in the trigeminal ganglion.
47. The method of any one of embodiments 32-46, wherein PS reduces pain signalling occurring in the trigeminal nucleus caudalis.
48. The method of any one of embodiments 32-47, wherein PS reduces pain signalling occurring in higher order neurons and/or pain sensing regions of the brain, for example trigeminothalamic neurons.
49. The method of any one of embodiments 32-48, wherein the pain is allodynia, for example cutaneous allodynia.
50. The method of embodiment 49, wherein the allodynia is mechanical allodynia and/or thermal allodynia.
51. The method of any one of embodiments 32-50, wherein the pain is hyperalgesia.
52. The method of any one of embodiments 32-51, wherein the migraine is episodic migraine or chronic migraine.
53. The method of any one of embodiments 32-52, wherein the migraine is migraine with aura or migraine without aura.
54. A method of treating and/or preventing neuropathic pain associated with post-herpetic neuralgia (PHN) comprising administering a therapeutically effective amount of phosphosulindac (PS) to a subject in need thereof such that neuropathic pain associated with PHN is treated and/or prevented.
55. The method of embodiment 54, wherein treating the neuropathic pain comprises reducing the neuropathic pain.
56. The method of embodiment 54 or 55, wherein preventing the neuropathic pain comprises decreasing the incidence of the neuropathic pain.
57. The method of any one of embodiments 54-56, wherein treating the neuropathic pain includes reducing one or more of the sensory symptoms associated with PHN.
58. The method of any one of embodiments 54-57, wherein preventing the neuropathic pain includes decreasing the incidence of one or more of the sensory symptoms associated with PHN.
59. The method of any one of embodiments 54-58, wherein the neuropathic pain is a sharp, burning, throbbing or stabbing.
60. The method of any one of embodiments 57-59, wherein the one or more sensory symptom is selected from itching or numbness.
61. The method of any one of embodiments 54-60, wherein PS reduces the neuronal signalling involved in the sensation of pain.
62. The method of any one of embodiments 54-61, wherein PS reduces pain generated via peripheral sensitization.
63. The method of any one of embodiments 54-62, wherein PS reduces pain generated via central sensitization.
64. The method of any one of embodiments 54-63, wherein PS reduces pain signalling occurring centrally.
65. The method of any one of embodiments 54-64, wherein the PS reduces pain signalling occurring in peripheral nerves, for example nerves innervating one or more dermatomes.
66. The method of any one of embodiments 54-65, wherein PS reduces pain signalling occurring in one or more spinal nerves, for example one or more cervical nerves, one or more thoracic nerves, one or more lumbar nerves, and/or one or more sacral nerves.
67. The method of any one of embodiments 54-66, wherein PS reduces pain signalling occurring in the dorsal root ganglion.
68. The method of any one of embodiments 54-67, wherein PS reduces pain signalling occurring in the spinal cord dorsal horn.
69. The method of any one of embodiments 54-68, wherein the neuropathic pain is allodynia.
70. The method of embodiment 69, wherein the allodynia is mechanical allodynia and/or thermal allodynia.
71. The method of any one of embodiments 54-70, wherein the neuropathic pain is hyperalgesia.
72. The method of any one of the preceding embodiments, wherein the subject is human.
73. The method of any one of the preceding embodiments, wherein PS has the formula I (PS-I): 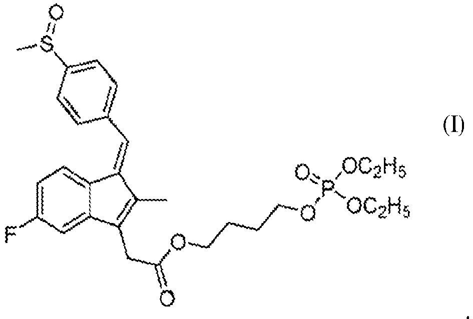
74. The method of any one of the preceding embodiments, wherein PS has the formula II (PS-II): 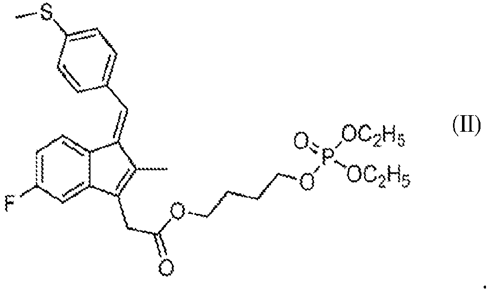
75. The method of any one of the preceding embodiments, wherein the therapeutically effective amount of PS is administered as a pharmaceutical composition further comprising a pharmaceutically acceptable excipient.
76. The method of embodiment 75, wherein the pharmaceutical composition comprising PS is formulated for topical administration.
77. The method of embodiment 76, wherein the pharmaceutical composition comprising PS is formulated as a semi-solid.
78. The method of embodiment 76, wherein the pharmaceutical composition comprising PS is formulated as a liquid.
79. The method of any one of embodiments 76-78, wherein the pharmaceutical composition comprising PS is a cream.
80. The method of any one of embodiments 76-78, wherein the pharmaceutical composition comprising PS is a gel, for example wherein the gel is a hydrogel.
81. The method of any one of embodiments 76-78, wherein the pharmaceutical composition comprising PS is a lotion.
82. The method of any one of embodiments 76-78, wherein the pharmaceutical composition comprising PS is an ointment.
83. The method of any one of embodiments 76-78, wherein the pharmaceutical composition comprising PS is a spray.
84. The method of embodiment 76, wherein the pharmaceutical composition comprising PS is formulated as a patch.
85. The method of any one of embodiments 75-84, wherein the pharmaceutical composition comprises PS at a concentration of about 0.5% to about 15% w/w of the pharmaceutical composition.
86. The method of embodiment 85, wherein the pharmaceutical composition comprises PS is at a concentration of about 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition.
87. The method of embodiment 86, wherein the pharmaceutical composition comprises PS at a concentration of less than or equal to 8% w/w of the pharmaceutical composition, for example about 5% or about 3% w/w of the pharmaceutical composition.
88. The method of embodiment 86, wherein the pharmaceutical composition comprises PS at a concentration of less than or about equal to 3% w/w of the pharmaceutical composition, for example about 2% or about 1% w/w of the pharmaceutical composition.
89. The method of any of embodiments 75-88, wherein the PS is administered at about 0.005 g/10cm² to about 0.25 g/10cm² of affected area.
90. The method of embodiment 89, wherein the PS is administered at about 0.005 g/10cm² of affected area.
91. The method of embodiment 89, wherein the PS is administered at about 0.01 g/10cm² of affected area.
92. The method of embodiment 89, wherein the PS is administered at about 0.05 g/10cm² of affected area.
93. The method of embodiment 89, wherein the PS is administered at about 0.1 g/10cm² of affected area.
94. The method of embodiment 89, wherein the PS is administered at about 0.15 g/10cm² of affected area.
95. The method of embodiment 89, wherein the PS is administered at about 0.2 g/10cm² of affected area.
96. The method of embodiment 89, wherein the PS is administered at about 0.25 g/10cm² of affected area.
97. The method of any one of embodiments 75-96, wherein the PS is applied to the affected area and left on the affected area for between about 1 hour and about 5 hours.
98. The method of embodiment 97, wherein the PS is applied to the affected area and left on the affected area for about 0.5 hours, for about 1 hour, for about 2 hours, for about 3 hours, for about 4 hours, or for about 5 hours.
99. The method of embodiment 97 or 98, wherein the PS is removed from the affected area after the dosing period, for example by washing off.
100. The method of embodiment 97 or 98, wherein a second or further application of PS is applied to the affected area after the dosing period.
101. The method of any one of embodiments 75-100, wherein the PS is applied once a day.
102. The method of any one of embodiments 75-100, wherein the PS is applied twice a day.
103. The method of any one of embodiments 75-100, wherein the PS is applied three times a day.
104. The method of any one of embodiments 75-100, wherein the PS is applied four times a day.
105. The method of any one of embodiments 75-104, wherein the PS is administered in a pharmaceutical composition.
106. A method of treating corneal neuropathic pain comprising administering a therapeutically effective amount of phosphosulindac (PS) to a subject in need thereof such that corneal neuropathic pain is treated.
107. The method of embodiment 106, wherein treating the corneal neuropathic pain comprises reducing the corneal neuropathic pain.
108. The method of embodiment 106 or 107, wherein treating the corneal neuropathic pain includes reducing one or more of the symptoms associated with corneal neuropathic pain.
109. The method of embodiment 108, wherein the one or more symptom is selected from paresthesia, photosensitivity, photoallodynia, anxiety, depression or apathy.
110. The method of embodiment 109, wherein the paresthesia includes one or more of numbness, tingling, pricking, or formication.
111. The method of any one of embodiments 106-110, wherein PS reduces pain generated via central sensitization.
112. The method of any one of embodiments 106-111, wherein PS reduces pain signalling occurring centrally.
113. The method of any one of embodiments 106-112, wherein PS reduces pain signalling occurring in the trigeminal ganglion.
114. The method of any one of embodiments 106-113, wherein PS reduces pain signalling occurring in the trigeminal nucleus caudalis.
115. The method of any one of embodiments 106-114, wherein PS reduces pain signalling occurring in the higher order neurons and/or pain sensing regions of the brain, for example trigeminothalamic neurons.
116. The method of any one of embodiments 106-115, wherein the corneal neuropathic pain is allodynia.
117. The method of embodiment 116, wherein the allodynia is mechanical allodynia and/or thermal allodynia.
118. The method of any one of embodiments 106-117, wherein the corneal neuropathic pain is hyperalgesia.
119. The method of any one of embodiments 106-118, wherein the subject is human.
120. The method of any one of embodiments 106-119, wherein PS has the formula I (PS-I).
121. The method of any one of embodiments 106-120, wherein PS has the formula II (PS-II).
122. The method of any one of embodiments 106-121, wherein the PS is administered to the ocular surface.
123. The method of any one of embodiments 106-122, wherein the PS is administered to the outer surface of one or more eyelids, for example wherein the one or more eyelids are one or both upper eyelids and/or one or both lower eyelids.
124. The method of any one of embodiments 106-123, wherein the therapeutically effective amount of PS is administered as a pharmaceutical composition further comprising a pharmaceutically acceptable excipient.
125. The method of embodiment 124, wherein the pharmaceutical composition comprising PS is formulated for topical administration.
126. The method of embodiment 125, wherein the topical administration is to the ocular surface.
127. The method of embodiment 125, wherein the topical administration is to the outer surface of one or more eyelids, for example wherein the one or more eyelids are one or both upper eyelids and/or one or both lower eyelids.
128. The method of embodiment 125-127, wherein the pharmaceutical composition comprising PS is formulated as a semi-solid.
129. The method of embodiment 125-127, wherein the pharmaceutical composition comprising PS is formulated as a liquid.
130. The method of embodiment 125-127, wherein the pharmaceutical composition comprising PS is a cream.
131. The method of embodiment 125-127, wherein the pharmaceutical composition comprising PS is a gel, for example wherein the gel is a hydrogel.
132. The method of embodiment 125-127, wherein the pharmaceutical composition comprising PS is a lotion.
133. The method of embodiment 125-127, wherein the pharmaceutical composition comprising PS is an ointment.
134. The method of embodiment 125-127, wherein the pharmaceutical composition comprising PS is formulated as an eye drop.
135. The method of embodiment 134, wherein the eye drop composition is an ophthalmic solution or ophthalmic emulsion for dropwise administration to the eye.
136. The method of embodiment 134 or 135, wherein the size of the drop is between about 10 and about 100 µL.
137. The method of any one of embodiments 124-136, wherein the pharmaceutical composition comprises PS at a concentration of about 0.5% to about 15% w/w of the pharmaceutical composition.
138. The method of embodiment 137, wherein the pharmaceutical composition comprises PS is at a concentration of about 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition.
139. The method of embodiment 138, wherein the pharmaceutical composition comprises PS at a concentration of less than or equal to 8% w/w of the pharmaceutical composition, for example about 5% or about 3% w/w of the pharmaceutical composition.
140. The method of embodiment 139, wherein the pharmaceutical composition comprises PS at a concentration of less than or about equal to 3% w/w of the pharmaceutical composition, for example about 2% or about 1% w/w of the pharmaceutical composition.
141. The method of any of embodiments 124-140, wherein the PS is administered at about 0.005 mg/cm² to about 0.25 mg/cm² of ocular surface.
142. The method of embodiment 141, wherein the PS is administered at about 0.005 mg/cm², 0.01 mg/cm², 0.05 mg/cm², 0.1 mg/cm², 0.15 mg/cm², 0.2 mg/cm², or 0.25 mg/cm² of ocular surface.
143. The method of any of embodiments 124-140, wherein the PS is administered at about 0.5 mg/cm² to about 100 mg/cm² of the outer surface of the eyelid.
144. The method of embodiment 143, wherein the PS is administered at about 0.5 mg/cm², 5 mg/cm², 10 mg/cm², 25 mg/cm², 50 mg/cm², 75 mg/cm² or 100 mg/cm² of the outer surface of the eyelid.
145. The method of any one of embodiments 124-144, wherein the PS is applied to the ocular surface and/or the outer surface of the eyelid, and left on the the ocular surface and/or the outer surface of the eyelid for between about 1 hour and about 5 hours.
146. The method of embodiment 145, wherein the PS is applied to the ocular surface and/or the outer surface of the eyelid, and left on the ocular surface and/or the outer surface of the eyelid for about 0.5 hours, for about 1 hour, for about 2 hours, for about 3 hours, for about 4 hours, or for about 5 hours.
147. The method of embodiment 145 or 146, wherein the PS is removed from the outer surface of the eyelid after the dosing period, for example by washing off.
148. The method of any one of embodiments 145-147, wherein a second or further application of PS is applied to the ocular surface and/or the outer surface of the eyelid after the dosing period.
149. The method of any one of embodiments 124-148, wherein the PS is applied once a day.
150. The method of any one of embodiments 124-148, wherein the PS is applied twice a day.
151. The method of any one of embodiments 124-148, wherein the PS is applied three times a day.
152. The method of any one of embodiments 124-148, wherein the PS is applied four times a day.
153. The method of any one of embodiments 141-152, wherein the PS is administered to the ocular surface and/or the outer surface of the eyelid in a pharmaceutical composition.
154. A method of treating and/or preventing an eye disease or condition comprising administering a therapeutically effective amount of phosphosulindac (PS) to a subject in need thereof such that the eye disease or condition is treated and/or prevented, wherein the PS is topically administered to the outer surface of one or more eyelids.
155. The method of embodiment 154, wherein the one or more eyelids is one or both upper eyelids.
156. The method of embodiment 154, wherein the one or more eyelids is one or both lower eyelids.
157. The method of any one of embodiments 154-156, wherein the eye disease or condition is an inflammatory eye disease or condition.
158. The method of any one of embodiments 154-157, wherein the eye disease or condition is dry eye disease (DED).
159. The method of any one of embodiments 154-158, wherein the eye disease or condition is corneal pain, for example corneal neuropathic pain.
160. A method of treating and/or preventing pain comprising administering a therapeutically effective amount of PS to a subject in need thereof such that the pain is treated and/or prevented.
161. PS for use in treating pain associated with central sensitization.
162. PS for use in treating and/or preventing migraine pain in a subject.
163. PS for use in treating and/or preventing neuropathic pain associated with PTPN.
164. PS for use in treating and/or preventing neuropathic pain associated with PHN.
165. PS for use in treating corneal neuropathic pain.
166. PS for use in treating and/or preventing an eye disease or condition, wherein the PS is topically administered to the outer surface of one or more eyelids.
167. PS for use in treating and/or preventing pain.
168. Use of PS for the manufacture of a medicament for treating pain associated with central sensitization.
169. Use of PS for the manufacture of a medicament for treating and/or preventing neuropathic pain associated with PTPN.
170. Use of PS for the manufacture of a medicament for treating and/or preventing migraine pain in a subject.
171. Use of PS for the manufacture of a medicament for treating and/or preventing neuropathic pain associated with PHN.
172. Use of PS for the manufacture of a medicament for treating corneal neuropathic pain.
173. Use of PS for the manufacture of a medicament for treating and/or preventing an eye disease or condition, wherein the PS is topically administered to the outer surface of one or more eyelids.
174. Use of PS for the manufacture of a medicament for treating and/or preventing pain.
175. The method of any one of embodiments 1-75, 106-121 and 154-160 , wherein the PS is administered orally.
176. PS for use of any one of embodiments 161-167, wherein the PS is administered orally.
177. The use of any one of embodiments 168-174, wherein the PS is administered orally.
178. A method of treating and/or preventing neuropathic pain associated with CIPN comprising administering a therapeutically effective amount of PS to a subject in need thereof such that the neuropathic pain associated with CIPN is treated and/or prevented, wherein the PS is administered orally.
179. A method of treating and/or preventing neuropathic pain associated with DPN comprising administering a therapeutically effective amount of PS to a subject in need thereof such that the neuropathic pain associated with DPN is treated and/or prevented, wherein the PS is administered orally.
180. The method, PS for use, or use of any one of embodiments 175-179, wherein the PS is formulated as a liquid or solid dosage form.
181. The method, PS for use, or use of embodiment 180, wherein the liquid dosage form is a pharmaceutically acceptable emulsion, microemulsion, solution, suspension, syrup or and elixir.
182. The method, PS for use, or use of embodiment 180, wherein the solid dosage form is a capsule, tablet, pill, powder, or granule.
183. The method, PS for use, or use of any one of embodiments 175-182, wherein the PS is administered orally at dosage levels of about 0.01 mg/kg to about 100 mg/kg, from about 0.05 mg/kg to about 50 mg/kg, or from about 0.1 mg/kg to about 10 mg/kg of subject body weight, for example about 1 mg/kg to about 5 mg/kg, for example about 3 mg/kg of subject body weight.
184. The method, PS for use, or use of any one of embodiments 175-183, wherein the PS is administered orally at a dosage of about 1 mg to about 2000 mg, of about 100 mg to 1500 mg, of about 200 mg to about 100 mg, of about 50 mg to about 400 mg, for example about 100 mg to about 350 mg, for example about 150 mg to about 300 mg, for example about 150mg to about 250 mg.
185. The method, PS for use, or use of any one of embodiments 175-184, wherein the PS is administered orally at a dosage of about 250 mg to about 300 mg, preferably about 250 mg.
186. The method, PS for use, or use of any one of embodiments 175-185, wherein the PS is administered orally once a day.
187. The method, PS for use, or use of any one of embodiments 175-186, wherein the PS is administered orally at least twice a day, at least three times a day, or at least four times a day.
188. The method, PS for use, or use of any one of embodiments 175-187, wherein the PS is administered orally two or three times a day.
189. The method, PS for use, or use of any one of embodiments 175-188, wherein the PS is administered orally at a dosage of from about 150 mg to about 200 mg twice a day.
190. The method, PS for use, or use of any one of embodiments 175-189, wherein the PS is administered orally at a daily dosage of about 300 mg to about 400 mg.
191. The method, PS for use, or use of any one of embodiments 175-190, wherein the PS is administered orally at a daily dosage of about 250 mg to about 300 mg (for example, about 250 mg) two or three times a day.
192. The method, PS for use, or use of any one of embodiments 175-191, wherein the PS is administered orally at a daily dosage of about 500 mg to up to about 900 mg a day.
193. The method of any one of claims 175-192, wherein the PS is administered orally in a pharmaceutical composition.

### EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### Example 1: The effect of PS in treating pain associated with central sensitization

### Methods

Central sensitization was established by administration of 10 mg/kg paclitaxel intraperitoneally into C57/BL mice. The paclitaxel was administered into all study groups once a day for 3 days.

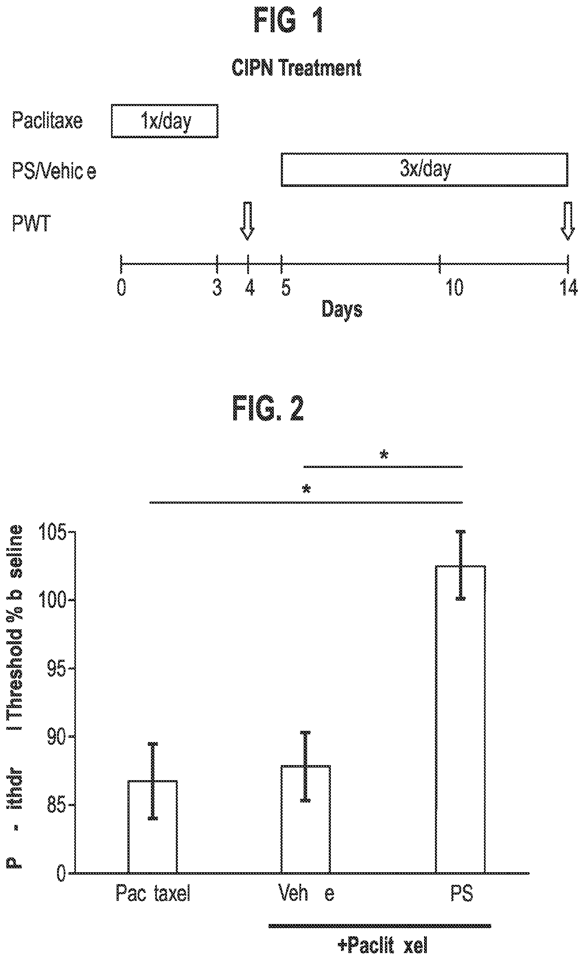

The results of Figure 2 demonstrate that paclitaxel causes a significant decrease in PWT compared to naive mice.

PS (as an 8% hydrogel) or vehicle control were administered topically to both hind paws of the mice 3 times a day for 10 days. The first doses were administered 2 days after the last administration of paclitaxel.

The study groups were as follows:
1. Group 1: paclitaxel only (*n*=9)
2. Group 2: paclitaxel plus vehicle (*n*=10)
3. Group 3: paclitaxel plus PS (*n*=10)

In order to determine the outcome of the treatment, pain threshold responses were measured using the well-established method of von Frey filaments. In particular, a simplified up-down method for estimating paw withdrawal threshold (PWT) using von Frey filaments was used (as described in Bonin et al., Molecular Pain (2014); 10(26):1-10)). The results of the PWT test are expressed as force applied (gm). The PWT test was performed at baseline *(i.e.,* 4 days after the first dose of paclitaxel administration (day -1)) and then on the final day of treatment with PS or vehicle *(i.e.,* day 10), about 30 minutes after the last application. The data is expressed as percent change from the respective baseline value.

Figure 1 provides an outline of the study.

### Results

As displayed in Figure 2, the administration of vehicle in the paclitaxel background has limited effect on the PWT compared to treatment with paclitaxel only, while administration of PS achieved a significant increase in PWT compared to vehicle and paclitaxel alone. The values, corresponding to Figure 2, are provided in Table 1.

**Table 1 - PWT in the 3 study groups**

| **Groups** | **PWT, % baseline** | **P values** |
|---|---|---|
| | *Mean ± SEM* | |
| Paclitaxel only | 86.75 ± 5.42 | |
| Paclitaxel + vehicle | 87.77 ± 5.02 | |
| Paclitaxel + PS | 102.55 ± 4.92 | *p<0.04 *vs* vehicle |
| | | *p<0.03 *vs* paclitaxel alone |

### Conclusions

The topical administration of PS significantly increased the PWT in mice with established pain associated with central sensitization. Therefore, PS treats the pain associated with central sensitization.

Contrary to the observations with typical NSAIDs, PS demonstrates a pain-relieving effect in a treatment model of pain associated with central sensitization. This striking activity of PS, in a specific animal model, supports observations that unlike typical NSAIDs, PS can effectively treat pain associated with central sensitization.

### Example 2: PS effectively treats pain associated with central sensitization caused by multiple different chemotherapeutics

### Methods

### Animals

Adult male C57BL/6J mice, 8 weeks of age at the beginning of the experiments and weighing 20-30 g, were purchased from The Jackson Laboratory (Bar Harbor, ME). Mice were housed in an AAALAC-accredited facility in groups of four. Food and water were available *ad libitum.* The mice in each cage were randomly allocated to treatment groups. All studies were conducted by experimenters blinded to the identity of the treatment groups. Experiments were performed during the light cycle (7:00 am to 7:00 pm) and animals were euthanized with CO₂ asphyxiation. Studies were approved by the relevant Institutional Animal Care and Use Committee and followed the National Institutes of Health Guidelines for the Care and Use of Laboratory Animals. Animal studies are reported in compliance with the ARRIVE guidelines.

### Phosphosulindac

PS was formulated as an 8% hydrogel ointment for topical administration.

### Induction of pain associated with central sensitization

Central sensitization was induced in mice with three different chemotherapeutic compounds using established protocols (Carozzi et al., Exp Neurol (2010); 226:301-309; Currie et al., PLoS Biol (2019); 17:e3000243; Eldridge et al., Toxicol Pathol (2020); 48:190-201). Each of the three chemotherapeutic compounds were prepared and dosed as follows.
*Paclitaxel:* paclitaxel (purchased from MilliporeSigma (St. Louis, MO)) was dissolved in a mixture of 1 volume ethanol / 1 volume Cremophor EL (EMD Millipore Corp, Burlington, MA) / 18 volumes distilled water. Paclitaxel was administered as four intraperitoneal injections of 8 mg/kg paclitaxel (in a volume of 1 ml/100 g body weight) every other day, resulting in a cumulative dose of 32 mg/kg.
*Oxaliplatin:* Oxaliplatin was dissolved in ddH₂O. Oxaliplatin 3 mg/kg was injected intraperitoneally daily for 5 days, followed by 5 days of no treatment, which was followed by another 5-day period of daily oxaliplatin intraperitoneal injections as previously for a total of ten injections leading to a cumulative dose of 30 mg/kg. All injections were administered intraperitoneally in a volume of 1 ml/100 g body weight.
*Vincristine:* Vincristine was dissolved in PBS. Two intraperitoneal injections of vincristine 1.5 mg/kg were made within one week for a total cumulative dose of 3 mg/kg. All injections were administered intraperitoneally in a volume of 1 ml/100 g body weight.

### Protocol for the treatment of established pain associated with central sensitization with PS

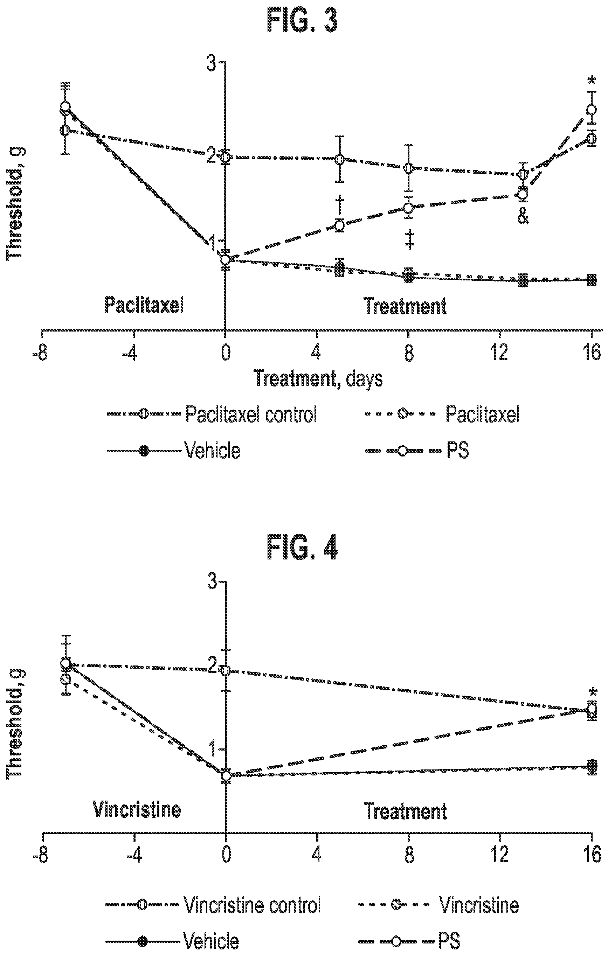

Once pain associated with central sensitization was established documented by reduced mechanical allodynia threshold, PS 8% or placebo hydrogel ointment was applied three times daily to the hind paws of the mice for the duration of the assessment period (see Figures 3-5). Mechanical allodynia was measured at the time points recorded in the figures.

### Assessment of mechanical allodynia (von Frey test)

Mechanical allodynia thresholds were determined using von Frey filaments according to an established method (Chaplan et al., J Neurosci Methods (1994); 53:55-63; Bagdas et al., Biochem Pharmacol (2015); 97:590-600). Briefly, mice were placed in a quiet room for 30 min and then were put in a Plexiglas cage with mesh metal flooring and allowed to acclimatise for 30 min before testing. A series of calibrated von Frey filaments (Stoelting, Wood Dale, IL) with incremental stiffness were applied perpendicularly to the paw with sufficient force to cause slight bending and held 2-3 s. This process was repeated at each level of stiffness 5 times, a few seconds apart. Paw withdrawn, licking or shaking were considered positive responses. The mechanical threshold, expressed as g, indicates the force of the von Frey filament to which the animal reacted.

### Statistical analysis

Results are expressed as mean ± SEM. PK parameters were calculated by Microsoft Excel and PKSolver. Non-compartmental analyses were employed. Analysis of variance (ANOVA) tests were conducted and followed by the Bonferroni post hoc test. Differences were determined to be significant at P<0.05.

### Results

The effect of PS in mice with pain associated with central sensitization caused by three different chemotherapeutic compounds was assessed. This reflects the clinical situation considered in Example 1 already, in which patients present with chronic pain associated with central sensitization.

As shown in Figures 3-5, each of the three anticancer drugs studied induced significant neuropathic pain, evidenced by changes in mechanical allodynia. Topical treatment with PS 8% ointment 3x/day was started after the neuropathic pain was established.

Each of the different chemotherapeutic compounds are discussed separately below.

### Paclitaxel (see Figure 3):

At baseline, all four study groups of mice had essentially identical allodynia scores (range 2.24±0.26 g to 2.49±0.24 g; *mean* ± *SEM* for this and all subsequent values). Paclitaxel administered to three study groups over 12 days greatly reduced (~85%) their mechanical allodynia scores indicative of pain associated with central sensitization. In contrast, the control group (non-paclitaxel, non-PS) showed a minor, statistically non-significant variation in allodynia scores throughout the entire study period.

When PS was applied to the paws of mice with paclitaxel-induced pain, their allodynia score showed progressive improvement from its lowest point at the initiation of treatment returning it to its baseline on day 16 (day 0=0.79±0.08 g vs. day 16=2.49±0.18 g; p=1.6x10⁻⁶). In contrast, the vehicle-treated group showed a mild deterioration of the allodynia score (day 0=0.79±0.11 g vs. day 16=0.56±0.05 g; p=NS). The paclitaxel only treated group showed changes in the allodynia score similar to those of the vehicle group (day 0=0.78± 0.08 g vs. day 16=0.57±0.05 g; p=NS).

The difference between the PS-treated group and its vehicle control first became statistically significant on day 5 (PS=1.17±0.07 g, vehicle=0.7±0.07 g; p=0.002), with their difference increasing thereafter and becoming maximal on day 16 (PS=2.49±0.18 g, vehicle=0.56±0.05 g; p=2.1x10⁻⁷).

### Vincristine (see Figure 4):

PS improved the mechanical allodynia induced by the commonly used vincristine. In the three groups it was administered, vincristine reduced the mechanical allodynia score by 61%-65% (day -7 scores range between 1.8±0.18 g and 2.0±0.24 g vs. day 0 = 0.7±0.07 g for all; p=3.2x10⁻⁶). In contrast, the control group that received the solvent alone showed no change in allodynia during these 7 days. PS treatment of mice with vincristine-induced neuropathic pain for 16 days markedly improved allodynia scores (114% increase compared to day 0; p=1.3x10⁻⁶), with their score being identical to that of the control group (no vincristine).

The difference between the PS-treated group and its vehicle control was statistically significant on day 16 (PS=1.5±0.09 g, vehicle=0.8±0.09 g; p=8.6x10⁻⁶). There was no appreciable change in allodynia scores during the same period of time in the vehicle and vincristine alone groups (0.7±0.07 g vs. 0.8±0.09 g for both).

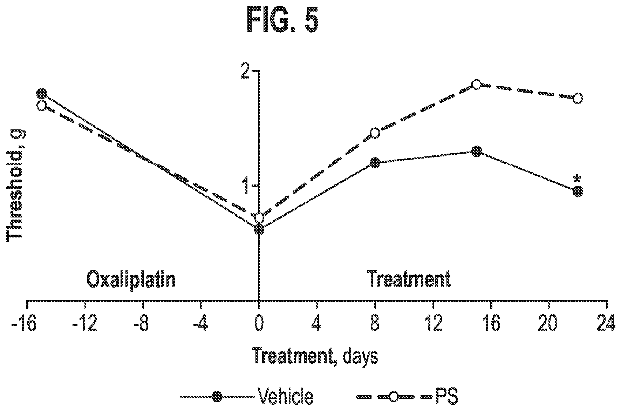

### Oxaliplatin (see Figure 5):

As expected, during oxaliplatin administration, the allodynia score was reduced by 65% and 56% in the two study groups at day 0 respectively.

PS treatment restored allodynia scores to the day -15 baseline (1.8±0.09 g vs. 1.76±0.13 g) whereas the vehicle group continued to show suppressed allodynia scores, being 47% lower on day 22 compared to day -15. The difference between the PS- and vehicle-treated groups became statistically significant on day 22 (p=0.004).

### Safety of PS

During all the studies, no topical or systemic side effects of PS ointment were observed when applied thrice daily to the hind paws of mice for up to 22 days. This finding is in keeping with the known safety profile of PS.

### Conclusions

The topical administration of PS significantly improves the mechanical allodynia score compared to that induced by three different chemotherapeutic compounds. Therefore, PS treats the pain associated with central sensitization by a range of different chemotherapies.

Consistent with the observations in Example 1, PS demonstrates a pain-relieving effect in a treatment model of pain associated with central sensitization caused by chemotherapeutic compounds from different therapeutic classes.

### Example 3: The activity of PS in treating pain associated with central sensitization is equivalent to that of lidocaine and pregabalin, but not shared by sulindac

The effect of PS was compared to that of known central acting analgesics (lidocaine and pregabalin) and PS's parent compound, sulindac, on pain associated with central sensitization. The treatment of pain protocol corresponds to that explained in Examples 1 and 2.

### Methods

The methods with respect to animals, induction of pain associated with central sensitization (via paclitaxel), assessment of mechanical allodynia and statistical analysis correspond to those outlined in Example 2.

Pain was established by administration of paclitaxel into C57/BL/6J mice. The paclitaxel was administered into all study groups below:
1. Group 1: paclitaxel plus vehicle (n=8)
2. Group 2: paclitaxel plus PS 5% (n=8)
3. Group 3: paclitaxel plus PS 1.2% (n=8)
4. Group 4: paclitaxel plus 0.7% sulindac (n=8)
5. Group 5: paclitaxel plus 5% lidocaine cream (n=8)
6. Group 6: paclitaxel plus pregabalin (10 mg/kg) (n=8)

Regarding the treatment with PS, sulindac, and vehicle: PS hydrogel 5%, PS 1.2%, 0.7% sulindac, or vehicle, was applied to both hind paws 3 times a day starting on day 0 and continued until day 15. 0.7% sulindac is the highest feasible concentration, and is equimolar to PS 1.2%.

Regarding the treatment with lidocaine: 5% lidocaine cream (a positive control) was applied once to both hind paws of the mice 30 minutes before measurement of PWT.

Regarding the treatment with pregabalin: pregabalin at 10 mg/kg, (a positive control) was administered orally once, one hour before measurement of PWT.

To determine the outcome of treatment, assessment of mechanical allodynia was performed. Additionally, in the groups of mice treated with vehicle or PS 5%, assessment of cold allodynia was performed using the acetone test. Both methods of assessing allodynia are described in Toma W, et al. Neuropharmacology 2017;117:305-15*.* Mechanical and cold allodynia are determined at least one day apart in the same animal.

Briefly, for mechanical allodynia, pain threshold responses were measured using the well-established method of von Frey filaments in line with that performed in the earlier Examples. The PWT test was performed on day -8 (before the first dose of paclitaxel), the day of starting treatment (day 0, at which central sensitization was fully established) and day 14 (treatment continued until day 15). The mechanical threshold, expressed as g, indicates the force of the von Frey filament to which the animal reacted.

For cold allodynia, mice with paclitaxel-induced central sensitization were treated with PS 5% or vehicle for 15 days after the induction of pain associated with central sensitization. The acetone test was used. Briefly, acetone was applied onto the plantar surface of each hind paw. The time each mouse spent licking, lifting, and/or shaking the hind paw recorded over 60 seconds was the score for cold allodynia. Measurements were performed on day -8 (before the first paclitaxel injection), the day of starting treatment (day 0) and then on day 15.

### Results

As expected, paclitaxel induced pain, as demonstrated by the reduction of the mechanical allodynia from a score of 1.83 ± 0.14 g prior to the administration of paclitaxel (*Mean ± SEM* for this and all subsequent values), to 0.55 ± 0.05 g on day 0 of the study (once central sensitization was fully established). Paclitaxel also sensitized mice to cold allodynia, as evidenced by changes in its scores before and after paclitaxel treatment (4.5 ± 0.24 sec vs. 7.1 ± 0.4 sec; p<0.0001).

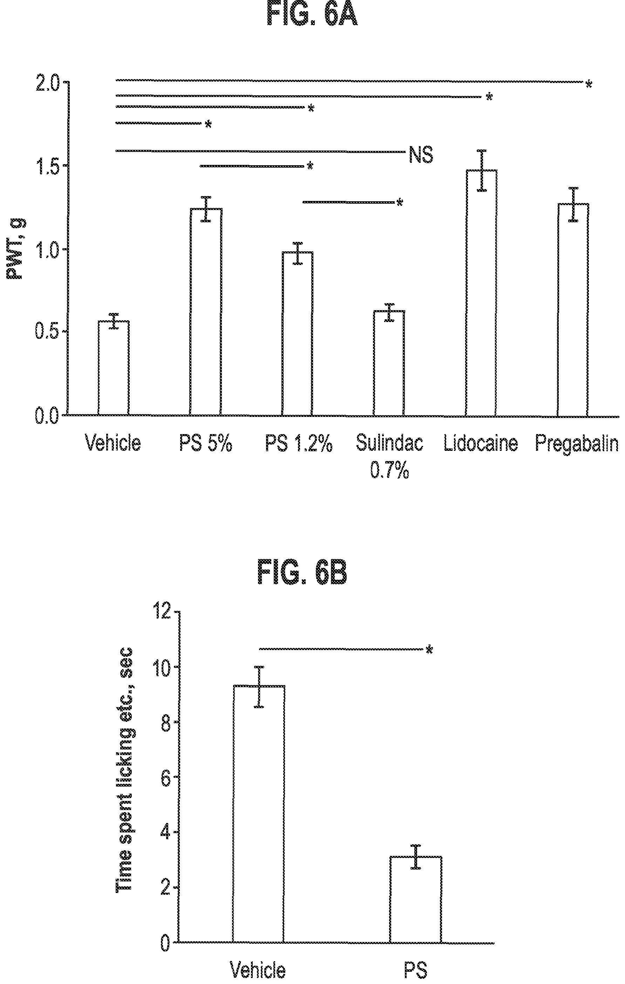

Treatment with PS improved the mechanical allodynia score in a dose-dependent manner (Figure 6A). Likewise, treatment with PS 5% improved the cold allodynia score. The cold allodynia score of the PS-treated group was significantly lower than that of the vehicle-treated group (3.1 ± 0.4 vs 9.3 ± 0.7; p<0.0001) (Figure 6B).

By contrast, administration of sulindac failed to demonstrate a significant effect on PWT, its score being similar to that of vehicle (0.62 ± 0.05 g vs. 0.56 ± 0.04 g; statistically not significant), while as expected both lidocaine and pregabalin positive controls achieved a significant increase in PWT compared to the vehicle (Figure 6A). Importantly, PS 1.2%, a concentration that is equimolar to sulindac 0.7%, significantly improved mechanical allodynia (p<0.0001). The values concerning mechanical allodynia, corresponding to Figure 6A, are provided in Table 2.

**Table 2 - PWT in the study groups**

| **Groups** | **PWT, g** | **P values** |
|---|---|---|
| | *Mean* ± *SEM* | |
| Paclitaxel + vehicle | 0.56 ± 0.04 | |
| Paclitaxel + PS 5% | 1.24 ± 0.07 | PS 5% vs. Vehicle: p<0.0001 |
| Paclitaxel + PS 1.2% | 0.98 ± 0.06 | PS 1.2% vs Vehicle: p<0.0001 |
| | | PS 1.2% vs PS 5%: p<0.008 |
| Paclitaxel + sulindac 0.7% | 0.62 ± 0.05 | Sulindac vs Vehicle: NS (p = 0.33) |
| | | Sulindac vs PS 1.2 %: p<0.0001 |
| Paclitaxel + 5% lidocaine cream | 1.48 ± 0.12 | Lidocaine vs Vehicle: p<0.0001 |
| Paclitaxel + pregabalin (10 mg/kg) | 1.28 ± 0.10 | Pregabalin vs Vehicle: p<0.0001 |

### Conclusions

Whereas PS was shown to be effective in the treatment of pain associated with central sensitization (see also Examples 1 and 2), strikingly its non-phosphorylated 'parent' sulindac (a typical NSAID) failed to achieve a rescue in the PWT in the mice model and so failed to treat pain associated with central sensitization. This was the case even though the sulindac was administered at a maximum non-toxic dose and in the same manner and formulation as PS. The positive controls, lidocaine and pregabalin, known to have direct activity on nerves and central sites of action in analgesia, demonstrated a significant reduction in pain, as expected. Therefore, the efficacy observed for locally administered PS is more similar to the centrally acting positive controls than to its closely related parent compound.

Accordingly, PS is mechanistically distinct from its parent NSAID and may be acting in a manner more similar to the centrally acting agents. These observations regarding the equivalent efficacy of PS and pregabalin and lidocaine in the treatment and prevention of pain associated with central sensitization reflect the central site of action of PS implicated above *(i.e.,* similar to that for pregabalin and lidocaine). These observations serve to demonstrate the potential of PS in the treatment of pain associated with central sensitization, akin to typical centrally acting analgesics (*e.g*., pregabalin).

### Example 4: The effect of PS in a rat model of central sensitization

Central sensitization was induced via increases in blood glucose in rats, using streptozotocin (STZ). This is a well-established model for generating chronic neuropathic pain (see Morrow, Current Protocols in Neuroscience (2004); 29(1): 1-11), in which the STZ, an antibiotic extract from *Streptomyces acromogenes,* selectively damages the β cells of the pancreas. As demonstrated, PS is effective in the treatment of central sensitization generated a further model of chronic pain.

### Methods

Sprague-Dawley rats were fasted for 4-6 hours before being injected intraperitoneally with 45 mg/kg STZ. This was performed for all experimental groups, except the naive group. In order to prevent hypoglycaemic death, rats received 10% sucrose water as the sole water source for the first 48 hours after injection. In order to ensure only those rats considered diabetic (*i.e.,* having non-fasting plasma glucose levels of >250 mg/dL) were included in the study, blood glucose was measured at 72 hours after the STZ injection.

Four weeks after STZ injection, the rats were randomised into treatment groups. This delay between STZ injection and treatment allows the establishment of chronic pain (allodynia) associated with central sensitization. PS (as an 8% hydrogel) or vehicle control were administered topically to both hind paws of the rats 3 times a day for 3 weeks starting at week 4 after STZ injection. 0.7% sulindac hydrogel was applied 3 times a day for 1 week - this is the highest safe sulindac concentration for these animals. 5% lidocaine cream (positive control) was applied only once to both hind paws of the rats 30 minutes before measurement of PWT. Finally, an additional positive control, pregabalin (at 10 mg/kg) or its vehicle were administered orally only once, one hour before measurement of PWT.

The rats were split into 8 study groups, with those in the experimental groups (1-4) having an average body weight of about 225 g, and those in the control groups (5-8) having an average body weight of about 335 g, as follows:
1. Group 1: naive rats (n = 5)
2. Group 2: STZ only (n = 6)
3. Group 3: STZ plus vehicle (n = 6)
4. Group 4: STZ plus PS (8% hydrogel) (n = 7)
5. Group 5: STZ plus oral vehicle (n = 8)
6. Group 6: STZ plus 0.7% sulindac hydrogel (n = 7)
7. Group 7: STZ plus 5% lidocaine cream (n = 8)
8. Group 8: STZ plus pregabalin (10mg/kg) (n = 7)

In order to determine the outcome of the treatment, mechanical allodynia was measured using the well-established method of von Frey filaments. In particular, a simplified up-down method for estimating paw withdrawal threshold (PWT) using von Frey filaments was used (as described in Bonin et al., Molecular Pain (2014); 10(26):1-10)). The results of the PWT test are expressed as force applied (gm). The PWT test was performed at weeks 4, 5, and 7, depending on the administration protocol, with the first measurement being the baseline (*i.e*., demonstrating the efficacy of STZ in establishing pain associated with central sensitization). The measurements on weeks 5 and 7 were performed 30 minutes after the last treatment with sulindac or PS, respectively. For completeness, the difference in average body weights of the rats in the experimental and control groups does not impact the ability to compare the results from these groups (*i.e.,* the rats were simply obtained from different batches and respond in the same way to the experimental procedure).

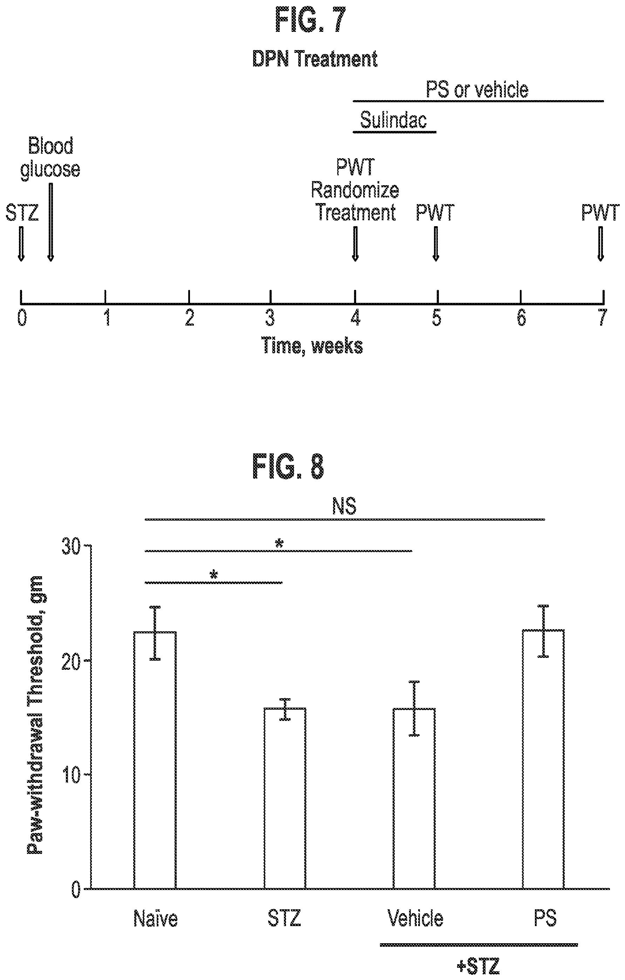

Figure 7 provides an outline of the study.

### Results

As displayed in Figure 8, the administration of STZ resulted in a significant decrease in PWT (p<0.02 vs naïve rats) 4 weeks after administration. Therefore, the model established pain associated with central sensitization, as expected. The additional administration of vehicle had no significant effect on the PWT compared to STZ only, with the reduction in PWT relative to naive rats similar to STZ alone. However, administration of PS achieved a significant increase in PWT compared to vehicle and STZ (p<0.04 and p<0.01, respectively), bringing the PWT back up to the threshold observed in naïve rats (p<0.01). The values, corresponding to Figure 8, are provided in Table 3.

**Table 3 - PWT in the first 4 study groups**

| **Groups** | **PWT, gm** | **P values** |
|---|---|---|
| | *Mean* ± *SEM* | |
| Naive | 22.4 ± 2.24 | |
| STZ only | 15.7 ± 0.84 | *p<0.02 *vs* naïve |
| STZ + vehicle | 15.8 ± 2.33 | NS *vs* STZ only |
| | | *p<0.01 *vs* naive |
| STZ + PS | 22.5 ± 2.20 | NS *vs* naive |
| | | *p<0.04 *vs* vehicle |
| | | *p<0.01 *vs* STZ only |

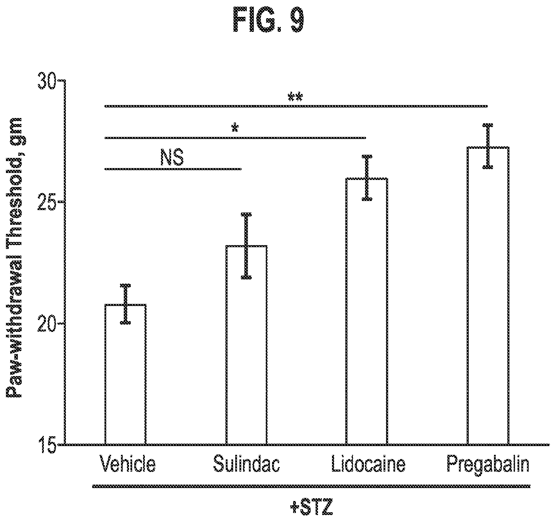

Furthermore, as displayed in Figure 9, administration of either vehicle or sulindac failed to suppress the reduced PWT caused by STZ (sulindac was not significantly different to vehicle). However, both lidocaine and pregabalin achieved significant increases in PWT compared to vehicle (p<0.03 and p<0.009 respectively). The values, corresponding to Figure 9, are provided in Table 4.

**Table 4 - PWT in the second 4 study groups**

| **Groups** | **PWT, gm** | **P values** |
|---|---|---|
| | *Mean* ± *SEM* | |
| STZ + oral vehicle | 20.8 ± 1.5 | |
| STZ + sulindac | 23.2 ± 2.6 | NS *vs* vehicle |
| STZ + lidocaine | 26.0 ± 1.8 | *p<0.03 *vs* vehicle |
| STZ + pregabalin | 27.3 ± 1.7 | **p<0.009 *vs* vehicle |

### Conclusions

The topical administration of PS is efficacious in normalizing the pain associated with central sensitization induced by diabetes in one of the most reliable animal models of the disease. Indeed, PS significantly increased the PWT in rats displaying central sensitization. Therefore, PS treats the pain associated with central sensitization induced by a further mechanism.

Whereas PS was effective, strikingly its non-phosphorylated 'parent' sulindac (a typical NSAID) failed to achieve a rescue in the PWT in the rat model and so failed to treat pain associated with central sensitization. This was the case even though the sulindac was administered at a maximum non-toxic dose and in the same manner and formulation as PS. The positive controls, lidocaine and pregabalin, known to have direct activity on nerves and central sites of action in analgesia, demonstrated a significant reduction in pain associated with central sensitization, as expected. Therefore, the efficacy observed for locally administered PS is more similar to the centrally acting positive controls than to its closely related parent compound, correlating with observations demonstrating the accumulation of PS at central sites of action.

Accordingly, PS is mechanistically distinct from its parent NSAID and may be acting in a manner more similar to the centrally acting agents. These observations serve to demonstrate the potential of PS in established chronic pain associated with central sensitization.

### Example 5: The effect of orally administered PS in treating neuropathic pain in a mouse model of CIPN

Further to the observations herein with topical administration of PS, the present Example demonstrates that oral administration of PS surprisingly treats neuropathic pain associated with CIPN, as demonstrated in an established animal model.

### Methods

Adult male C57BL/6J mice, 8 weeks of age at the beginning of the experiments and weighing 20-30 g, were purchased from The Jackson Laboratory (Bar Harbor, ME). Mice were housed in an AAALAC-accredited facility in groups of four. Food and water were available *ad libitum.* Experiments were performed during the light cycle (7:00 am to 7:00 pm) and animals were euthanized with CO₂ asphyxiation. The mice in each cage were randomly allocated to treatment groups. All studies were conducted by experimenters blinded to the identity of the treatment groups.

Studies were approved by the Institutional Animal Care and Use Committee of Stony Brook University and followed the National Institutes of Health Guidelines for the Care and Use of Laboratory Animals. Animal studies are reported in compliance with the ARRIVE guidelines (Kilkenny et al, 2010).

CIPN was induced in 8-weeks-old male C57BL/6J mice (~25 g). CIPN was induced in mice with chemotherapeutic agents using established protocols (Carozzi et al, 2010; Currie et al, 2019; Eldridge et al, 2020). Briefly, four intraperitoneal injections of paclitaxel at 8 mg/kg were given every other day, resulting in a cumulative dose of 32 mg/kg. This dosing regimen of paclitaxel produces pain associated with CIPN with time courses that are similar to those of pain after paclitaxel administration in cancer patients.

PS, and sulindac were given by oral gavage, three times a day for 7 days. Their doses were as follows: PS, 50 mg/kg; sulindac, 33 mg/kg (equimolar to PS).

Mechanical allodynia was determined for all mice study groups below:
1. Group 1: naïve mice *(i.e.* no paclitaxel, n=8)
2. Group 2: paclitaxel only (n=8)
3. Group 3: paclitaxel plus vehicle (n=8)
4. Group 4: paclitaxel plus PS (n=8)
5. Group 5: paclitaxel plus sulindac (n=8)

Mechanical allodynia was determined using the method of von Frey filaments in line with that performed in the earlier Examples. The PWT test was performed on day 7. The mechanical threshold, expressed as g, indicates the force of the von Frey filament to which the animal reacted.

Additionally, thermal allodynia was determined for all mice study groups below:
1. Group 1: paclitaxel plus vehicle (n=8)
2. Group 2: paclitaxel plus PS (n=8)
3. Group 3: paclitaxel pluse sulindac (n=8)

Briefly, thermal allodynia tests use a radiant heat source (Hargreaves et al., 1988). The animal is placed into a plastic box with an elevated glass floor. The beam of the lamp probe is focused on the paw plantar surface, until the animal withdraws the paw, and sensors in the device shut off the lamp. The thermal allodynia threshold is defined as the latency time until paw withdrawal (PWL) and expressed in seconds (sec) or thermal allodynia threshold. The response of thermal allodynia to PS and sulindac was tested on day 16 of treatment.

### Results

The administration of paclitaxel resulted in a significant decrease in PWT (p<0.001 *vs.* naive mice), as expected. Therefore, the model established neuropathic pain associated with chemotherapy, manifested as allodynia. The additional administration of vehicle has no significant effect on the PWT compared to treatment with paclitaxel only, while administration of PS achieved a significant increase in PWT compared to vehicle (p<0.003 vs vehicle). By contrast, administration of sulindac failed to demonstrate a significant effect on PWT, its score being similar to that of vehicle (0.56 ± 0.05 g vs. 0.54 ± 0.03 g; statistically not significant). The values are provided in Table 5.

**Table 5 - PWT in the study groups**

| **Groups** | **PWT,** g | **P values** |
|---|---|---|
| | *Mean* ± *SEM* | |
| Naïve | 1.55±0.12 | |
| Paclitaxel only | 0.53 ± 0.03 | p<0.001 *vs* naïve |
| Paclitaxel + vehicle | 0.54 ± 0.03 | NS *vs* paclitaxel only |
| Paclitaxel + PS | 0.99 ± 0.13 | p<0.003 *vs* vehicle |
| | | p<0.016 *vs* sulindac |
| Paclitaxel + sulindac | 0.56 ± 0.05 | NS *vs* vehicle |

To confirm the positive effects of PS as assessed with mechanical allodynia, paw withdrawal latency (PWL) were assessed in thermal allodynia tests in CIPN model mice. PS significantly increased the PWL compared to vehicle control (p < 0.04). In contrast, the effect of sulindac was not significant compared to vehicle control. PWL values are provided in Table 6.

**Table 6 - PWL in the study groups**

| **Groups** | **PWL,** sec | **P values** |
|---|---|---|
| | *Mean* ± *SEM* | |
| Paclitaxel + vehicle | 7.80 ± 0.7 | |
| Paclitaxel + PS | 10.78 ± 1.1 | p<0.04 *vs* vehicle |
| | | p<0.05 *vs* sulindac |
| Paclitaxel + sulindac | 7.87 ± 0.7 | NS *vs* vehicle |

### Conclusions

Similar to the observations with topically administered PS, the oral administration of PS resulted in a significant increase in PWT and PWL, in contrast to its parent compound, sulindac. These positive results are surprising not least in light of the ability of orally administered PS to achieve significant analgesic effects on pain originating at distal peripheral sites but persisting via activity of central neurons even in the absence of on-going peripheral triggers. However, these observations support earlier observations suggesting that PS acts centrally and demonstrate the broad applicability of PS in treating different forms of neuropathic pain. Indeed, further observations herein indicate that orally administered PS reaches central sites of pain generation, thus cementing the unprecedented activity of PS as a direct acting nerve analgesic effective at central sites of action.

### Example 6: The effect of PS in treating neuropathic pain associated with PTPN

Preliminary *in vivo* evidence indicates that the topical administration of PS, in the form of a hydrogel, achieves a striking analgesic effect on neuropathic pain associated with PTPN. This effect was surprising but is consistent with the data in WO2022/251805 and WO2022/251806 where PS is shown to have a direct analgesic effect on two distinct types of neuropathic pain (*i.e*., associated with CIPN and DPN).

### Example 7: The effect of topically administered PS in treating and/or preventing neuropathic pain in a mouse model of PTPN

Neuropathic pain associated with traumatic nerve injury can be regenerated in a rat animal model using the chronic constriction injury (CCI) model. In this model the sciatic nerve is compressed to imitate peripheral nerve injury. The model generates particular phenotypes observed in PTPN in humans, including, for example, allodynia.

The model is carried out according to the procedure developed by Bennett & Xie (Pain (1988);33(1):87-107). Briefly, the sciatic nerve is exposed and subject to the placement of four loose chromic catgut ligatures around the nerve. The ligatures occlude but do not arrest epineural blood flow. After three weeks following nerve injry, the rats are assessed for hind paw mechanical withdrawal thresholds, an indication of mechanical allodynia. Sensory testing utilizes von Frey filaments with reproducible calibrated buckling forces varying from 0.4 - 10g utilizing the Chaplan up and down method. Allodynia is tested by perpendicularly touching the hindpaw causing slight buckling of the filament for approximately 5 seconds. Based on the response pattern and the force of the final filament, the hindpaw withdrawal threshold (g) is calculated (Chaplan et al., Journal of neuroscience methods (1994);53(1), 55-63).

The ability of a topical formulation of PS, administered to the hindpaw, to reduce allodynia, compared to its parent compound (sulindac) and positive controls, is confirmed in this model system.

### Methods

Adult male C57BL/6J mice, 8 weeks of age at the beginning of the experiments and weighing 20-30 g, were purchased from The Jackson Laboratory (Bar Harbor, ME). Mice were housed in an AAALAC-accredited facility in groups of four. Food and water were available *ad libitum.* Experiments were performed during the light cycle (7:00 am to 7:00 pm) and animals were euthanized with CO₂ asphyxiation. The mice in each cage were randomly allocated to treatment groups. All studies were conducted by experimenters blinded to the identity of the treatment groups.

Studies were approved by the Institutional Animal Care and Use Committee of Stony Brook University and followed the National Institutes of Health Guidelines for the Care and Use of Laboratory Animals. Animal studies are reported in compliance with the ARRIVE guidelines (Kilkenny et al, 2010).

Chronic constriction injury-induced peripheral neuropathy (a form of post-traumatic peripheral neuropathy) was induced in 8 weeks old male C57BL/6J mice (~25 g) under anesthesia. The left sciatic nerve was exposed by a skin incision and cutting through the connective tissue between the gluteus superficialis and biceps femoris muscles. Two chronic gut ligatures with a 7-0 suture were tied loosely around the sciatic nerve 1 mm apart, to just occlude but not arrest epineurial blood flow. For the control sham mice, a similar skin incision was made on the left side but without ligating the sciatic nerve. In either case, the surgical wound was closed with sutures in the muscle and staples in the skin. Pain hypersensitivity testing was performed after 5-7 days of recovery from surgery.

PS (as a 5% gel), sulindac (as a 0.7% gel, the highest feasible concentration of sulindac), or vehicle control were administered topically onto the left paw of mice three times a day for 14 days. Pregabalin (10 mg/ml) was administered once by oral gavage one hour prior to determining mechanical allodynia, and was used as the positive control.

Mechanical allodynia was determined for all mice study groups below:
1. Group 1: ligation only *(i.e,* surgical control with no treatment, n=5-7)
2. Group 2: vehicle control (n=5-7)
3. Group 3: PS 5% (n=5-7)
4. Group 4: 0.7% sulindac (n=5-7)
5. Group 5: pregabalin (n=5-7)

Briefly, for determining mechanical allodynia, pain threshold responses were measured using the well-established method of von Frey filaments in line with that performed in the earlier Examples. The PWT test was performed on day 14. The mechanical threshold, expressed as g, indicates the force of the von Frey filament to which the animal reacted.

Thermal allodynia was also determined for the mice study groups below:
1. Group 1: vehicle control (n=6)
2. Group 2: PS 5% (n=6)

Thermal allodynia was determined using a radiant heat source (Hargreaves et al., 1988), in line with that performed in earlier Examples.

### Results

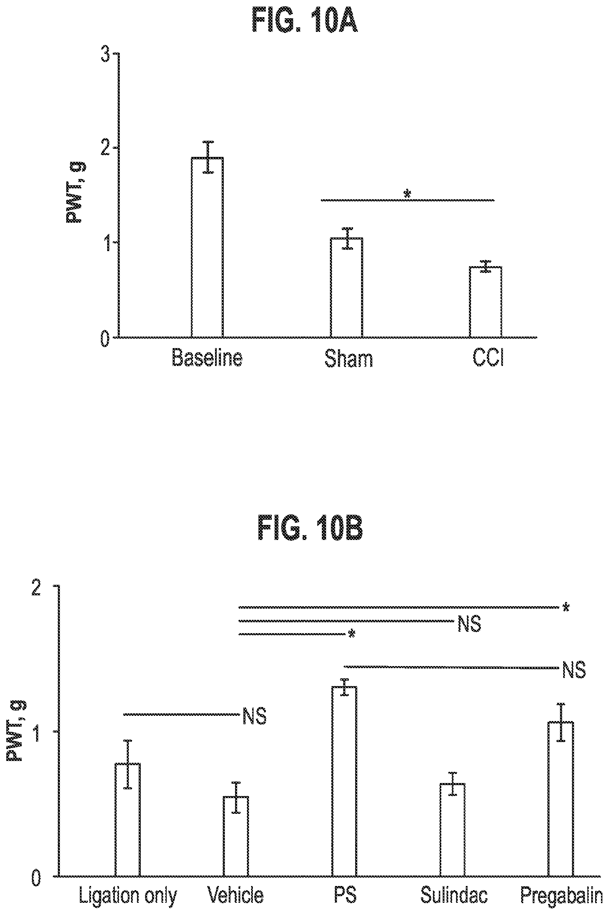

PTPN was induced in 31 mice as described above; 9 mice underwent the sham procedure. The induction of neuropathic pain associated with PTPN was confirmed by determining mechanical allodynia. At baseline (n=40) the PWT was 1.90±0.14 g (*mean ± SEM* for this and all subsequent values). Following ligation, the PWT values were as follows: sham operated mice (n=9) 1.04±0.10 g; CCI mice (n=31) 0.74±0.05g (p<0.01 compared to sham). These results are summarized in Figure 10A. Therefore, the chronic constriction injury model ensures a significant reduction in PWT compared to sham operated animals, confirming establishment of central sensitization (manifested as allodynia). Mice were randomized into study groups.

As displayed in Figure 10B, administration of vehicle had no significant effect on the PWT, whilst pregabalin significantly increased the PWT compared to vehicle treated mice, as expected. Sulindac failed to change the PWT value compared to vehicle control. However, administration of PS achieved a significant increase in PWT compared to vehicle (p < 0.005). The PWT value achieved in PS-treated mice was greater even than pregabalin-treated mice (1.30 ± 0.15 g vs 1.1 ± 0.10 g; Mean ± SEM). The values, corresponding to Figure 10B, are provided in Table 7.

**Table 7 - PWT in the study groups**

| **Group** | **PWT, g** | |
|---|---|---|
| | *Mean* ± *SEM* | **P values** |
| Ligation only | 0.77±0.13 | |
| Vehicle | 0.54±0.04 | NS *vs* ligation only |
| PS | 1.30±0.15 | p<0.005 *vs* vehicle |
| Sulindac | 0.63±0.07 | NS *vs* vehicle |
| Pregabalin | 1.1±0.10 | p<0.04 *vs* vehicle |
| | | NS *vs* PS |

To confirm the positive effects of PS as assessed with mechanical allodynia, paw withdrawal times (PWL) were assessed in thermal allodynia tests. PTPN was induced in 12 mice as described above. PS significantly increase the PWL time compared to vehicle control (3.4±1.0 g vs 9.1±2.9 g, p < 0.05). PWL values are provided in Table 8.

**Table 8 - PWL in the study groups**

| **Group** | **PWL, sec** | |
|---|---|---|
| | *Mean* ± *SEM* | **P value** |
| Vehicle | 3.4±1.0 | |
| PS | 9.1±2.9 | p<0.05 vs vehicle |

### Conclusions

The topical administration of PS significantly increased the PWT in mice with chronic constriction injury, an established model of PTPN. It also significantly increased PWL in thermal allodynia tests. Therefore, PS treats established pain associated with PTPN and further supports the broad applicability of PS in treating different forms of neuropathic pain.

Whereas PS was effective in treating pain associated with central sensitization (manifested as allodynia) in this PTPN model, reflecting the ability of PS to provide a direct analgesic effect on pain associated with central sensitization as observed in the previous Examples, its non-phosphorylated 'parent' sulindac (a typical NSAID) failed to achieve a rescue in the PWT in the mouse model and so failed to treat pain associated with PTPN. This was the case even though the sulindac was administered at a maximum non-toxic dose and in the same manner and formulation as PS. The positive control pregabalin, known to have central sites of action in analgesia, demonstrated a significant reduction in pain associated with PTPN, as expected. Therefore, the efficacy observed for locally administered PS is more similar to the centrally acting positive controls than to its closely related parent compound, correlating with observations demonstrating the accumulation of PS at central sites of action.

Accordingly, PS is mechanistically distinct from its parent NSAID and may be acting in a manner more similar to the centrally acting agents. These observations serve to demonstrate the potential of PS in established chronic pain associated with PTPN. Furthermore, these results, in combination with the observations herein of the accumulation of PS at central sites of action, point to an unprecedented and surprising broad applicability of PS in treating and preventing pain having central mechanisms *(i.e.,* generated in central sites or due to central sensitization).

### Example 8: The effect of topically administered PS at a distal site in treating and/or preventing neuropathic pain in a mouse model of PTPN

The previous Example in mice with neuropathic pain associated with PTPN (via the chronic constriction injury model) demonstrates the remarkable ability of PS to treat pain associated with PTPN. Strikingly, and in line with other observations herein regarding the ability of PS to traverse along neurons towards central sites, the present example demonstrates that PS imparts its analgesic effect at central sites of action.

### Methods

In a first experiment, PTPN was induced using chronic constriction injury in 8 weeks old male C57BL/6J mice (~25 g) under anesthesia, as previously described, by ligating the left sciatic nerve, to induce neuropathic pain manifested as allodynia affecting the left limb of the mice (*i.e.,* reducing the paw withdrawal threshold).

PS (as 8% gel), or vehicle control were administered topically onto the contralateral (*i.e.* right) hind paw of mice 3 times a day for 4 days prior to determining mechanical allodynia on the operated limb.

Mechanical allodynia was determined for all mice study groups below:
1. Group 1: ligation only (*i.e,* surgical control with no treatment, n=6)
2. Group 2: vehicle control (n=6)
3. Group 3: PS 8% (n=6)

Mechanical allodynia was determined using the method of von Frey filaments in line with that performed in the earlier Examples, assessing the paw withdrawal threshold of the operated left limb. The PWT test was performed on day 4. The mechanical threshold, expressed as g, indicates the force of the von Frey filament to which the animal reacted.

Additionally, in a second study, the speed at which PS can treat pain associated with PTPN was assessed by assessing the effect of PS on mechanical allodynia shortly after topical administration of PS. As above, PTPN was induced using chronic constriction injury, by ligating the left sciatic nerve, to induce neuropathic pain manifested as allodynia affecting the left limb of the mice. PS (as a 5% gel) was applied only once onto the contralateral (right) hind paw of mice, prior to determining mechanical allodynia on the operated limb at different time points post-dosing.

Mechanical allodynia (via the PWT test) was determined for all mice study groups below:
1. Group 1: ligation only *(i.e,* surgical control with no treatment, n=5)
2. Group 2: PS 5%, assessed at t = 0.5 h post-dosing (n=5)
3. Group 3: PS 5%, assessed at t = 1.0 h post-dosing (n=5)
4. Group 4: PS 5%, assessed at t = 2.0 h post-dosing (n=5)

### Results

In the first experiment, PTPN was induced in 18 mice as described above and mice were randomized into study groups. As expected, treating mice with the vehicle control did not results in a significant change from PWT values following ligation (0.52±0.09 g vs 0.53±0.03 g). Remarkably, treatment with PS on the contralateral paw significantly increased PWT compared to both vehicle and the surgical control (p < 0.001). The values are provided in Table 9.

**Table 9 - PWT in the study groups**

| **Group** | **PWT, g** | |
|---|---|---|
| | *Mean* ± *SEM* | **P values** |
| Ligation only | 0.53±0.03 | |
| Vehicle | 0.52±0.09 | |
| PS | 1.16±0.04 | p<0.001 *vs* vehicle |
| | | p<0.001 *vs* ligation only |

In the second experiment, PTPN was induced in 20 mice as described above and mice were randomized into study groups. Building on the surprising results of the first experiment, significant increases in PWT were observed across all tested time points, including the shortest time point of 0.5 h after dosing (p < 0.0005). The values are provided in Table 10.

**Table 10 - PWT in the study groups**

| **Group** | **PWT, g** | **P values** *vs ligation only* |
|---|---|---|
| | *Mean* ± *SEM* | |
| Ligation only | 0.58 ± 0.02 | |
| t = 0.5 h | 1.24 ± 0.10 | <0.0005 |
| t = 1.0 h | 1.16 ± 0.16 | <0.01 |
| t = 2.0 h | 1.10 ± 0.19 | <0.03 |

### Conclusions

The topical administration of PS on the contralateral, unoperated, paw of mice with neuropathic pain associated with PTPN achieved a significant increase in PWT. In line with the observations herein with respect to the ability of PS to traverse along neurons towards central sites, this experiment confirms that PS accumulates in central sites of action at sufficient concentrations to directly inhibit neuronal pain signalling. Remarkably, a significant increase in PWT was observed as little as 0.5 hours following administration of PS on the contralateral paw. This suggests that PS has an ability to accumulate at therapeutically relevant amounts and act at central sites rapidly (as indicated by PK analysis herein). In this way, PS is able to act directly on centrally located neurons to block pain signalling generated at central sites, for example via central sensitization where pain is sensed even in the absence of an ongoing noxious peripheral stimulus.

As provided in the earlier examples, the parent NSAID of PS *(i.e.,* sulindac) failed to treat the neuropathic pain associated with PTPN. The present example further confirms that PS is acting in a mechanistically distinct way compared to its parent NSAID. PS reaches central sites of action rapidly and has a direct effect on the neuronal signalling, something which sulindac, and other typical NSAIDs fail to achieve. These observations support the potential of PS to treat chronic pain indications in which pain is sensed as a consequence of the activity of centrally acting neurons.

### Example 9: The effect of orally administered PS in treating neuropathic pain in a mouse model of PTPN

Orally administered PS was investigated in a mouse model of PTPN, building on the results observed with topically applied PS and further observations herein demonstrating the broad applicability of PS as a centrally acting analgesic.

### Methods

Adult male C57BL/6J mice, 8 weeks of age at the beginning of the experiments and weighing 20-30 g, were purchased from The Jackson Laboratory (Bar Harbor, ME). Mice were housed in an AAALAC-accredited facility in groups of four. Food and water were available *ad libitum.* Experiments were performed during the light cycle (7:00 am to 7:00 pm) and animals were euthanized with CO₂ asphyxiation. The mice in each cage were randomly allocated to treatment groups. All studies were conducted by experimenters blinded to the identity of the treatment groups.

Studies were approved by the Institutional Animal Care and Use Committee of Stony Brook University and followed the National Institutes of Health Guidelines for the Care and Use of Laboratory Animals. Animal studies are reported in compliance with the ARRIVE guidelines (Kilkenny et al, 2010).

Chronic constriction injury-induced peripheral neuropathy (a form of post-traumatic peripheral neuropathy) was induced in 8 weeks old male C57BL/6J mice (~25 g) under anesthesia, as previously described, by ligating the left sciatic nerve. This establishes neuropathic pain associated with PTPN (manifested as allodynia).

PS was administered orally three times a day for 6 days, at 100 mg/kg.

Mechanical allodynia was determined for all mice study groups below:
1. Group 1: ligation only *(i.e,* surgical control with no treatment, n=9)
2. Group 2: vehicle (n=9)
3. Group 3: PS (n=9)

Mechanical allodynia was determined using the method of von Frey filaments in line with that performed in the earlier Examples. The PWT test was performed on day 6. The mechanical threshold, expressed as g, indicates the force of the von Frey filament to which the animal reacted.

### Results

As expected, treating mice with the vehicle control did not results in a significant change from PWT values following ligation. In contrast, treatment with orally administered PS significantly increased PWT compared to vehicle control treatment. The values are provided in Table 11A.

**Table 11A - PWT in the study groups**

| **Groups** | **PWT, g** | **P value** |
|---|---|---|
| | *Mean* ± *SEM* | |
| Ligation only | 0.55 ± 0.05 | |
| Vehicle | 0.58 ± 0.06 | NS *vs* ligation only |
| PS | 1.63 ± 0.38 | p<0.02 *vs* vehicle |

In another experiment in the same model system described herein, the parent compound, sulindac, was shown to be ineffective in treating neuropathic pain associated with PTPN. Indeed, sulindac, administered orally at 33 mg/kg had no significant effect on PWT compared to vehicle (Table 11B).

**Table 11B - PWT in the study groups**

| **Groups** | **PWT, g** | **P value** |
|---|---|---|
| | *Mean* ± *SEM* | |
| Ligation only | 0.56 ± 0.01 | |
| Vehicle | Day 6 = 0.51 ± 0.01 | Day 6 = NS *vs* ligation only |
| Sulindac | Day 6 = 0.6 ± 0.03 | Day 6 = NS *vs* vehicle |

### Conclusions

Similar to the observations with topically administered PS, the oral administration of PS resulted in a significant increase in PWT in a mouse model of PTPN. This activity is in contrast to the parent compound, sulindac, which fails to achieve analgesia in this challenging pain model. The analgesic effect of orally administered PS compared to vehicle in this animal model was improved compared to topically administered PS.

These positive results affirm data herein in this model with topical PS, and confirm that the PS is not acting as a local analgesic, but instead is able to reach central sites of action and achieve significant effects on pain known to be associated with central sensitization, manifested as allodynia. The ability of PS to act on centrally generated pain even upon oral administration, is particularly surprising, and supported by further observations herein that indicate that orally administered PS reaches central sites of pain generation. The results further confirm the broad applicability of PS, as an apparent direct acting nerve analgesic, in treating different forms of neuropathic pain.

### Example 10: The effect of orally administered PS, and the failure of sulindac, in treating neuropathic pain in a mouse model of PTPN

To support the observations in previous Examples, lower doses of orally administered PS were tested for efficacy in treating neuropathic pain associated with PTPN, and also compared to the parent compound sulindac.

### Methods

Chronic constriction injury was exploited to induce neuropathic pain associated with PTPN. The neuropathic pain was induced in 8 weeks old male C57BL/6J mice (~25 g) under anesthesia, as previously described, by ligating the left sciatic nerve. This establishes neuropathic pain associated with PTPN (manifested as allodynia).

PS, sulindac, or vehicle control were administered orally once a day for 6 days or 14 days, as indicated in the Table below. The dose of PS was 50 mg/kg; the dose of sulindac was 33 mg/kg (equimolar with PS).

Mechanical allodynia was determined for all mice study groups below:
1. Group 1: ligation only *(i.e,* surgical control with no treatment, n=9)
2. Group 2: vehicle (n=9)
3. Group 3: PS (n=9)
4. Group 4: sulindac (n=9)

Mechanical allodynia was determined using von Frey filaments in line with that performed in the earlier Examples. The PWT test was performed on day 6 or day 14, as indicated. The mechanical threshold, expressed as g, indicates the force of the von Frey filament to which the animal reacted.

### Results

As expected, treating mice with the vehicle control did not result in a significant change from PWT values comparted to ligation alone. This failure persisted until day 14. In contrast, treatment with orally administered PS significantly increased PWT compared to vehicle control treatment (day 6: 1.22 ± 0.14 g vs 0.51 ± 0.01 g, p <0.0002). This effect was increased over the longer, 14-day administration time (1.62 ± 0.13 g vs 0.56 ± 0.03 g, p <0.00001). In contrast, sulindac failed to achieve any significant change compared to vehicle control, at either time point. The values are provided in Table 12.

**Table 12- PWT in the study groups**

| **Groups** | **PWT, g** | **P value** |
|---|---|---|
| | *Mean* ± *SEM* | |
| Ligation only | 0.56 ± 0.01 | |
| Vehicle | Day 6 = 0.51 ± 0.01 | Day 6 = NS *vs* ligation only |
| | Day 14 = 0.56 ± 0.03 | Day 14 = NS *vs* ligation only |
| PS | Day 6 = 1.22 ± 0.14 | Day 6 = p<0.0002 *vs* vehicle |
| | Day 14 = 1.62 ± 0.13 | Day 14 = p<0.00001 *vs* vehicle |
| Sulindac | Day 6 = 0.6 ± 0.03 | Day 6 = NS *vs* vehicle |
| | Day 14 = 0.6 ± 0.0 | Day 14 = NS *vs* vehicle |

### Conclusions

These results confirm the striking effect of higher doses of PS in treating pain associated with PTPN as observed herein. In particular, the oral administration of PS at both a lower dose and lower frequency of administration remarkably still resulted in a significant increase in PWT in a mouse model of PTPN. Such an effect is surprising and demonstrates that PS is a particularly effective compound in treating established neuropathic pain even upon oral administration. This effect is strengthened over a longer treatment period, at which point the effect is comparable to the higher dose used in previous Examples. These positive results further point to the clinical applicability of orally administered PS in treating challenging pain indications, including those with established neuropathic pain and having central sites of action. Indeed, the observations point to the utility of lower doses of PS over extended dosing intervals.

As consistently observed herein, the anti-inflammatory activity of sulindac is simply insufficient for treatment of the complex neuropathic pain developed in this animal model, with the success of orally administered PS further pointing to a direct activity of PS on centrally located neurons involved in persistent pain signalling. PS therefore shows distinct and unexpected activity compared to the parent compound in a further model of neuropathic pain upon oral administration, even at lower doses. This confirms observations that PS is acting in a mechanistically distinct way compared to its parent NSAID.

### Example 11: Comments on observations in relation to PTPN

Preliminary *in vivo* observations demonstrate an unprecedented analgesic activity of PS in treating neuropathic pain associated with PTPN. These observations are surprising, but are consistent with the data in WO2022/251805 and WO2022/251806 (both hereby incorporated by reference in their entirety) where PS is shown to have a direct analgesic effect on two distinct types of neuropathic pain *(i.e.,* associated with CIPN and DPN). In light of the potent analgesic efficacy of PS in two pathophysiologically distinct forms of neuropathic pain, the present inventor hypothesised that PS might have more broadly applicable analgesic activities in other forms of neuropathic pain, perhaps (but without wishing to be bound by theory) based on the ability of PS to traverse towards central sites of action and to directly impact nerve signalling associated with central sensitization. The inventor's initial observations, and the further data in an appropriate animal model of pain associated with PTPN presented herein, confirm this hypothesis, demonstrating an unprecedented analgesic activity of PS in treating neuropathic pain associated with PTPN. Such a broad applicability as an analgesic across different forms of neuropathic pain is not guaranteed - indeed, gabapentin shows different efficacy in the treatment of different forms of neuropathic pain. Further experiments, described herein, using well-established animal models of neuropathic pain associated with PTPN confirm these observations. The inventor expects that PS is having a direct effect on neuronal pain signalling generated by the trauma suffered by the peripheral nerves, and may be preventing the generation of pain at central sites by reducing signalling caused by central sensitization. This is suggested by evidence indicating the ability of PS to reduce allodynia associated with PTPN, even when administered at a site distal to the peripheral site of pain generation, and the observed ability of PS to be stable in and traverse along centrally projecting neurons towards central sites of action, even upon oral administration. Strikingly, the topical administration of PS to a single paw achieved a striking reduction in allodynia (a manifestation of central sensitization) triggered by post-traumatic peripheral neuropathy in the contralateral paw in a PTPN animal model. Therefore, PS appears to achieves its analgesic effects by targeting neuronal activity at central sites of action, and thus can be therapeutically effective even when administered distally (or indeed orally). Such a therapeutic applicability of PS is unprecedented. In light of the ability of PS to traverse towards central sites of action upon topical administration, these findings represent a striking contribution in providing a straightforward treatment of neuropathic pain associated with PTPN. Indeed, the topical route provides low systemic clearance, reduced drug interactions, increased patient tolerability, and facile combination with oral medications. PS may also be administered orally for treatment of neuropathic pain associated with PTPN. Indeed, surprisingly, oral administration in the appropriate animal model achieved analgesia.

These observations demonstrate a previously unrecognised activity and therapeutic utility of PS, a compound which falls within the broader class of NSAIDs but does not share all of the properties of this family of compounds. Indeed, unlike typical NSAIDs, PS does not inhibit activity of COX-1 or COX-2 and does not block the synthesis or activity of prostaglandins. In fact, PS has instead been shown to trigger increased COX activity and significantly increased PGE₂ levels (Mackenzie et al. (2010) Gastroenterology 139(4): 1320-32). The preliminary observations indicate a role of PS as an analgesic, targeting pain signalling associated with PTPN, an activity which is distinct from the established role of PS, and typical NSAIDs, as anti-inflammatory agents. Indeed, such anti-inflammatory activity has been shown to be ineffective in the treatment of peripheral neuropathic pain (Moore et al. (Cochrane Database of Systematic Reviews (2015); 10: 1-25)) and NSAIDs, for example ketorolac, have shown limited analgesic activity against neuropathic pain associated with PTPN.

Earlier observations regarding the activity of PS are limited to its anti-inflammatory activity. For example, WO 2019/067919 suggests a role for PS in the treatment of DED, using an acute DED model, in which concanavalin A (ConA) is administered to rabbit lacrimal glands concurrently with PS. In this context, the anti-inflammatory activity of PS results in a limited inflammatory response to ConA, thus preventing the establishment of DED. These observations confirm the anti-inflammatory activity of PS and suggest its utility in preventing establishment and maintenance of inflammatory components of DED. The observations in this acute DED model fail to provide any evidence of the ability of PS as an analgesic, able to act directly on nerves to reduce nerve signalling caused by neuropathic pain. Any reduction in pain in this acute DED model can be assumed only to be a consequence of PS inhibiting the inflammatory response *(i.e.,* the pathology responsible for triggering activation of the pain sensors). In fact, the results of the DED model suggest that PS improves corneal sensitivity, implicating an increase in nociception, the opposite effect to that desired for an analgesic. Of course, irrespective of any suggestion towards analgesic activity of PS, such activity observed in an acute DED model provides no indication of a corresponding activity in neuropathic pain, and certainly not in neuropathic pain associated with PTPN.

The observations herein demonstrate that PS has therapeutic utility beyond an anti-inflammatory activity associated with typical NSAIDs. In contrast, certain NSAIDs, for example ketorolac, are ineffective analgesics in treating neuropathic pain associated with PTPN. Furthermore, Moore et al. (Cochrane Database of Systematic Reviews (2015); 10: 1-25), outlined that NSAIDs have no therapeutic efficacy in peripheral neuropathic pain. The activity of PS suggested herein contrasts with the inability of typical NSAIDs to provide a direct analgesic effect on damaged neurons in neuropathic pain as observed in the prior art. Without wishing to be bound by theory, the reason for the absence of any response to typical NSAIDs in the prior art is likely that the pain is caused by neuropathic nerve damage, rather than by inflammation (*i.e*., any anti-inflammatory activity of typical NSAIDs is not sufficient to prevent or treat the neuropathic pain). Accordingly, the analgesic activity of PS indicated herein is unique and not shared by typical NSAIDs. Any alleged analgesic activity of NSAIDs observed in the prior art is a reflection of their anti-inflammatory activity *(i.e.,* stopping potential triggers causing the pain) rather than an actual analgesic activity directed towards nerve signalling *(i.e.,* that would result in a reduction in pain caused by nerve damage and sensitization). For example, WO 2008/014066 implicates sulindac in treating pain associated with a chronic constriction injury model, but any alleged effect is concluded to be due to the reduction of pro-inflammatory cytokines, rather than an effect on pain signalling pathways *per se.* Indeed, as noted above, the anti-inflammatory activity of typical NSAIDs has been considered to have no therapeutic utility in treating neuropathic pain. Irrespective, any alleged activity of sulindac cannot be extrapolated to PS in light of the distinct activities of sulindac and PS - the observations herein demonstrate the utility of PS in treating neuropathic pain associated with PTPN. Any suggestions that typical NSAIDs have analgesic activity are associated with a requirement for inhibition of the COX pathways and synthesis of prostaglandins *(i.e.,* activities of typical NSAIDs that are not shared by PS). Without wishing to be bound by theory, the observations herein indicate that PS effectively treats neuropathic pain associated with PTPN by targeting neuronal signalling generated via central sensitization, a key component of neuropathic pain associated with PTPN.

Therefore, the present inventor has demonstrated a new surprising activity for PS in the treatment and/or prevention of neuropathic pain associated with PTPN. As outlined above, the observations implicate an activity of PS that goes beyond the anti-inflammatory activity previously observed for PS and related NSAIDs. Furthermore, the ease by which PS can be administered, for example topically, and its limited adverse effects (Mackenzie et al. (2010) Gastroenterology 139(4): 1320-32) render it an improved therapy for neuropathic pain associated with PTPN.

Furthermore, surprisingly the analgesic activity of PS was maintained even upon oral administration in animal models establishing neuropathic pain associated with PTPN. The ability of PS to act on neuropathic pain associated with PTPN is particularly surprising, and supported by further observations herein that indicate that orally administered PS reaches central sites of pain generation.

### Example 12: The effect of PS in treating migraine pain

Preliminary *in vivo* evidence indicates that the topical administration of PS, in the form of a hydrogel, achieves a striking analgesic effect on migraine pain. This effect was surprising but is consistent with the data in WO2022/251805 and WO2022/251806 where PS is shown to have a direct analgesic effect on two distinct types of neuropathic pain *(i.e.,* associated with CIPN and DPN).

### Example 13: The effect of PS, and failure of sulindac, in treating and/or preventing pain in a mouse model of migraine

The nitroglycerin (NTG)-induced migraine mouse model (Bates et al, 2010) is a particularly useful and clinically relevant animal model for migraine. NTG causes activation and sensitization of primary afferent and second-order trigeminovascular neurons. These pathophysiologies are thought to be the underlying mechanism of migraine headache (with the repeated activation of dural afferents believed to occur in patients with recurrent migraine headache), and many craniofacial nociceptive symptoms. NTG also mediates facial cutaneous and hind-paw hypersensitivity to non-noxious peripheral stimuli, indicative of allodynia, a manifestation of central sensitization. Accordingly, the ability of NTG to trigger central sensitization renders this model applicable for both migraine and other pain disorders associated with central sensitization more generally. In this model, rats are tested for mechanical allodynia (a pain response to stimuli which are not normally painful) using von Frey fibres, which are small calibrated fibres which deliver a calibrated amount of force.

Historical data in this model shows mechanical nociception sensitivity, which is alleviated with current standard of care compounds, suggesting this model has clinical translation. As foreshadowed above, sensitivity to non-noxious stimuli is referred to as allodynia and has been shown as a potential clinical correlate in migraine patients. Indeed, patients with chronic or transformed migraine exhibit facial allodynia even on days when they do not have a headache (Cooke et al., The Journal of Head and Face Pain (2007); 47(4), 531-539). Improvements in allodynia, for example shown by an increase in paw withdrawal threshold in the present model, correlate with treatment of pain similar to that experienced in migraine patients.

The ability of a topical formulation of PS to reduce allodynia, compared to its parent compound (sulindac) and positive controls, is confirmed in this model system.

### Methods

Adult male C57BL/6J mice, 8 weeks of age at the beginning of the experiments and weighing 20-30 g, were purchased from The Jackson Laboratory (Bar Harbor, ME). Mice were housed in an AAALAC-accredited facility in groups of four. Food and water were available *ad libitum.*

Experiments were performed during the light cycle (7:00 am to 7:00 pm) and animals were euthanized with CO₂ asphyxiation. The mice in each cage were randomly allocated to treatment groups. All studies were conducted by experimenters blinded to the identity of the treatment groups.

Studies were approved by the Institutional Animal Care and Use Committee of Stony Brook University and followed the National Institutes of Health Guidelines for the Care and Use of Laboratory Animals. Animal studies are reported in compliance with the ARRIVE guidelines (Kilkenny et al, 2010).

An NTG-induced migraine mouse model similar to that described in Bates et al, 2010 was used. Specifically, instead of the intraperitoneal injection of NTG, a finely crushed 0.4 mg NTG tablet (Greenstone Brand) was administered sublingually. As a control, a Practi-Nitroglycerin Sublingual 0.4 mg Simulated Medication tablet was used. The mechanical nociceptive threshold was reduced as early as 30 minutes post NTG administration and persisted for about 24 hours.

PS (as an 8% gel), sulindac 5.3% (solubilised in DMSO), or vehicle control gel was administered topically onto both hind paws of each mouse 30 minutes after administration of NTG.

Mechanical allodynia was determined for all mice study groups below:
1. Group 1: vehicle control (n=6)
2. Group 2: PS 8% (n=6)
3. Group 3: sulindac 5.3% (n=6)

For determining mechanical allodynia, pain threshold responses were measured using the well-established method of von Frey filaments in line with that performed in the earlier Examples. The PWT test, assessing mechanical allodynia, was performed 30 minutes after treatment. The mechanical threshold, expressed as g, indicates the force of the von Frey filament to which the animal reacted.

### Results

Initially, we confirmed the NTG model of migraine followed by an assessment of the effect of PS and sulindac in treating migraine pain. In the first study, we used 6 separate groups of mice, 3 for the 30 min time point and 3 for the 60 minute time point, as determination of mechanical allodynia on the same animals within 30 minutes may affect the results. As shown in the table below, the administration of NTG generates allodynia within 30 minutes of NTG administration, in line with the understanding that this compound rapidly triggers neuronal activity corresponding to central sensitization. For completeness, the oral NTG placebo control had no effect on PWT compared to baseline (*i.e*., did not generate allodynia) (see Table 13A).

**Table 13A - Confirmation of NTG model**

| **Group** *n=6*/*group* | **PWT, g 30 min** | **PWT, g 60 min** | **P value** *vs. respective baseline* |
|---|---|---|---|
| | *Mean* ± *SEM* | | |
| Baseline | 1.28±0.05 | 1.65±0.07 | |
| NTG | 0.70±0.12 | 0.51±0.05 | 30 min, P<0.0025 |
| | | | 60 min, P<0.00008 |
| NTG placebo | 1.38±0.10 | 1.58±0.21 | 30 min, NS |
| | | | 60 min, NS |

As expected and outlined in Table 13B, following migraine induction, vehicle-treated mice had lower PWT values compared to baseline values measured in mice which were not administered NTG. These results are consistent with the induction of migraine via the activity of NTG. The topical administration of PS achieved a significant increase in PWT compared to vehicle (p < 0.003). On the contrary, the parent compound, sulindac, failed to improve the PWT compared to vehicle. The values are provided in Table 13B.

**Table 13B - PWT in the study groups**

| **Group** *n=6*/*group* | **Baseline** | **Post-treatment** | **P value** (post-treatment) |
|---|---|---|---|
| *Mean* ± *SEM* | | | |
| ***Vehicle*** | 1.58±0.11 | 0.53±0.04 | vs. baseline: <0.00001 |
| ***PS*** | 1.72±0.10 | 1.00±0.12 | vs. vehicle: <0.003 |
| ***Sulindac*** | 1.43±0.15 | 0.55±0.02 | vs. vehicle: NS |
| | | | vs. PS: <0.003 |

### Conclusions

The topical administration of PS significantly increased the PWT in mice with NTG-induced migraine compared to vehicle control. Therefore, strikingly PS treats the pain associated with migraine. In line with other observations herein, this experiment confirms the failure of the parent compound, sulindac, even at particularly high concentrations, to treat pain associated with migraine, indicating that merely anti-inflammatory activity is insufficient in this animal model of migraine pain. Indeed, the allodynia associated with NTG administration is due to activation of nerve signalling through the trigeminal nucleus caudalis, confirming the observations herein that the analgesic activity of PS is distinct from any anti-inflammatory activity, and is more like the direct nerve acting activity of centrally acting agents. Without wishing to be bound by theory, the ability of PS to treat pain triggered by the administration of NTG further confirms the activity of PS to act directly on pain generating nerve signalling and to combat pain associated with central sensitization. Furthermore, in line with the observations herein regarding the rapid accumulation of PS at central sites of action, these results confirm the ability of PS to traverse from peripheral sites of administration to distal sites of pain generation in order to achieve its analgesic effects.

### Example 14: The effect of orally administered PS in treating pain in a mouse model of migraine

Orally administered PS was investigated in a mouse model of migraine pain, building on the results observed with topically applied PS and further observations herein demonstrating the broad applicability of PS as a centrally acting analgesic for the treatment of migraine pain and pain associated with central sensitization.

### Methods

In line with the earlier Examples, migraine was induced using 0.4 mg of NTG was administered sublingually in the form of a finely crushed 0.4 mg NTG tablet (Greenstone Brand). Mechanical allodynia in response to NTG manifests in 30 minutes and lasts less than 24 hours. The ability of NTG to trigger central sensitization, manifested as allodynia, renders this model applicable for both migraine and other pain disorders associated with central sensitization more generally.

PS and sulindac were administered orally to each mouse. PS was administered at 50 mg/kg and sulindace was administered at 33 mg/kg (equimolar to the PS dose), 30 minutes after NTG administration.

Mechanical allodynia was determined for all mice study groups below:
1. Group 1: baseline (*i.e,* no NTG treatment, n=6)
2. Group 2: vehicle (n=6)
3. Group 3: PS (n=6)
4. Group 4: sulindac (n=6)

Mechanical allodynia was determined using the method of von Frey filaments in line with that performed in the earlier Examples. The PWT test was performed 30 minutes after PS or vehicle control administration. The mechanical threshold, expressed as g, indicates the force of the von Frey filament to which the animal reacted.

### Results

As expected, following migraine induction, vehicle-treated mice had lower PWT values compared to baseline values measured prior to the induction of migraine (*i.e.,* sublingual administration of NTG establishes allodynia that is not reversed by administration of vehicle). These results are consistent with the induction of migraine. Oral administration of PS achieved a significant increase in PWT compared to vehicle (p < 0.002). Oral administration of sulindac did not significantly increase PWT compared to vehicle. The values are provided in Table 14.

**Table 14 - PWT in the study groups.**

| **Group** | **PWT, g** | **P value** |
|---|---|---|
| | *Mean* ± *SEM* | |
| Baseline | 1.62±0.11 | |
| Vehicle | 0.68±0.07 | p<0.001 *vs* baseline |
| PS | 1.96±0.49 | p<0.02 *vs.* vehicle |
| Sulindac | 0.60±0.04 | NS *vs* vehicle |

### Conclusions

Similar to the observations with topically administered PS, the oral administration of PS resulted in a significant increase in PWT in a mouse model of migraine compared to vehicle control, in contrast to the oral administration of sulindac which had no significant effect. These positive results affirm data herein in this model with topical PS, and confirm that the PS is not acting as a local analgesic, but instead reaches central sites of action and achieves significant effects on pain known to be generated at central sites (*e.g*., via central sensitization). The ability of PS to act on centrally generated pain even upon oral administration, is particularly surprising, and supported by further observations herein that indicate that orally administered PS reaches central sites of pain generation. The failure of orally administered sulindac to treat pain in this pain model, which generates pain associated with central sensitization, corresponds to the observations with topical sulindac in other models of centrally generated pain, demonstrating that the mere anti-inflammatory activity of sulindac is insufficient to resolve the pain signalling in this model. Sulindac certainly fails to act as an analgesic against neuronal pain signalling, certainly not on pain signalling generated at central sites of action. On the contrary, and without wishing to be bound by theory, the results confirm the broad applicability of PS, as an apparent direct acting nerve analgesic, in treating different forms of neuropathic pain and pain having a central site of generation. Indeed, the allodynia associated with NTG administration is due to activation of nerve signalling through the trigeminal nucleus caudalis, confirming the observations herein that the analgesic activity of PS is distinct from any anti-inflammatory activity, and is more like the direct nerve acting activity of centrally acting agents.

### Example 15: The effect of topiramate, sumatriptan, propranolol and gabapentin in a mouse model of migraine pain

To confirm the clinical applicability of our mouse model of migraine pain, the ability of four compounds - topiramate, sumatriptan, propranolol and gabapentin - to reduce allodynia was tested. These compounds are widely used in the treatment or prophylaxis of pain associated with migraine, and their administration was confirmed to reduce allodynia in this mouse model of migraine pain.

### Methods

In line with the earlier Examples, migraine was induced using 0.4 mg of NTG administered sublingually in the form of a finely crushed 0.4 mg NTG tablet (Greenstone Brand). Mechanical allodynia in response to NTG manifests in 30 minutes and lasts less than 24 hours.

A single intraperitoneal dose of topiramate (30 mg/kg), sumatriptan (600 µg/kg), propranolol (10 mg/kg), gabapentin (30 mg/kg) or vehicle was administered 30 min after the induction of migraine, at the indicated doses.

Mechanical allodynia was determined for all mice study groups below (with 5 mice per group):
1. Group 1: baseline *(i.e,* no NTG treatment)
2. Group 2: vehicle
3. Group 3: topimarate
4. Group 4: sumatriptan
5. Group 5: propanolol
6. Group 6: gabapentin

Mechanical allodynia was determined using the method of von Frey filaments in line with that performed in the earlier Examples. The PWT test was performed 30 minutes after compound or vehicle control administration. The mechanical threshold, expressed as g, indicates the force of the von Frey filament to which the animal reacted.

### Results

As expected, following migraine induction, vehicle-treated mice had lower PWT values compared to baseline values measured prior to the induction of migraine (*i.e.*, sublingual administration of NTG establishes allodynia that is not reverse by administration of vehicle). These results are consistent with the induction of migraine pain. The administration of all four compounds achieved a significant increase in PWT compared to vehicle. The values are provided in Table 15.

**Table 15 - PWT across the study groups**

| **Group** | **PWT,** g | **P value** |
|---|---|---|
| | *Mean ± SEM* | |
| Baseline | 1.68 ± 0.24 | |
| Vehicle | 0.50 ± 0.03 | p<0.00001 *vs* baseline |
| Topiramate | 1.40 ± 0.14 | p<0.0002 *vs* vehicle |
| Sumatriptan | 1.34 ± 0.12 | p<0.0001 *vs* vehicle |
| Propanolol | 1.37±0.14 | p<0.0003 *vs* vehicle |
| Gabapentin | 1.44±0.25 | p<0.01 *vs* vehicle |

### Conclusions

The administration of all four compounds, known to be effective in treating migraine pain, resulted in significant increased in PWT in a mouse model of migraine, compared to vehicle control. These data confirm the clinical applicability of the mouse model for determining the therapeutic potential of compounds in the treatment of migraine pain. This further supports the conclusions in previous Examples that PS is suitable for treating migraine pain, as well as different forms of neuropathic pain and pain having a central site of generation.

Notably, these clinical compounds are known to work directly on central neurons through mechanisms that include reducing action potential firing, thereby attenuating pain signalling. As previously noted, a persistently high state of action potential firing is associated with central sensitisation, which is manifested upon NTG administration in this model. Accordingly, the use of PS as disclosed in the Examples herein achieves analgesic activity similar to that observed for direct nerve acting clinical treatments for migraine pain. These observations further support the hypothesis that PS achieve treatment of pain, for example migraine pain, by direct action on neurons, in particular those involved in generating pain associated with central sensitization.

### Example 16: The effect of orally administered PS, and failure of sulindac, in treating pain in a mouse model of chronic migraine

Building on the observations herein in the mouse model of migraine pain, the analgesic efficacy of orally administered PS was investigated in mice with manifestations of chronic *(i.e.,* on-going) migraine pain. In contrast to the earlier experiments, the pain established in this model is chronic, creating further manifestations of central sensitization, thus generating a more challenging pain model.

### Methods

An NTG-induced chronic migraine model was used (Pradhan et al, 2014) (Moye & Pradhan, 2017). In particular, mice receive sublingual NTG 0.4 mg in the form of a finely crushed 0.4 mg NTG tablet (Greenstone Brand) every two days, receiving a total of five doses. For control mice, an NTG placebo (Practi-Nitroglycerin Sublingual 0.4 mg Simulated Medication tablet) was administered in the same way. The long-term dosing of NTG ensures the effects of central sensitization are generated over a longer period, enabling a consideration of the efficacy of PS in the treatment of pain associated with on-going sensitization from centrally located neurons.

As for the migraine pain model considered elsewhere herein, the ability of NTG to trigger central sensitization, manifested as allodynia, renders this model applicable for both migraine and other pain disorders associated with central sensitization more generally.

PS and sulindac were administered orally to each mouse. PS was administered at 50 mg/kg and sulindac was administered at 33 mg/kg (equimolar to the PS dose), once a day for 11 days, concurrent with NTG administration.

Mechanical allodynia was determined for all mice study groups below:
1. Group 1: placebo NTG *(i.e,* no NTG treatment, n=8)
2. Group 2: NTG (n=8)
3. Group 3: NTG + vehicle (n=8)
4. Group 4: NTG + PS (n=8)
5. Group 5: NTG + sulindac (n=8)

Mechanical allodynia was determined using von Frey filaments in line with that performed in the earlier Examples. The PWT test was performed prior to NTG (or placebo NTG) administration to determine baseline values, as indicated. The PWT test was then subsequently performed 30 minutes after PS, sulindac or vehicle control administration. The mechanical threshold, expressed as g, indicates the force of the von Frey filament to which the animal reacted.

### Results

As expected, following administration of NTG, mice had lower PWT values compared to baseline values measured prior to administration (0.66 ± 0.11 g vs 1.95±0.18 g, p<0.0003). These results are consistent with the induction of pain associated with chronic migraine. The placebo NTG did not significantly alter the baseline value. There was no significant different in PWT values for vehicle-treated mice after 11 days compared to NTG-treated mice *(i.e.,* sublingual administration of NTG establishes allodynia that is not reversed by administration of vehicle). Oral administration of PS, however, achieved a significant increase in PWT compared to vehicle (1.18 ± 0.10 g vs 0.58 ± 0.02 g, p<0.0001). In contrast, in line with observations elsewhere herein, oral administration of sulindac did not significantly increase PWT compared to vehicle. The values are provided in Table 16.

**Table 16 - PWT in the study groups**

| **Group** | **PWT, g** | **P value** |
|---|---|---|
| | *Mean* ± *SEM* | |
| ***Baseline*** | | |
| Placebo NTG | 2.11 ± 0.32 | |
| NTG | 1.95 ± 0.18 | |
| NTG + vehicle | 2.05 ± 0.14 | |
| NTG + PS | 2.28 ± 0.34 | |
| NTG + sulindac | 1.80 ± 0.12 | |

| **Day 11** | | |
|---|---|---|
| Placebo NTG | 1.79 ± 0.11 | NS *vs* baseline placebo |
| NTG | 0.66 ± 0.11 | p<0.0003 *vs* baseline NTG |
| | | p<0.0001 *vs* placebo day 11 |
| NTG + vehicle | 0.58 ± 0.02 | p<0.00001 *vs* baseline vehicle |
| | | NS *vs* NTG day 11 |
| NTG + PS | 1.18 ± 0.10 | p<0.01 *vs* baseline PS |
| | | p<0.0001 *vs* vehicle day 11 |
| NTG + sulindac | 0.43 ± 0.03 | p<0.0001 *vs* baseline sulindac |
| | | NS *vs* vehicle day 11 |

### Conclusions

Similar to the observations with orally administered PS in the migraine pain model herein, the oral administration of PS resulted in a significant increase in PWT in a mouse model of chronic migraine compared to vehicle control, in contrast to the oral administration of sulindac which had no significant effect. These striking results confirm the data herein that orally administered PS can treat pain associated with central sensitization manifested in this model of migraine pain.

Furthermore, the data demonstrate that orally administered PS can continue to treat pain associated with central sensitization over a long period of sustained pain generation and sensation *(i.e.,* corresponding to pain manifestations in chronic migraine). These observations are particularly striking as continued administration of NTG causes consistent activation of centrally located neurons corresponding to the decoupled neuronal signalling experienced during central sensitization, further supporting a role of PS in acting directly on neuronal signalling generated at central sites, in line with further observations herein.

### Example 17: The effect of topiramate and sumatriptan in a mouse model of chronic migraine

Similar to the short-term migraine pain experiments, to confirm the clinical applicability of the chronic migraine pain model, the ability of topiramate and sumatriptan to reduce allodynia was tested.

### Methods

The NTG-induced chronic migraine model was used as described previously.

12 days following the first dose of NTG, a single intraperitoneal dose of topiramate (30 mg/kg), sumatriptan (600 µg/kg), or vehicle was administered.

Mechanical allodynia was determined for all mice study groups below:
1. Group 1: NTG + vehicle (n=8)
2. Group 2: NTG + topiramate (n=8)
3. Group 3: NTG + sumatriptan (n=8)

Mechanical allodynia was determined using von Frey filaments in line with that performed in the earlier Examples. The PWT test was performed prior to NTG administration to determine baseline values and also at day 12, prior to compound administration, as indicated. The PWT test was then subsequently performed 30 minutes after topiramate or sumatriptan administration. The mechanical threshold, expressed as g, indicates the force of the von Frey filament to which the animal reacted.

### Results

As expected, following administration of NTG, vehicle-treated mice had lower PWT values compared to baseline values measured prior to NTG administration. These results are consistent with the induction of migraine pain. The administration of both topiramate and sumatriptan achieved a significant increase in PWT compared to vehicle. The values are provided in Table 17.

**Table 17 - PWT in the study groups**

| **Group** | **PWT, g** | **P value** |
|---|---|---|
| | *Mean* ± *SEM* | |
| ***Baseline*** | | |
| Vehicle | 1.72 ± 0.15 | |
| Topiramate | 2.40 ± 0.24 | |
| Sumatriptan | 1.90 ± 0.06 | |

| **Day 12 (pre-treatment)** | | |
|---|---|---|
| Vehicle | 0.70 ± 0.18 | |
| Topiramate | 0.60 ± 0.00 | |
| Sumatriptan | 0.57 ± 0.02 | |

| **Post-compound administration** | | |
|---|---|---|
| Vehicle | 0.56 ± 0.04 | p<0.0001 *vs* baseline vehicle |
| Topiramate | 1.76 ± 0.11 | p<0.04 *vs* baseline topiramate |
| | | p<0.00001 *vs* Day 12 topiramate |
| | | p<0.0001 *vs* vehicle administration |
| Sumatriptan | 1.33 ± 0.17 | p<0.01 *vs* baseline sumatriptan |
| | | p<0.004 *vs* Day 12 sumatriptan |
| | | p<0.001 *vs* vehicle administration |

### Conclusions

Similar to the observations in the model of short-term migraine pain, the administration of topiramate and sumatriptan, compounds known to be effective in treating migraine pain likely via direct action of neuronal signalling, resulted in significant increase in PWT, compared to vehicle control, in this chronic migraine pain model. These data therefore confirm the clinical applicability of the long-term NTG administration model for determining the therapeutic potential of compounds in treating chronic migraine pain.

### Example 18: Comments on observations in relation to migraine pain

Preliminary in vivo observations demonstrate an unprecedented analgesic activity of PS in treating migraine pain. These observations are surprising, but are consistent with the data in WO2022/251805 and WO2022/251806 (both hereby incorporated by reference in their entirety) where PS is shown to have a direct analgesic effect on two distinct types of neuropathic pain (*i.e.,* associated with CIPN and DPN). In light of the potent analgesic efficacy of PS in two pathophysiologically distinct forms of neuropathic pain, the present inventor hypothesised that PS might have more broadly applicable analgesic activities, perhaps (but without wishing to be bound by theory) based on the ability of PS to reach central sites of action and to directly impact nerve signalling associated with central sensitization. The inventor's initial observations, and the further data in an appropriate animal model of migraine pain, presented herein confirm this hypothesis, demonstrating an unprecedented analgesic activity of PS in treating migraine pain. Further experiments, described herein, using well-established animal models confirm these observations. The inventor expects that PS is having a direct effect on neuronal pain signalling generated by migraine pathophysiology, and in particular is preventing the generation of pain at central sites by reducing signalling caused by central sensitization. This is suggested by evidence indicating the ability of PS to reduce allodynia and supported by the observed ability of PS to be stable in and traverse along centrally projecting neurons towards central sites of action, even upon oral administration. Such therapeutic applicability of PS is unprecedented. In light of the ability of PS to traverse towards central sites upon topical administration, these findings represent a striking contribution in providing a straightforward treatment of pain associated with widespread headache disorders. Indeed, the topical route provides low systemic clearance, reduced drug interactions, increased patient tolerability, and facile combination with oral medications. PS may also be administered orally for treatment of migraine pain - indeed, surprisingly, oral administration in the appropriate animal model achieved analgesia.

These observations demonstrate a previously unrecognised activity and therapeutic utility of PS, a compound which falls within the broader class of NSAIDs but does not share all of the properties of this family of compounds. Indeed, unlike typical NSAIDs, PS does not inhibit activity of COX-1 or COX-2 and does not block the synthesis or activity of prostaglandins. In fact, PS has instead been shown to trigger increased COX activity and significantly increased PGE₂ levels (Mackenzie et al. (2010) Gastroenterology 139(4): 1320-32). As explained herein, migraine is not considered an inflammatory disorder, and is instead considered to have pain pathophysiology arising as a consequence of neuronal dysfunction and sensitization within the trigeminal pathway. Accordingly, a purely anti-inflammatory activity would not achieve the analgesic effect required to treat migraine pain. Indeed, although NSAIDs are postulated to treat migraine, studies have demonstrated that members of this class of compounds are not effective analgesics for this indication. For example, naproxen was shown not to be clinically useful for treating migraine (Law et al. Cochrane Database of Systematic Reviews (2013); 10: 1-45). To the extent NSAIDs are suggested for treating pain *(i.e.,* rather than inflammation) associated with migraine, this activity is limited to their ability to inhibit prostaglandin synthesis by blocking the COX pathway. As explained above, PS does not share these activities of typical NSAIDs. Accordingly, the analgesic activity of PS in treating migraine pain is unexpected. Of course, the treatment of pain *per se,* as suggested by the preliminary observations herein, is distinct from the established role of PS, and typical NSAIDs, as anti-inflammatory agents.

Earlier observations regarding the activity of PS are limited to its anti-inflammatory activity. For example, WO 2019/067919 suggests a role for PS in the treatment of DED, using an acute DED model, in which concanavalin A (ConA) is administered to rabbit lacrimal glands concurrently with PS. In this context, the anti-inflammatory activity of PS results in a limited inflammatory response to ConA, thus preventing the establishment of DED. These observations confirm the anti-inflammatory activity of PS and suggest its utility in preventing establishment and maintenance of inflammatory components of DED. The observations in this acute DED model fail to provide any evidence of the ability of PS as an analgesic, able to act directly on nerves to reduce the dysfunctional nerve signalling and central sensitization implicated in the migraine pain. Any reduction in pain in this acute DED model can be assumed only to be a consequence of PS inhibiting the inflammatory response *(i.e.,* the pathology responsible for triggering activation of the pain sensors). In fact, the results of the DED model suggest that PS improves corneal sensitivity, implicating an increase in nociception, the opposite effect to that desired for an analgesic. Of course, irrespective of any suggestion towards analgesic activity of PS, such activity observed in an acute DED model provides no indication of a corresponding activity in migraine pain.

The observations herein demonstrate that PS has therapeutic utility beyond an anti-inflammatory activity, as suggested for typical NSAIDs. Such NSAIDs, for example naproxen, are ineffective in reducing migraine pain (Law et al. Cochrane Database of Systematic Reviews (2013); 10: 1-45) and such a failure is likely a consequence of the purely anti-inflammatory activity of typical NSAIDs. As explained above, such anti-inflammatory activity is not sufficient to treat migraine pain, which is a purely neuronal disorder. Any suggestions of the activity of typical NSAIDs in treating migraine pain (rather than an inflammatory response) are associated with a requirement for inhibition of the COX pathways and synthesis of prostaglandins *(i.e.,* activities of typical NSAIDs not shared by PS). Without wishing to be bound by theory, the observations herein indicate that PS effectively treats migraine pain by targeting neuronal signalling generated via central sensitization, a key component of pain associated with migraine.

Therefore, the present inventor has demonstrated a new surprising activity for PS in the treatment and/or prevention of migraine pain. As outlined above, this activity represents a mechanism unique to PS compared to typical NSAIDs. Furthermore, the ease by which PS can be administered, for example topically, and its limited adverse effects (Mackenzie et al. (2010) Gastroenterology 139(4): 1320-32) render it an improved therapy for migraine pain.

Furthermore, surprisingly the analgesic activity of PS was maintained even upon oral administration in animal models of migraine pain. The ability of PS to act on migraine pain in an animal model in which the pain is manifested to trigger central sensitization, is particularly surprising, and supported by further observations herein that indicate that orally administered PS reaches central sites of pain generation.

### Example 19: The effect of PS in treating neuropathic pain associated with PHN

Preliminary in vivo evidence indicates that the topical administration of PS, in the form of a hydrogel, achieves a striking analgesic effect on neuropathic pain associated with PHN. This effect was surprising but is consistent with the data in WO2022/251805 and WO2022/251806 where PS is shown to have a direct analgesic effect on two distinct types of neuropathic pain (*i.e.,* associated with CIPN and DPN).

### Example 20: The effect of PS, and failure of sulindac, in treating and/or preventing neuropathic pain in a mouse model of PHN

Neuropathic pain associated with PHN can be regenerated in a rat animal model using injection of varicella zoster virus (VZV) into the footpad of a rat. The VZV reaches the dorsal root gangia and generates symptoms corresponding to allodynia and hyperalgesia. Therefore, this model generates particular phenotypes observed in PHN in humans, including, for example, allodynia.

The model is carried out according to the procedure originally developed by Sadzot-Delvaux et al. (Neurology (1995); 45(12 Suppl 8): S18-20). Briefly, rats are inoculated subcutaneously with VZV in the footpad and then subjected to behavioral tests that include measurement of paw withdrawal time in response to mechanical or thermal stimuli. The paw withdrawal threshold decreases significantly within 3-5 days post-injection and remains low for over a month, before resolving within 100 days of injection. After development of allodynia, the rats are assessed for hind paw mechanical withdrawal thresholds, an indication of mechanical allodynia. Sensory testing utilizes von Frey filaments. Allodynia is tested by perpendicularly touching the hindpaw causing slight buckling of the filament. Based on the response pattern and the force of the final filament, the hindpaw withdrawal threshold is calculated.

Corresponding mice models are available, as described herein.

### Methods

Adult male BALB/c mice, 8 weeks of age at the beginning of the experiments and weighing 20-30 g, were purchased from The Jackson Laboratory (Bar Harbor, ME). Mice were housed in an AAALAC-accredited facility in groups of four. Food and water were available *ad libitum.* Experiments were performed during the light cycle (7:00 am to 7:00 pm) and animals were euthanized with CO₂ asphyxiation. The mice in each cage were randomly allocated to treatment groups. All studies were conducted by experimenters blinded to the identity of the treatment groups.

Studies were approved by the Institutional Animal Care and Use Committee of Stony Brook University and followed the National Institutes of Health Guidelines for the Care and Use of Laboratory Animals. Animal studies are reported in compliance with the ARRIVE guidelines (Kilkenny et al, 2010).

PHN was induced in mice by infecting them with the HSV-1 KOS strain virus and following established protocols (Ou et al, Front Immunol, 14, 1026269, 2023; Takasaki et al, Anesthesiology, 96(5), 1168-74, 2002).

Briefly, BALB/c male mice were anesthetized with isoflurane (2%) and their mid-flank and right paw were clipped and depilated with a chemical depilatory (Veet Hair Remover; Reckitt Benckiser). Three days later, HSV-1 (10⁶ PFUs in 20 µl PBS) was inoculated on the shin of the right hind paw (5x5 mm) after scarification with sandpaper. The virus was applied directly to the scarified area. The contralateral hind paw was without inoculation. Mock infection was performed using the same virus inactivated by heating at 60°C for 1 h.

At the development stage of skin lesions (3-5 days post inoculation) many vesicles appeared on the back of the mice and the surrounding inoculated area and were characterized as mild-to-moderate herpes zoster-like lesions.

Five days post-inoculation, PS (as a 5% gel), or vehicle control were administered topically onto both hind paws of mice three times a day for three days.

Mechanical allodynia was determined for all mice study groups below:
1. Group 1: sham inoculated (n=5-7)
2. Group 2: vehicle control (n=5-7)
3. Group 3: PS 5% (n=5-7)

In a second experiment, the effect of the parent compound, sulindac, was tested in the same mouse model. Five days post-inoculation, sulindac (as a 5% gel), or vehicle control were administered topically onto both hind paws of mice three times a day for three days.

Mechanical allodynia was determined for all mice study groups below:
1. Group 1: vehicle control (n=5-7)
2. Group 2: sulindac 5% (n=5-7)

For determining mechanical allodynia, pain threshold responses were measured using the well-established method of von Frey filaments in line with that performed in the earlier Examples. The PWT test, assessing mechanical allodynia, was performed either pre-inoculation, five days post-inoculation, or three days after treatment, as indicated. The mechanical threshold, expressed as g, indicates the force of the von Frey filament to which the animal reacted.

### Results

Allodynia was evident on both hind paws, i.e., ipsilateral, and contralateral to the virus inoculation site, and was statistically no different in intensity between them, as reported previously (Chen & Pan, Brain Res, 1042(1), 108-13, 2005).

As expected, and outlined in Table 18A, sham inoculated mice had similar PWT values compared to pre-inoculation mice, indicating the sham fails to generate any pain. Five days post induction, a significant decrease in PWT values was observed in both inoculated groups, reflecting successful induction of neuropathic pain associated with PHN. Remarkably, administration of PS was able to achieve a significant increase in PWT compared to vehicle post-treatment (p < 0.0001). The results are provided in Table 18A.

**Table 18A - PWT across the study groups**

| **Group** | **PWT, g** | **P value** |
|---|---|---|
| | *Mean* ± *SEM* | |
| **Pre-inoculation** | | |
| Sham | 1.22 ± 0.08 | |
| Vehicle | 1.27 ± 0.07 | |
| PS | 1.27 ± 0.19 | |

| **Post-inoculation, day 5** | | |
|---|---|---|
| Sham | 1.16 ± 0.12 | NS *vs* pre-inoculation sham |
| Vehicle | 0.52 ± 0.04 | p<0.001 *vs* sham |
| PS | 0.53 ± 0.06 | p<0.001 *vs* sham |

| **Post-treatment** | | |
|---|---|---|
| Sham | 1.18±0.14 | NS *vs* post-inoculation sham |
| | | p<0.05 *vs* vehicle |
| Vehicle | 0.52 ± 0.01 | NS *vs* post-inoculation vehicle |
| PS | 0.93 ± 0.06 | p<0.0001 *vs* vehicle |

In the second experiment, the administration of sulindac post-inoculation did not improve PWT values compared to vehicle control (0.56 ± 0.06 *vs* 0.65±0.07; NS). The values are provided in Table 18B.

**Table 18B - PWT across the study groups**

| **Group** | **PWT, g** | **P value** |
|---|---|---|
| | *Mean* ± *SEM* | |
| **Post-inoculation and treatment** | | |
| Vehicle | 0.65±0.07 | |
| Sulindac | 0.56 ± 0.06 | NS *vs* vehicle |

### Conclusions

The topical administration of PS significantly increased the PWT in mice with PHN compared to vehicle control. Therefore, strikingly, PS treats the pain associated with PHN, a form of neuropathic pain known to be particularly difficult to treat.

As discussed herein, the pain associated with PHN is associated with central sensitisation, as manifested by generation of allodynia. Therefore, these results, in combination with the observations herein of the accumulation of PS at central sites of action, point not only to a the analgesic activity of PS in treating neuropathic pain associated with PHN, but also to an unprecedented and surprisingly broad applicability of PS in treating and preventing pain having central mechanisms (*i.e.,* generated in central sites or due to central sensitization), even in particularly challenging models of neuropathic pain.

In contrast, the parent compound, sulindac, was not able to improve PWT values compared to vehicle control. This was despite the exploitation of high relative concentrations of sulindac, again confirming that the anti-inflammatory activity of typical NSAIDs is insufficient to treat the challenging pain generated in these models.

### Example 21: Comments on preliminary observations in relation to PHN

Preliminary in vivo observations demonstrate an unprecedented analgesic activity of PS in treating neuropathic pain associated with PHN. These observations are surprising, but are consistent with the data in WO2022/251805 and WO2022/251806 (both hereby incorporated by reference in their entirety) where PS is shown to have a direct analgesic effect on two distinct types of neuropathic pain (*i.e.,* associated with CIPN and DPN). In light of the potent analgesic efficacy of PS in two pathophysiologically distinct forms of neuropathic pain, the present inventor hypothesised that PS might have more broadly applicable analgesic activities in other forms of neuropathic pain, perhaps (but without wishing to be bound by theory) based on the ability of PS to traverse towards central sites of action and to directly impact nerve signalling associated with central sensitization. The inventor's initial observations herein confirm this hypothesis, demonstrating an unprecedented analgesic activity of PS in treating neuropathic pain associated with PHN. Such a broad applicability as an analgesic across different forms of neuropathic pain is not guaranteed - indeed, gabapentin shows different efficacy in the treatment of different forms of neuropathic pain. Further experiments using well-established animal models of neuropathic pain associated with PHN will confirm these observations. The inventor expects that PS is having a direct effect on neuronal pain signalling generated in response to the reactivation of VZV and the propagation of the virus causing nerve damage both in the periphery and at central sites, and may be preventing the generation of pain at central sites by reducing signalling caused by central sensitization. Such a therapeutic applicability of PS is unprecedented. In light of the ability of PS to traverse towards central sites upon topical administration, these findings represent a striking contribution in providing a straightforward treatment of neuropathic pain associated with PHN. Indeed, the topical route provides low systemic clearance, reduced drug interactions, increased patient tolerability, and facile combination with oral medications.

These observations demonstrate a previously unrecognised activity and therapeutic utility of PS, a compound which falls within the broader class of NSAIDs but does not share all of the properties of this family of compounds. Indeed, unlike typical NSAIDs, PS does not inhibit activity of COX-1 or COX-2 and does not block the synthesis or activity of prostaglandins. In fact, PS has instead been shown to trigger increased COX activity and significantly increased PGE₂ levels (Mackenzie et al. (2010) Gastroenterology 139(4): 1320-32). The observations herein indicate a role of PS as an analgesic, targeting pain signalling associated with PHN, an activity which is distinct from the established role of PS, and typical NSAIDs, as anti-inflammatory agents. Indeed, such anti-inflammatory activity has been shown to be ineffective in the treatment of neuropathic pain associated with PHN (Moore et al. (Cochrane Database of Systematic Reviews (2015); 10: 1--25)).

Earlier observations regarding the activity of PS are limited to its anti-inflammatory activity. For example, WO 2019/067919 suggests a role for PS in the treatment of DED, using an acute DED model, in which concanavalin A (ConA) is administered to rabbit lacrimal glands concurrently with PS. In this context, the anti-inflammatory activity of PS results in a limited inflammatory response to ConA, thus preventing the establishment of DED. These observations confirm the anti-inflammatory activity of PS and suggest its utility in preventing establishment and maintenance of inflammatory components of DED. The observations in this acute DED model fail to provide any evidence of the ability of PS as an analgesic, able to act directly on nerves to reduce nerve signalling caused by neuropathic pain. Any reduction in pain in this acute DED model can be assumed only to be a consequence of PS inhibiting the inflammatory response (*i.e.,* the pathology responsible for triggering activation of the pain sensors). In fact, the results of the DED model suggest that PS improves corneal sensitivity, implicating an increase in nociception, the opposite effect to that desired for an analgesic. Of course, irrespective of any suggestion towards analgesic activity of PS, such activity observed in an acute DED model provides no indication of a corresponding activity in neuropathic pain, and certainly not in neuropathic pain associated with PHN.

The observations herein indicate that PS has therapeutic utility beyond an anti-inflammatory activity associated with typical NSAIDs. In contrast, certain NSAIDs, are ineffective analgesics in treating neuropathic pain associated with PHN. Indeed, Moore et al. (Cochrane Database of Systematic Reviews (2015); 10: 1-25), outlined that NSAIDs have no therapeutic efficacy in peripheral neuropathic pain associated with PHN. The activity of PS suggested herein contrasts with the inability of typical NSAIDs to provide a direct analgesic effect on damaged neurons in neuropathic pain as observed in the prior art. Without wishing to be bound by theory, the reason for the absence of any response to typical NSAIDs in the prior art is likely that the pain is caused by neuropathic nerve damage, rather than by inflammation (*i.e.,* any anti-inflammatory activity of typical NSAIDs is not sufficient to prevent or treat the neuropathic pain). Accordingly, the analgesic activity of PS indicated herein is unique and not shared by typical NSAIDs. Any alleged analgesic activity of NSAIDs observed in the prior art is a reflection of their anti-inflammatory activity (*i.e.,* stopping potential triggers causing the pain) rather than an actual analgesic activity directed towards nerve signalling (*i.e.,* that would result in a reduction in pain caused by nerve damage and sensitization). Indeed, as noted above, the anti-inflammatory activity of typical NSAIDs has been considered to have no therapeutic utility in treating neuropathic pain associated with PHN. Irrespective, any alleged activity of typical NSAIDs in treating neuropathic pain associated with PHN cannot be extrapolated to PS in light of the distinct activities of PS compared to typical NSAIDs - the observations herein indicate the utility of PS in treating neuropathic pain associated with PHN. Any suggestions that typical NSAIDs have analgesic activity are associated with a requirement for inhibition of the COX pathways and synthesis of prostaglandins (*i.e.,* activities of typical NSAIDs that are not shared by PS). Without wishing to be bound by theory, the observations herein indicate that PS effectively treats neuropathic pain associated with PHN by targeting neuronal signalling generated via central sensitization, a key component of neuropathic pain associated with PHN.

Therefore, the present inventor indicates a new surprising activity for PS in the treatment and/or prevention of neuropathic pain associated with PHN. As outlined above, the preliminary observations implicate an activity of PS that goes beyond the anti-inflammatory activity previously observed for PS and related NSAIDs.Furthermore, the ease by which PS can be administered, for example topically, and its limited adverse effects (Mackenzie et al. (2010) Gastroenterology 139(4): 1320-32) render it an improved therapy for neuropathic pain associated with PHN.

### Example 22: The effect of PS in treating corneal neuropathic pain

Preliminary in vivo evidence indicates that the topical administration of PS to the ocular surface, achieves a striking analgesic effect on corneal neuropathic pain. Furthermore, topical administration of PS to the outer surface of the eyelid dramatically reduced chronic pain in a subject after kerato-refractive surgery (LASIK). This effect was surprising but is consistent with the data in WO2022/251805 and WO2022/251806 where PS is shown to have a direct analgesic effect on two distinct types of neuropathic pain (*i.e.,* associated with CIPN and DPN).

### Example 23: Assessing the effect of PS in treating corneal neuropathic pain in a rabbit model

Animal models involving damage to the ocular surface rely heavily on induction of corneal inflammation which can interfere with observations regarding treatment of corneal neuropathic pain. Instead, corneal neuropathic pain can be generated in a rabbit model in which the ciliary branches innervating the cornea are ligated. The compression of the nerves generates sensitization causing pain hypersensitivity.

The model is a modified version of the procedure developed by Seyed-Razavi *et al.*

(Investigative Ophthalmology & Visual Science (2017); 58:3951). The model herein uses rabbits rather than mice. In summary, following a lateral canthotomy, two tractional nylon sutures are placed temporally and the lateral conjunctival fornix incised circumferentially (90 degrees). The eye globe is rotated nasally by gently pushing the nasal conjunctival fornix with blunt tip curved forceps. A silk suture is placed and tightened around the exposed optic nerve and surrounding ciliary nerve branches, ligating the ciliary nerves without severing them. Rabbits undergoing all surgical steps yet lacking the ligation suture serve as sham controls. Following the procedure, two nylon sutures are used to join the eyelids together. Subsequently, different behavioural responses can be monitored to demonstrate the establishment of central sensitization without any associated corneal peripheral pathology, an indication of corneal neuropathic pain. Exemplary behavioural responses include the occurrence of eye-wiping, blinking, and/or squinting. For completeness, these behaviors can be further triggered by drops of hyperosmolar saline solution. Indeed, as observed in Seyed-Razavi *et al.* mice with nerve ligation show an increase in the number of nociceptive paw wipes to the affected eye. Therefore, this is an appropriate model for determining the efficacy of analgesic agents in the treatment of corneal neuropathic pain. In this model of corneal neuropathic pain, acute acting analgesics (*i.e.,* with activity on nociceptors in the cornea) will not effectively resolve the hypersensitivity as this is generated as a result of dysfunction of centrally located neurons (*i.e.*, central sensitization).

### Example 24: Comments on preliminary observations in relation to corneal neuropathic pain

Preliminary *in vivo* observations demonstrate an unprecedented analgesic activity of PS in treating corneal neuropathic pain. These observations are surprising, but are consistent with the data in WO2022/251805 and WO2022/251806 (both hereby incorporated by reference in their entirety) where PS is shown to have a direct analgesic effect on two distinct types of neuropathic pain (*i.e.,* associated with CIPN and DPN). In light of the potent analgesic efficacy of PS in two pathophysiologically distinct forms of neuropathic pain, the present inventor hypothesised that PS might have more broadly applicable analgesic activities in other forms of neuropathic pain, perhaps based on the ability of PS to traverse towards central sites of action (even upon topical eyelid administration) and to directly impact nerve signalling associated with central sensitization. The inventor's initial observations herein confirm this hypothesis, demonstrating an unprecedented analgesic activity of PS in treating corneal neuropathic pain. Indeed, PS demonstrated a striking reduction in corneal neuropathic pain experienced by a subject after LASIK surgery. Such a broad applicability as an analgesic across different forms of neuropathic pain is not guaranteed - indeed, gabapentin shows different efficacy in the treatment of different forms of neuropathic pain. Further experiments using the animal model of corneal neuropathic pain will confirm these observations. Without wishing to be bound by theory, the inventor expects that PS is having a direct effect on neuronal pain signalling generated by central sensitization associated with corneal neuropathic pain. Such a therapeutic applicability of PS is unprecedented. In light of the ability of PS to traverse towards central sites of action upon topical administration (even to the outer surface of the eyelid), these findings represent a striking contribution in providing a straightforward treatment of corneal neuropathic pain. Indeed, the topical route, certainly via the outer surface of the eyelid, provides low systemic clearance, reduced drug interactions, increased patient tolerability, and facile combination with oral medications.

These observations demonstrate a previously unrecognised activity and therapeutic utility of PS, a compound which falls within the broader class of NSAIDs but does not share all of the properties of this family of compounds. Indeed, unlike typical NSAIDs, PS does not inhibit activity of COX-1 or COX-2 and does not block the synthesis or activity of prostaglandins. In fact, PS has instead been shown to trigger increased COX activity and significantly increased PGE₂ levels (Mackenzie et al. (2010) Gastroenterology 139(4): 1320-32). The observations herein indicate a role of PS as an analgesic, targeting pain signalling associated with corneal neuropathic pain, an activity which is distinct from the established role of PS, and typical NSAIDs, as anti-inflammatory agents. Indeed, such anti-inflammatory activity has been shown to be ineffective in the treatment of neuropathic pain (Moore et al. (Cochrane Database of Systematic Reviews (2015); 10: 1-25)). In line with this, for corneal neuropathic pain patients, anti-inflammatory drugs are ineffective.

Earlier observations regarding the activity of PS are focused on its anti-inflammatory activity and provide no indication of an analgesic activity in corneal neuropathic pain. For example, WO 2019/067919 suggests a role for PS in the treatment of DED, using an acute DED model, in which concanavalin A (ConA) is administered to rabbit lacrimal glands concurrently with PS. In this context, the anti-inflammatory activity of PS results in a limited inflammatory response to ConA, thus preventing the establishment of DED. These observations confirm the anti-inflammatory activity of PS and suggest its utility in preventing establishment and maintenance of inflammatory components of DED. The observations in this acute DED model fail to provide any evidence of the ability of PS to act directly on nerves to reduce nerve signalling caused by neuropathic pain. Any reduction in pain in this acute DED model can be assumed only to be a consequence of PS inhibiting the inflammatory response (*i.e.,* the pathology responsible for triggering activation of the pain sensors). In addition to indicating the anti-inflammatory activity of PS in DED, the authors of WO 2019/067919 suggest that PS decreases corneal sensitivity in an acute corneal sensation model. These observations of alleged reduced sensitivity to an acute stimulus, which clearly does not generate persistent pain (*i.e.,* sensation occurring in the absence of the peripheral stimulus), do not suggest the ability of PS to treat pain associated with central sensitization, a mechanism known to be involved in for corneal neuropathic pain. Indeed, the effect of PS in this acute model was observed immediately, implicating local action of PS, not dissimilar to the activity observed for the typical NSAID, ketorolac, in the same model. Such peripheral activity does not demonstrate an efficacy of PS in treating pain generated at central sites of action. Indeed, clinical guidance in the field recommends avoiding the use of NSAIDs for the treatment of types of pain associated with central sensitization (*e.g.*, neuropathic pain), and ketorolac has been shown to have limited analgesic activity in models of such pain. Furthermore, the results of the DED model suggest that PS restores suppressed corneal sensitivity, implicating a role for PS in increasing rather than reducing nociception. Of course, irrespective of any suggestion towards analgesic activity of PS, such activity observed in an acute DED model provides no indication of a corresponding activity in corneal neuropathic pain.

The observations herein demonstrate that PS has therapeutic utility beyond an anti-inflammatory activity associated with typical NSAIDs. In contrast, NSAIDs are ineffective analgesics in treating neuropathic pain. Indeed, Moore et al. (Cochrane Database of Systematic Reviews (2015); 10: 1-25), outlined that NSAIDs have no therapeutic efficacy in neuropathic pain. The activity of PS suggested herein contrasts with the inability of typical NSAIDs to provide a direct analgesic effect on sensitized neurons in neuropathic pain as observed in the prior art. Without wishing to be bound by theory, the reason for the absence of any response to typical NSAIDs in the prior art is likely that the pain is caused by neuropathic nerve damage, rather than by inflammation (*i.e.,* any anti-inflammatory activity of typical NSAIDs is not sufficient to prevent or treat the neuropathic pain). Accordingly, the analgesic activity of PS indicated herein is unique and not shared by typical NSAIDs. Any alleged analgesic activity of NSAIDs observed in the prior art is a reflection of their anti-inflammatory activity (*i.e.,* stopping potential triggers causing the pain) rather than an actual analgesic activity directed towards nerve signalling (*i.e.,* that would result in a reduction in pain caused by sensitization). Irrespective, any alleged activity of typical NSAIDs cannot be extrapolated to PS in light of the distinct activities of PS. Any suggestions that typical NSAIDs have analgesic activity are associated with a requirement for inhibition of the COX pathways and synthesis of prostaglandins (*i.e.,* activities of typical NSAIDs that are not shared by PS). Without wishing to be bound by theory, the observations herein indicate that PS treats corneal neuropathic pain by targeting neuronal signalling generated via central sensitization, a key component of corneal neuropathic pain.

Therefore, the present inventor has demonstrated a new surprising activity for PS in the treatment of corneal neuropathic pain. As outlined above, the observations implicate an activity of PS that goes beyond the activity previously observed for PS and related NSAIDs. Furthermore, the ease by which PS can be administered, for example topically, and its limited adverse effects (Mackenzie et al. (2010) Gastroenterology 139(4): 1320-32) render it an improved therapy for corneal neuropathic pain via topical administration, in particular to the outer surface of the eyelids.

### Example 25: Pharmacokinetics and biodistribution of PS

Given, as demonstrated herein, the broad applicability of PS for treating and/or preventing pain with central sites of action, for example pain associated with central sensitization, neuropathic pain associated with PTPN, migraine pain, neuropathic pain associated with PHN, and corneal neuropathic pain, may be a reflection of the ability of PS to reach and remain stable in particular sites of action. Accordingly, the site of action of PS was investigated. Despite being topically administered, PS was found to traverse within nerves from the periphery towards central sites.

### Methods

PS 8% ointment was applied topically to each hind paw (50 µl per paw) with gentle rubbing. At 0.5, 1, 3, 5, 12, 18, and 24 h, mice (n =4-5 mice/time point) were euthanized with CO₂ inhalation. Blood was drawn immediately after death. Tissues, including paw skin, paw muscle, leg muscle, the sciatic nerve and lumbar DRG bilaterally were dissected quickly, immediately frozen in liquid nitrogen and stored at -80°C until analyzed.

In a separate experiment we studied 8 mice with paclitaxel-induced PN treated with PS 8% ointment 3x/d for 2 wks. Mice were euthanized as above 30 minutes after the last dose of PS. From these mice we harvested both sciatic nerves dividing each into proximal and distal halves, and combined the corresponding halves of every two animals for assay of drug levels.

As previously described (Wen et al., Int J Pharm (2019); 557:273-279), each plasma sample was mixed with double volume of acetonitrile and centrifuged at 13,200 rpm for 15 min. Tissue samples were weighed, ddH₂O (100-300 µL, depending on tissue weight) was added and they were homogenized. Following addition of acetonitrile (twice the volume of the homogenate), the mixture was sonicated for 10 min, centrifuged at 13,200 rpm for 15 min, and analyzed by HPLC, as reported (Wen *et al.,* 2019). The limit of quantitation is 0.1 µM for PS and 0.05 µM for sulindac, sulindac sulfone, sulindac sulfide and their glucuronidated derivatives.

### Results

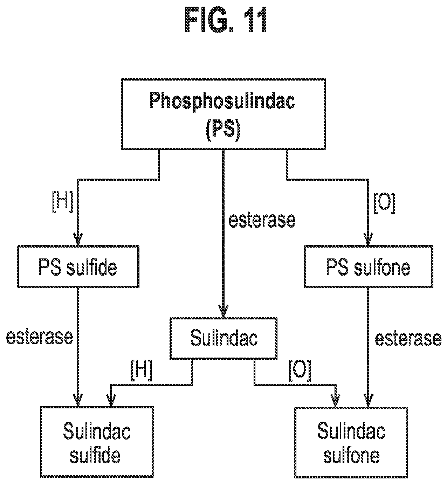

PS can be rapidly metabolized into several metabolites both *in vitro* and *in vivo* that include PS sulfide, PS sulfone, sulindac, sulindac sulfide and sulindac sulfone (Figure 11). Glucuronides of sulindac and its metabolites, mainly forming in the liver, have also been identified. Since the metabolism and PK/biodistribution of PS vary depending on its route of administration, we studied both in normal mice in which PS was administered topically to their hind paws, with particular attention to the sciatic nerve and the dorsal root ganglia (DRG), as examples of peripheral and more centrally located neuronal sites.

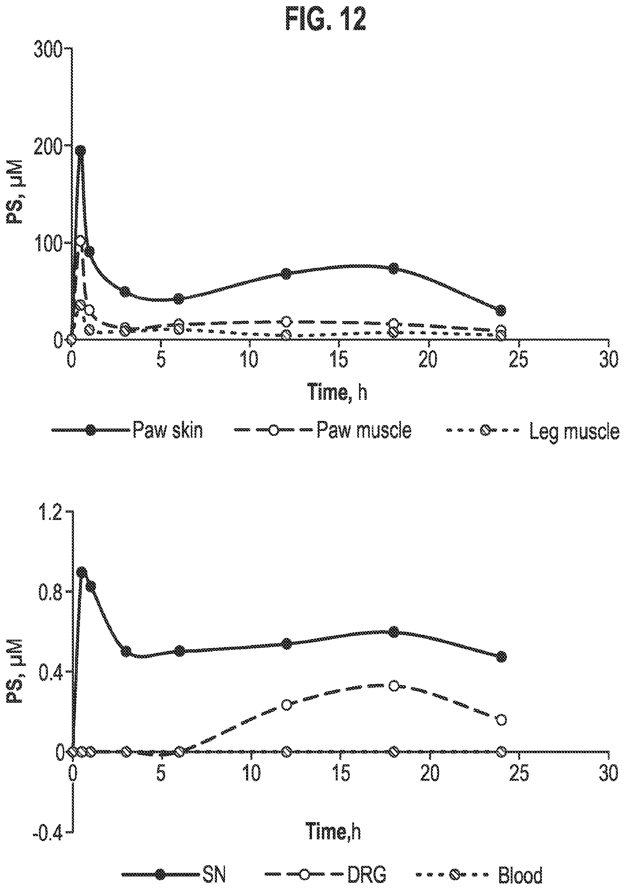

As shown in Figure 12 and Table 19, PS was detected in paw skin, the site of its application, the muscles underneath the skin, leg muscles, the sciatic nerve, and DRG. As expected (Xie et al., Br J Pharmacol (2012a); 165:2152-2166), no PS was detected in the systemic circulation.

The concentration of PS progressively decreased from the skin to its most distant DRG, evidenced by the respective values of both Cₘₐₓ (from 194.7 ± 5.3 µM to 0.3 ± 0.1 µM) and AUC₀₋₂₄ₕ (from 1,609.8 µM·h to 4.5 µM·h). The Tₘₐₓ of PS was the same in all tissues (0.5 h) with the exception of DRG that showed a prolonged Tₘₐₓ (18 h), reflecting perhaps the manner in which PS reaches it, as discussed below. Another interesting feature is the difference in t_{1/2} of the skin and the muscles, which is within a relatively narrow range (11.4 - 20.6 h), in contrast to the much-prolonged value of 57.4 h in the sciatic nerve and the likely even more prolonged value in DRG, which could not be determined with reasonable accuracy.

These differences indicate differential metabolic capacity regarding PS between the nerve and skin and muscles.

**Table 19 - PK parameters of PS in mouse tissue and peripheral blood of normal mice**

| **Tissue** | **Cₘₐₓ, µM** | **Tₘₐₓ, h** | **T_{1/2}, h** | **AUC₀₋₂₄ₕ, µM·h** |
|---|---|---|---|---|
| | *mean* ±*SEM* | | | |
| Paw skin | 194.7 ± 5.3 | 0.5 | 16.1 | 1,609.8 |
| Paw muscle | 101.7 ± 4.6 | 0.5 | 11.4 | 411.2 |
| Leg muscle | 35.0 ± 3.6 | 0.5 | 20.6 | 171.6 |
| SN | 0.9 ± 0.1 | 0.5 | 57.4 | 12.0 |
| DRG | 0.3 ± 0.1 | 18 | * | 4.5 |
| Blood | ** | ** | ** | ** |

| | | | | |
|---|---|---|---|---|
| *, cannot be determined. **, cannot be calculated because intact PS was undetectable. N=4-5 mice/time point. | | | | |

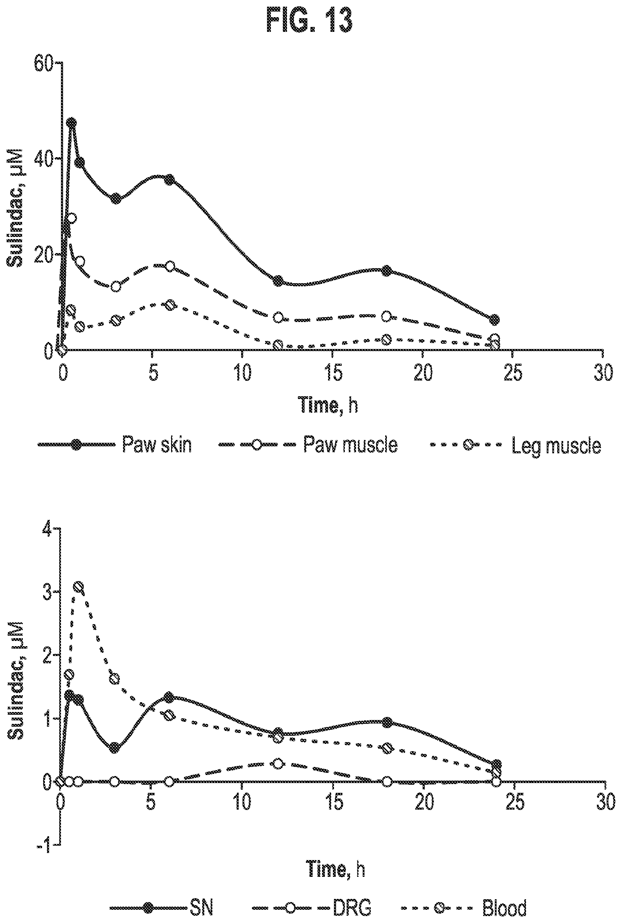

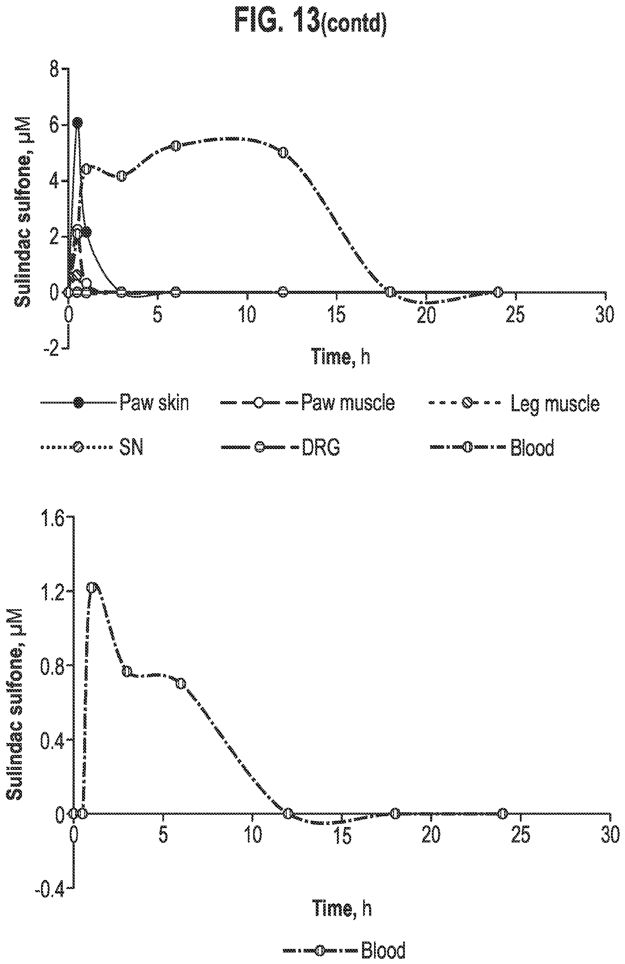

Only three metabolites of PS were detected: sulindac, sulindac sulfone, and sulindac sulfide, (Figure 13 and Table 20). No glucuronidated products were detected. Sulindac was the quantitatively dominant metabolite, with sulindac sulfide and sulindac sulfone levels being <20% of those of sulindac. Sulindac levels were around 25% of those of PS in all tissues except the sciatic nerve (higher) and DRG (equal).

**Table 20 - PK parameters of sulindac in mouse tissue and peripheral blood of normal mice**

| **Tissue** | **Cₘₐₓ, µM** | **Tₘₐₓ, h** | **T_{1/2}, h** | **AUC₀₋₂₄ₕ, µM·h** |
|---|---|---|---|---|
| | *mean* ±*SEM* | | | |
| Paw skin | 47.4 ± 3.8 | 0.5 | 47.4 | 516.6 |
| Paw muscle | 27.5 ± 3.3 | 0.5 | 7.9 | 238.6 |
| Leg muscle | 8.4 ± 2.1 | 0.5 | 8.2 | 71.8 |
| SN | 1.4 ± 0.3 | 0.5 | 12.3 | 22.3 |
| DRG | 0.3 ± 0.1 | 12 | * | 1.6 |
| Blood | 3.1± 0.5 | 1 | 6.2 | 21.2 |

| | | | | |
|---|---|---|---|---|
| *, cannot be determined. N =4-5 mice/time point. | | | | |

The absence of PS in the circulation, the very high Tₘₐₓ of DRG compared to all others, and the lower levels of PS in DRG compared to the sciatic nerve suggest that PS reached the DRG by traversing from the skin through the sciatic nerve.

To further explore this conclusion, we compared the levels of PS in the proximal and distal half of the sciatic nerve of mice 30 min after its application to the hind paw. The two values were strikingly different, with those of the distal half being 18.5-fold higher than those of its proximal half (17 ± 5.1 µM vs. 0.9 ± 0.3 µM; Table 21). The concentration of the three metabolites of PS (sulindac, sulindac sulfide and sulindac sulfone) was also higher in the distal half compared to the proximal half (4.5 - 8.5-fold higher). These findings support the notion that PS reaches the DRG from the site of its application by direct tissue transfer or transport and not via the circulation.

**Table 21 - PS and its metabolites in the proximal and distal half of the sciatic nerve of mice**

| | **Distal** | **Proximal*** | **Fold difference** |
|---|---|---|---|
| | | µM*, mean*±*SEM* | |
| PS | 17.0 ± 5.1 | 0.9 ± 0.3 | 18.9 |
| Sulindac | 3.4 ± 1.1 | 0.4 ± 0.03 | 8.5 |
| Sulindac sulfide | 3.5 ± 1.0 | 0 | -- |
| Sulindac sulfone | 0.9 ± 0.2 | 0.2 ± 0.08 | 4.5 |

| | | | |
|---|---|---|---|
| *All differences between proximal and distal values are statistically significant (p<0.001). N=8 mice/group. | | | |

### Conclusions

These experiments demonstrate that topically administered PS can reach key sites of action known to be involved in the generation of pain associated with the indications described herein (*e.g.,* pain associated with central sensitization; neuropathic pain associated with PTPN; neuropathic pain associated with PHN; migraine pain (and pain of other headache disorders); and corneal neuropathic pain). Furthermore, in light of its rapid metabolism in the bloodstream, the results demonstrate that PS reaches these sites of action by traversing along peripheral neurons towards the central nervous system, being found in meaningful concentrations in the DRG. Therefore, without wishing to be bound by theory, these observations confirm that PS likely performs its analgesic activity, in the indications disclosed herein, directly on neurons and likely within central sites of action, similar to the activity of direct nerve targeting and centrally acting analgesics such as lidocaine and pregabalin. Indeed, based on the ability of topical PS to traverse along peripheral neurons towards central sites of action in these experiments, one would expect topical administration of PS to the skin in the vicinity peripheral nerves to allow an accumulation of PS in higher order neurons responsible for the chronic signalling associated with central sensitization. For example, in PTPN, one would expect topical administration of PS to the skin in the vicinity of the injured peripheral nerve to allow an accumulation of PS within proximal peripheral neurons or in higher order neurons responsible for the chronic signalling associated with central sensitization. For example, in PHN, one would expect topical administration of PS to the skin of one or more thoracic dermatomes to allow an accumulation of PS within one or more thoracic nerves or in higher order neurons responsible for the chronic signalling associated with central sensitization. For example, for migraine, one would expect topical administration of PS to the skin in the vicinity of the trigeminal neuron to allow an accumulation of PS within neurons of the trigeminal pathway, for example the trigeminal ganglion, or further towards the CNS in the trigeminal nucleus caudalis, within the trigeminocervical complex.

The observations of accumulation of PS in central sites of action are supported by the observations that PS clearly inhibits neuronal pain signalling at central sites of action (*e.g.,* the analgesic effect against central sensitization triggered by NTG, the analgesic effect upon administration to a contralateral, unoperated, paw, and the ability of orally administered PS to get to central sites of pain generation and achieve meaningful analgesia).

### Example 26: Pharmacokinetics and biodistribution of PS upon administration to outer surface of eyelid

Given the ability of PS to traverse along peripheral nerves, the inventor investigated whether topical administration of PS to the outer surface of the eyelid would permit sufficient accumulation of PS on the cornea to enable PS to traverse along peripheral corneal nerves towards central sites of action associated with generation of corneal neuropathic pain.

### Methods

The pharmacokinetics and biodistribution of 3% PS gel applied to the outer surface of both upper eyelids was studies in male New Zealand White rabbits.

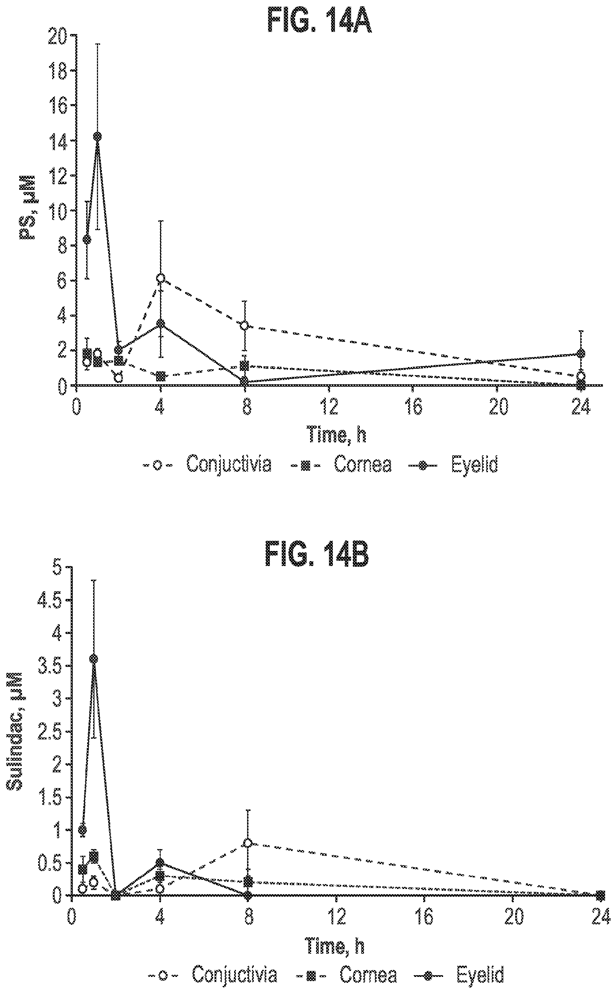

Three rabbits (n = 6 eyes) were sacrificed at each of the time points shown in Figure 14. Eyelid and tissues of the globe were dissected and frozen quickly in liquid nitrogen until the levels of PS and its metabolites were determined by HPLC as described Xie et al. (Br J Pharmacol (2012);165:2152-66).

### Results

As shown in Figure 14A and Tables 22 and 23, aside from the eyelid, to which the PS gel was applied, PS achieved therapeutically relevant levels in the cornea and conjunctiva, indicating sufficient penetration through the eyelid. The distribution of PS was restricted to the surface tissues of the eye (anterior chamber), being undetectable in the aqueous humor and the entire posterior chamber. The PK data further demonstrate that PS is particularly stable in the cornea. Accordingly, PS can remain in the corneal tissue (*i.e.,* exposed to centrally projecting corneal nerves) at therapeutic levels for long periods.

**Table 22 - Amount of PS in rabbit ocular tissues**

| | **PS, µM** | | | | | |
|---|---|---|---|---|---|---|
| **Tissue** | **0.5h** | **1h** | **2h** | **4h** | **8h** | **24h** |
| | Mean ± SEM | | | | | |
| Conjunctiva | 1.3 ± 0.1 | 1.8 ± 0.3 | 0.4 ± 0.1 | 6.1 ± 3.3 | 3.4 ± 1.4 | 0.5 ± 0.4 |
| Cornea | 1.8 ± 0.9 | 1.3 ± 0.2 | 1.4 ± 0.1 | 0.5 ± 0.1 | 1.1 ± 0.6 | 0.0 |
| Eyelid | 8.3 ± 2.2 | 14.2 ± 5.3 | 2.0 ± 0.5 | 3.5 ± 1.9 | 0.2 ± 0.2 | 1.8 ± 1.3 |
| AH | 0 | 0.0 | 0 | 0 | 0 | 0 |
| Vitreous | 0 | 0.0 | 0 | 0 | 0 | 0 |

**Table 23 - PK parameters of PS in rabbit ocular tissues**

| | **PS** | | | |
|---|---|---|---|---|
| **Tissue** | **C_{Max}, µM** | **T_{Max}, h** | **T_{1/2}, h** | **AUC₀₋₂₄ₕ, µM·h** |
| Conjunctiva | 5.8 | 4 | 5.7 | 58.6 ± 0.9 |
| Cornea | 1.8 | 0.5 | 23 | 6.4 ± 0.4 |
| Eyelid | 14.2 | 1 | 14.4 | 45.2 ± 0.5 |

Furthermore, the metabolism of PS in the cornea and conjunctiva was limited. Indeed, only low levels of sulindac, the main hydrolysis product of PS, were detected in the cornea and conjunctiva (see Figure 14B and Tables 24 and 25).

**Table 24 - Amount of sulindac (a metabolite of PS) in rabbit ocular tissues**

| **Sulindac, µM** | | | | | | |
|---|---|---|---|---|---|---|
| **Tissue** | **0.5h** | **1h** | **2h** | **4h** | **8h** | **24h** |
| | Mean ± SEM | | | | | |
| Conjunctiva | 0.1 ± 0.1 | 0.2 ± 0.1 | 0.0 | 0.1 ± 0.1 | 0.8 ± 0.5 | 0.0 |
| Cornea | 0.4 ± 0.2 | 0.6 ± 0.1 | 0.0 | 0.3 ± 0.1 | 0.2 ± 0.2 | 0.0 |
| Eyelid | 1.0 ± 0.1 | 3.6 ± 1.2 | 0.0 | 0.5 ± 0.2 | 0.0 | 0.0 |
| AH | 0.0 | 0 | 0.0 | 0 | 0 | 0 |
| Vitreous | 0.0 | 0 | 0.0 | 0.0 | 0 | 0 |

**Table 25 - PK parameters of sulindac (a metabolite of PS) in rabbit ocular tissues**

| | **Sulindac** | | | |
|---|---|---|---|---|
| **Tissue** | **C_{Max}, µM** | **T_{Max}, h** | **T_{1/2}, h** | **AUC₀₋₂₄ₕ, µM·h** |
| Conjunctiva | 0.8 | 8 | ND | 2.5 ± 0.3 |
| Cornea | 0.6 | 1 | 7.4 | 2.4 ± 0.5 |
| Eyelid | 3.6 | 1 | 1.1 | 5.5 ± 0.8 |

### Conclusions

The experiments herein demonstrate that, when administered to the outer surface of the eyelid, PS is able to penetrate the eyelid and reach the cornea in therapeutically relevant amounts. Therefore, topical administration of PS to the eyelid, which has significant benefits for patients, including avoiding stinging sensations experienced when using certain eyedrops and assisting patients with reduced motor activity, is a viable administration route for any eye disease or condition.

In light of the observations herein demonstrating the ability of PS to traverse peripheral neurons towards central sites of pain generation, without wishing to be bound by theory, PS is likely achieving its therapeutic activity on corneal neuropathic pain by traversing along corneal neurons towards central sites. In this experiment, PS is seen to penetrate the eyelid and reach the densely innervated cornea in therapeutically relevant levels. Without wishing to be bound by theory, by reaching and remaining stable at the cornea, the PS can trasverse along the centrally projecting corneal neurons towards central sites of action where it can impart its striking analgesic activity directly on neurons associated with central sensitization and generation of corneal neuropathic pain. Indeed, this is in line with the preliminary observations herein that PS effectively treats corneal neuropathic pain, even when administered to the outer surface of the eyelid.

### Example 27: Pharmacokinetics and biodistribution of PS upon oral administration

The Examples herein demonstrate a broad applicability of PS as an analgesic compound with direct nerve acting activity, even at central sites of action. The ability of topical PS to rapidly target even centrally generated pain is a reflection of its ability to traverse along peripheral nerves towards central sites involved in pain generation. The observations of the analgesic activity of orally administered PS in particularly challenging animal models of pain associated with central neuronal activity, led to a consideration of the potential site of action of orally administered PS. To the inventor's surprise, orally administered PS was seen to accumulate in therapeutically relevant amounts in the pain sensing centres of the brain, further pointing to a direct analgesic activity of PS on centrally located pain generating neurons.

### Methods

### Dosing and tissue harvesting

A single dose of PS was given by oral gavage to groups of three Sprague-Dawley rats, each weighing 200-300 g (Charles River Labs, Wilmington, MA), which were euthanized at the following time points via CO2 inhalation: 0.5, 1, 3 and 6 h post-dose. PS was dissolved in 10% ethanol and then suspended in corn oil.

Approximately 800 µL of blood were collected through cardiac puncture and immediately centrifuged.

Tissue samples were harvested using separate dissection instruments for each tissue sample to avoid cross contamination, and snap frozen. The levels of the test drug were determined by HPLC.

To determine the levels of PS in the wall of the stomach and duodenum, we harvested these two organs *in toto* last and after washing away their contents with PBS, three times and with 10% DMSO in PBS twice to remove gastric contents and residual PS that might be adsorbed onto the gastroduodenal mucosa. Control experiments revealed that a 10% solution of DMSO did not damage the mucosa and removed quantitatively any adherent PS following its oral administration.

As previously described (Wen et al, 2019), each plasma sample was mixed with double volume of acetonitrile and centrifuged at 13,200 rpm for 15 min. Tissue samples were weighed, ddH2O (100-300 µL, depending on tissue weight) was added, and they were homogenized. Following addition of acetonitrile (twice the volume of the homogenate), the mixture was sonicated for 10 min, centrifuged at 13,200 rpm for 15 min, and analyzed by HPLC, as reported (Wen et al, 2019). The lower limit of quantitation is 0.1 µM for PS and 0.05 µM for sulindac, sulindac sulfone, sulindac sulfide and their glucuronidated derivatives.

### HPLC analysis

It was performed following a previously reported method (Xie et al, 2012). Briefly, the HPLC system consisted of a Waters Alliance 2695 Separations Module (Milford, MA, USA) equipped with a Waters 2998 photodiode array detector (328 nm) and a Thermo Hypersil BDS C18 column (150 × 4.6 mm, particle size 3 µm). The mobile phase consisted of a gradient between solvent A (formic acid, CH3CN, H2O (0.1:4.9:95 v/v/v)) and solvent B (CH3CN) at a flow rate of 1 mL·min-1 at 30°C. We applied gradient elution from 30 to 100% solvent B from 0-6 min, and it was maintained at 100% solvent B until 8 min.

### Results

As foreshadowed above, the possibility that PS may reach the brain following oral administration was assessed. PS was administered to rats once orally and levels were determined in the stomach wall, the vagus nerve (the anterior and posterior trunks that innervate the stomach), the medulla (medulla oblongata, where the vagus originates), and the cerebellum. The medulla includes multiple nuclei and tracts that play a critical role in transmitting signals between the spinal cord and the higher parts of the brain, for example those involved in the sensation of pain. The vagus nerve has four nuclei in the medulla. The cerebellum lies posterior and partially superior to the medulla. All these tissues therefore represent an uninterrupted anatomical continuum.

As shown in Table 26, after the oral administration of PS (250 mg/kg), PS was detected in the gastric wall, the vagus nerve, the medulla and the cerebellum. No PS was detected in the systemic circulation, as expected (Xie et al, 2012).

The concentration of PS progressively decreased from the stomach to the cerebellum, evidenced by the respective values of Cₘₐₓ (from 661.0 ± 6.5 µM to 0.3 ± 0.07 µM). The Tₘₐₓ of PS was the same in all tissues (0.5 h), suggesting the transportation of PS to the CNS, via the vagus nerve, is rapid, as predicted from further observations herein. These data establish that that orally administered PS reaches the CNS from the stomach, and that its transportation to the CNS occurs via the vagus nerve and not through the circulation. This notion is supported by the progressive decrease of the Cₘₐₓ and AUC₀₋₂₄ₕ values of PS from the gastric wall to the cerebellum, and the absence of detectable levels of PS in the blood.

**Table 26 - PK parameters of PS in rat tissues and peripheral blood**

| **Tissue** | **Cₘₐₓ, µM** | **Tₘₐₓ, h** | **T_{1/2}, h** | **AUC₀₋₂₄ₕ, µM·h** |
|---|---|---|---|---|
| | *Mean* ± *SEM* | | | |
| Stomach | 661.0 ± 6.5 | 0.5 | 3.3 | 1275.7 |
| Vagus nerve | 1.2 ± 0.2 | 0.5 | 3.3 | 1.7 |
| Medulla | 0.5 ± 0.1 | 0.5 | -* | 0.3 |
| Cerebellum | 0.3 ± 0.07 | 0.5 | -* | 0.2 |
| Blood | -* | -* | -* | -* |

| | | | | |
|---|---|---|---|---|
| *, cannot be determined | | | | |

### Conclusions

In line with the observations herein demonstrating the ability of topical PS to traverse along peripheral neurons towards central sites, the present Example confirms that this ability is maintained even upon oral administration of PS. PS is seen preferentially binding to the stomach lining, where it is taken up by neurons connecting to the vagus nerve. The PS then traverses along the vagus nerve towards the brain, where it is found in therapeutically relevant amounts in the medulla and cerebellum.

In line with the observations with the sciatic nerve, the absence of PS in detectable levels in the blood, confirms that PS must be reaching distal sites of action via translocation along neurons. This unprecedented mechanism of action further explains how PS is able to achieve striking analgesic effects in multiple challenging animal models of neuropathic pain and pain associated with central sensitization. Without wishing to be bound by theory, the experiments confirm PS as a broadly applicable pain therapy with direct analgesic effects on neuronal pain signalling, even at sites of pain generation and sensation in the brain. As outlined elsewhere herein, the analgesic effects of PS are distinct to its anti-inflammatory activity - indeed, the centrally generated pain signalling is a consequence of pathological nerve activity (*e.g.,* due to central sensitization), rather than inflammation.

### Example 28: Summary of observations

The observations herein demonstrate an unprecedented analgesic activity of PS in treating pain associated with central sensitization. The observations herein of the ability of PS to treat allodynia (an established feature of central sensitization), in multiple distinct animal models of established chronic pain, generated via hyperexcitability of centrally located neurons, provide a broadly applicable role for PS as an analgesic, with central effects, for treating pain associated with central sensitization. This broad applicability is supported by observations herein that PS traverses peripheral neurons towards central sites of action known to be implicated in the generation and maintenance of pain associated with central sensitization. Such broad applicability can be predicted on the basis of the therapeutic efficacy of PS in multiple distinct models generating central sensitization. Furthermore, PS is shown herein to be effective in an animal model of migraine, exploiting administration of NTG, which is known to trigger central sensitization of centralised neurons. Furthermore, the observations herein demonstrate that PS achieves effective analgesia even upon administration to sites distal to the site of peripheral injury (*e.g.,* a contralateral paw), suggesting an activity of PS at central sites of action. Furthermore, surprisingly, PS demonstrates effective analgesia even upon oral administration in a number of complex pain models each having a distinct initial pathology. In support of observations regarding the biodistribution of PS upon topical administration, and the inability of PS to be maintained at detectable levels in the blood, these observations demonstrate that PS achieves its analgesic activity likely by accumulating at therapeutically relevant amounts in central sites, where it is able to impart its activity directly on pain generating neuronal signalling. This is confirmed by the biodistribution of PS upon oral administration.

The therapeutic effect of PS on mechanical allodynia is very strong and fairly rapid via topical administration. Indeed, upon topical administration, PS is shown to follow an ascending trajectory along peripheral neurons towards the spinal cord and can achieve a significant analgesic effect lasting up to two weeks. The topical route provides low systemic clearance, reduced drug interactions, increased patient tolerability, and facile combination with oral medications. PS may also be administered orally for treatment of pain associated with central sensitization. Indeed, surprisingly, oral administration of PS achieved analgesia in a number of challenging animal models establishing pain associated with central sensitization (manifested as allodynia).

These observations demonstrate a previously unrecognised activity and therapeutic utility of PS, a compound which falls within the broader class of NSAIDs but does not share all of the properties of this family of compounds. Indeed, unlike typical NSAIDs, PS does not inhibit activity of COX-1 or COX-2 and does not block the synthesis or activity of prostaglandins. In fact, PS has instead been shown to trigger increased COX activity and significantly increased PGE₂ levels (Mackenzie et al. (2010) Gastroenterology 139(4): 1320-32). In contrast to previous observations for NSAIDs regarding their inefficacy in treating pain associated with central sensitization, the data herein demonstrate that the activity of PS is more similar to analgesic agents which target neuronal activity directly, and with potential to act at central sites. Indeed, the results above confirm that PS can reduce pain from allodynia which is a known manifestation of central sensitization. In the chemotherapy-derived pain treatment model, pain was established for five days prior to treatment randomisation thus establishing central sensitization demonstrated by the allodynia (compared to the baseline). Similarly, the pain generated in the diabetes model was established for four weeks prior to treatment randomisation thus clearly establishing central sensitization demonstrated by the allodynia. Furthermore, the migraine pain model, exploiting NTG, is an established model for generating central sensitization. Therefore, without wishing to be bound by theory, PS is having a direct effect on neuronal pain signalling generated by central sensitization, and acts in a similar way to the mechanism of action of established anaesthetics. Indeed, the results show the ability of PS to reduce pain signalling from central sensitization, implicating central sites of action for the analgesic activity of this compound. This is confirmed by further observations herein with respect to the analgesic activity of PS when administered at distal sites (*i.e.,* a contralateral paw) or even orally, indicating, without wishing to be bound by theory, that PS is likely to act directly on neuronal activity and at central sites of pain generation. Of course, this activity is distinct from the established role of PS, and typical NSAIDs, as anti-inflammatory agents.

Earlier observations regarding the activity of PS are limited to its anti-inflammatory activity. For example, WO 2019/067919 suggests a role for PS in the treatment of DED, using an acute DED model, in which concanavalin A (ConA) is administered to rabbit lacrimal glands concurrently with PS. In this context, the anti-inflammatory activity of PS results in a limited inflammatory response to ConA, thus preventing the establishment of DED. These observations confirm the anti-inflammatory activity of PS and suggest its utility in preventing establishment and maintenance of inflammatory components of DED. The observations in this acute DED model fail to provide any evidence of the ability of PS to act directly on nerves to reduce nerve signalling caused by pain central sensitization. Any reduction in pain in this acute DED model can be assumed only to be a consequence of PS inhibiting the inflammatory response (*i.e.,* the pathology responsible for triggering activation of the pain sensors). In addition to indicating the anti-inflammatory activity of PS in DED, the authors of WO 2019/067919 suggest that PS decreases corneal sensitivity in an acute corneal sensation model. These observations of alleged reduced sensitivity to an acute stimulus, which clearly does not generate persistent pain (*i.e.,* sensation occurring in the absence of the peripheral stimulus), do not suggest the ability of PS to treat pain associated with central sensitization (*i.e.*, generated in central sites of action). Indeed, the effect of PS in this acute model was observed immediately, implicating local action of PS, not dissimilar to the activity observed for the typical NSAID, ketorolac, in the same model. Such peripheral activity does not demonstrate an efficacy of PS in treating pain generated at central sites of action. Indeed, clinical guidance in the field recommends avoiding the use of NSAIDs for the treatment of types of pain associated with central sensitization (*e.g.*, neuropathic pain), and ketorolac has been shown to have limited analgesic activity in models of such pain. Furthermore, the results of the DED model suggest that PS restores suppressed ocular sensitivity, implicating a role for PS in increasing rather than reducing nociception.

Similar to these earlier observations in acute models, PS has been shown to reduce sensitivity in an acute model of nociceptive pain (WO 2013/130625 and WO 2014/047592). The hot plate assay used in these experiments generates nociceptive signalling in response to an acute stimulus and fails to generate pain associated with central sensitization. Furthermore, PS was pre-administered in these experiments. Accordingly, contrary to the observations herein, these acute experiments fail to indicate an ability of PS to reach sites of action associated with centrally generated pain and certainly fail to demonstrate an ability of PS to treat pain associated with central sensitization.

Therefore, irrespective of any suggestion towards a peripheral analgesic activity of PS, such activity observed in acute models, provides no indication of a corresponding activity in the treatment of pain associated with central sensitiation.

The experiments herein were performed in specific animal models in which central sensitization, manifested as allodynia, has been established, and demonstrate a surprising therapeutic efficacy of PS in treating pain generated centrally. As outlined above, specific animal models are important during the development of therapies for treating pain associated with central sensitization. Indeed, given the pathogenesis of such pain, involving alterations in the sensitivity of centrally located neurons, observations of efficacy of a particular compound, for example, in an acute pain model cannot indicate the utility of that compound in treating pain associated with central sensitization. Accordingly, the efficacy of a compound in treating pain associated with central sensitization is demonstrated by observations indicating the ability of the compound to reverse manifestations of central sensitization (*e.g.,* allodynia) in a model system in which chronic pain has been established. In this way, the animal models used in early testing before further clinical development are important. Based on the specific animal models of pain generated via sensitization of central neurons (*e.g*., allodynia), the observations herein demonstrate an unprecedented efficacy of PS in the treatment of pain associated with central sensitization.

The observations herein demonstrate that PS has therapeutic utility beyond an anti-inflammatory activity associated with typical NSAIDs. In contrast, certain NSAIDs are ineffective analgesics in treating pain associated with central sensitization (*e.g.*, due to neuropathic pain). Indeed, Moore et al. (Cochrane Database of Systematic Reviews (2015); 10: 1-25), outlined that NSAIDs have no therapeutic efficacy in pain disorders associated with central sensitization (*i.e.,* neuropathic pain). The activity of PS observed herein, corresponding to that of pregabalin and lidocaine, contrasts with the inability of typical NSAIDs to provide a direct analgesic effect on hypersensitized neurons involved in the generation of pain associated with central sensitization. The distinct activity of PS compared to typical NSAIDs is confirmed by the comparison with its parent compound, sulindac, in the experiments above. Sulindac failed to reduce established allodynia, indicating that, unlike PS, sulindac does not provide a direct analgesic effect on pain associated with central sensitization. Without wishing to be bound by theory, the reason for the absence of any response to typical NSAIDs in the prior art is likely that the pain is caused by central sensitization, rather than by inflammation (*i.e.,* any anti-inflammatory activity of typical NSAIDs is not sufficient to prevent or treat pain associated with central sensitization). Accordingly, the analgesic activity of PS observed herein is unique and not shared by typical NSAIDs. Based on the observations herein for sulindac, any alleged analgesic activity of NSAIDs observed in the prior art is a reflection of their anti-inflammatory activity (*i.e.,* stopping the initial triggers causing the pain) rather than an actual analgesic activity directed towards nerve signalling. For example, WO 2008/014066 implicates sulindac in treating pain associated with a chronic constriction injury model, but any alleged effect is concluded to be due to the reduction of pro-inflammatory cytokines, rather than an effect on pain signalling pathways *per se.* Indeed, if typical NSAIDs, for example sulindac, were capable of acting directly on neurons with analgesic activity, then sulindac would have been expected to reduce the allodynia observed in the model above. Irrespective, any suggestions that typical NSAIDs have analgesic activity are associated with a requirement for inhibition of the COX pathways and synthesis of prostaglandins (*i.e.,* activities of typical NSAIDs that are not shared by PS).

Therefore, the present inventor has demonstrated a new surprising activity for PS in the treatment and/or prevention of pain associated with central sensitization. As outlined above, this activity goes beyond the anti-inflammatory activity previously observed for PS and related NSAIDs. In fact, unlike typical NSAIDs, the observations herein demonstrate that PS has a direct activity on nerves implicated in persistent pain generated by central sensitization, corresponding to the site and mechanism of action of established analgesics, such as lidocaine and pregabalin. Furthermore, the ease by which PS can be administered, for example topically, and its limited adverse effects (Mackenzie et al. (2010) Gastroenterology 139(4): 1320-32) render it an improved therapy for pain associated with central sensitization compared even to established centrally acting analgesics. Furthermore, the unprecedented ability of PS to target central sites of action, even upon oral administration, render it a particularly useful analgesic for a broad range of indications having a component of central sensitization.

As noted herein, specific animal models are important during the development of therapies for treating neuropathic pain. Indeed, given the pathogenesis of pain associated with peripheral neuropathy, observations of efficacy of a particular compound in an alternative pain model cannot indicate the utility of that compound in treating neuropathic pain. In line with this, typically, it is not possible to extrapolate the use of effective drugs from other forms of neuropathic pain to the neuropathic pain of particular interest, even if the clinical syndrome is similar. For example, gabapentin shows different efficacy in the treatment of different forms of neuropathic pain. Accordingly, the animal model used in early testing before further clinical development is crucial. However, the inventor's observations herein demonstrate, for the first time, that the analgesic activity of PS, is broadly applicable. This is demonstrated via the efficacy of PS in treating multiple neuropathic pain indications, confirmed in multiple specific animal models. These observations enabled the inventor to rationalise that the accumulation of PS in central sites of action may impart a general analgesic activity that is effective irrespective of the peripheral pathogenesis of the pain. Strikingly, this activity is achieved not only via topical administration to peripheral neurons, but PS is able to accumulate at central sites of pain generation even upon oral administration.

It will be understood that the inventor's work has been described above by way of example only and modifications may be made while remaining within the scope and spirit of the invention.

The invention further provides the following further numbered embodiments.
1. A method of treating and/or preventing neuropathic pain associated with post-traumatic peripheral neuropathy (PTPN) comprising administering a therapeutically effective amount of phosphosulindac (PS) to a subject in need thereof such that neuropathic pain associated with PTPN is treated and/or prevented.
2. The method of embodiment 1, wherein treating the neuropathic pain comprises reducing the neuropathic pain.
3. The method of embodiment 1 or 2, wherein preventing the neuropathic pain comprises decreasing the incidence of the neuropathic pain.
4. The method of any one of the preceding embodiments, wherein treating the neuropathic pain includes reducing one or more of the sensory symptoms associated with PTPN.
5. The method of any one of the preceding embodiments, wherein preventing the neuropathic pain includes decreasing the incidence of one or more of the sensory symptoms associated with PTPN.
6. The method of embodiment 4 or 5, wherein the one or more sensory symptom is selected from paresthesia, burning sensations and stabbing sensations.
7. The method of embodiment 6, wherein the paresthesia includes one or more of numbness, tingling, pricking, or formication.
8. The method of any one of the preceding embodiments, wherein PS reduces the neuronal signalling involved in the sensation of pain.
9. The method of any one of the preceding embodiments, wherein PS reduces pain generated via peripheral sensitization.
10. The method of any one of the preceding embodiments, wherein PS reduces pain generated via central sensitization.
11. The method of any one of the preceding embodiments, wherein PS reduces pain signalling occurring centrally.
12. The method of any one of the preceding embodiments, wherein the PS reduces pain signalling occurring in peripheral nerves.
13. The method of any one of the preceding embodiments, wherein PS reduces pain signalling occurring in the dorsal root ganglion.
14. The method of any one of the preceding embodiments, wherein PS reduces pain signalling occurring in the spinal cord dorsal horn.
15. The method of any one of the preceding embodiments, wherein the neuropathic pain is allodynia.
16. The method of embodiment 15, wherein the allodynia is mechanical allodynia and/or thermal allodynia.
17. The method of any one of the preceding embodiments, wherein the neuropathic pain is hyperalgesia.
18. The method of any one of the preceding embodiments, wherein the neuropathic pain associated with PTPN is caused by neurapraxia, for example a nerve compression injury.
19. The method of any one of the preceding embodiments, wherein the neuropathic pain associated with PTPN is caused by axonotmesis, for example a nerve crush injury.
20. The method of any one of the preceding embodiments, wherein the neuropathic pain associated with PTPN is caused by one or more of the following: carpal tunnel syndrome; pronator teres syndrome; radial tunnel syndrome; suprascapular nerve entrapment; thoracic outlet syndrome; ulnar nerve entrapment (cubital tunnel syndrome or Guyon's canal syndrome); meralgia paresthetica; peroneal nerve compression; pudendal nerve entrapment syndrome; sciatica; tarsal tunnel syndrome; herniated cervical disc; herniated thoracic disc; and/or herniated lumbar disc.
21. The method of any one of the preceding embodiments, wherein the subject is human.
22. The method of any one of the preceding embodiments, wherein PS has the formula I (PS-I): 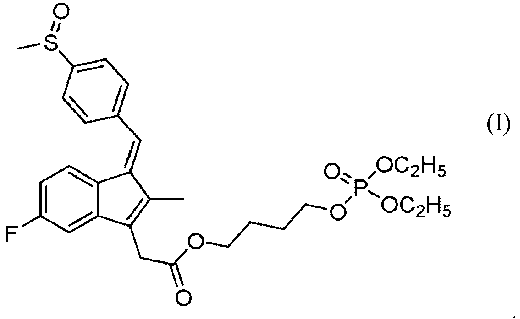
23. The method of any one of the preceding embodiments, wherein PS has the formula II (PS-II): 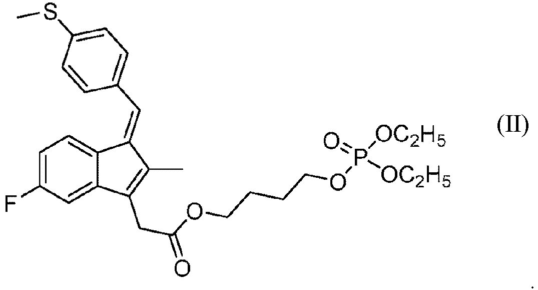
24. The method of any one of the preceding embodiments, wherein the therapeutically effective amount of PS is administered as a pharmaceutical composition further comprising a pharmaceutically acceptable excipient.
25. The method of embodiment 24, wherein the pharmaceutical composition comprising PS is formulated for topical administration.
26. The method of embodiment 25, wherein the pharmaceutical composition comprising PS is formulated as a semi-solid.
27. The method of embodiment 25, wherein the pharmaceutical composition comprising PS is formulated as a liquid.
28. The method of embodiment 25-27, wherein the pharmaceutical composition comprising PS is a cream.
29. The method of embodiment 25-27, wherein the pharmaceutical composition comprising PS is a gel, for example wherein the gel is a hydrogel.
30. The method of embodiment 25-27, wherein the pharmaceutical composition comprising PS is a lotion.
31. The method of embodiment 25-27, wherein the pharmaceutical composition comprising PS is an ointment.
32. The method of embodiment 25-27, wherein the pharmaceutical composition comprising PS is a spray.
33. The method of embodiment 25, wherein the pharmaceutical composition comprising PS is formulated as a patch.
34. The method of any one of embodiments 24-33, wherein the pharmaceutical composition comprises PS at a concentration of about 0.5% to about 15% w/w of the pharmaceutical composition.
35. The method of embodiment 34, wherein the pharmaceutical composition comprises PS is at a concentration of about 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition.
36. The method of embodiment 35, wherein the pharmaceutical composition comprises PS at a concentration of less than or equal to 8% w/w of the pharmaceutical composition, for example about 5% or about 3% w/w of the pharmaceutical composition.
37. The method of embodiment 36, wherein the pharmaceutical composition comprises PS at a concentration of less than or about equal to 3% w/w of the pharmaceutical composition, for example about 2% or about 1% w/w of the pharmaceutical composition.
38. The method of any of embodiments 24-37, wherein the PS is administered at about 0.005 g/10cm² to about 0.25 g/10cm² of affected area.
39. The method of embodiment 38, wherein the PS is administered at about 0.005 g/10cm² of affected area.
40. The method of embodiment 38, wherein the PS is administered at about 0.01 g/10cm² of affected area.
41. The method of embodiment 38, wherein the PS is administered at about 0.05 g/10cm² of affected area.
42. The method of embodiment 38, wherein the PS is administered at about 0.1 g/10cm² of affected area.
43. The method of embodiment 38, wherein the PS is administered at about 0.15 g/10cm² of affected area.
44. The method of embodiment 38, wherein the PS is administered at about 0.2 g/10cm² of affected area.
45. The method of embodiment 38, wherein the PS is administered at about 0.25 g/10cm² of affected area.
46. The method of any one of embodiments 24-45, wherein the PS is applied to the affected area and left on the affected area for between about 1 hour and about 5 hours.
47. The method of embodiment 46, wherein the PS is applied to the affected area and left on the affected area for about 0.5 hours, for about 1 hour, for about 2 hours, for about 3 hours, for about 4 hours, or for about 5 hours.
48. The method of embodiment 46 or 47, wherein the PS is removed from the affected area after the dosing period, for example by washing off.
49. The method of embodiment 46 or 47, wherein a second or further application of PS is applied to the affected area after the dosing period.
50. The method of any one of embodiments 24-49, wherein the PS is applied once a day.
51. The method of any one of embodiments 24-49, wherein the PS is applied twice a day.
52. The method of any one of embodiments 24-49, wherein the PS is applied three times a day.
53. The method of any one of embodiments 24-49, wherein the PS is applied four times a day.
54. The method of any one of embodiments 38-53, wherein the PS is administered in a pharmaceutical composition.
55. The method of any one of embodiments 1-24, wherein the PS is administered orally.
56. The method of embodiment 55, wherein the PS is formulated as a liquid or solid dosage form.
57. The method of embodiment 56, wherein the liquid dosage form is a pharmaceutically acceptable emulsion, microemulsion, solution, suspension, syrup or and elixir.
58. The method of embodiment 56, wherein the solid dosage form is a capsule, tablet, pill, powder, or granule.
59. The method of any one of embodiments 55-58, wherein the PS is administered orally at dosage levels of about 0.01 mg/kg to about 100 mg/kg, from about 0.05 mg/kg to about 50 mg/kg, or from about 0.1 mg/kg to about 10 mg/kg of subject body weight, for example about 1 mg/kg to about 5 mg/kg, for example about 3 mg/kg of subject body weight.
60. The method of any one of embodiments 55-58, wherein the PS is administered orally at a dosage of about 1 mg to about 2000 mg, of about 100 mg to 1500 mg, of about 200 mg to about 100 mg, of about 50 mg to about 400 mg, for example about 100 mg to about 350 mg, for example about 150 mg to about 300 mg, for example about 150mg to about 250 mg.
61. The method of any one of embodiments 55-60, wherein the PS is administered orally at a dosage of about 250 mg to about 300 mg, preferably about 250 mg.
62. The method of any one of embodiments 55-61, wherein the PS is administered orally once a day.
63. The method of any one of embodiments 55-62, wherein the PS is administered orally at least twice a day, at least three times a day, or at least four times a day.
64. The method of embodiment 63, wherein the PS is administered orally two or three times a day.
65. The method of any one of embodiments 55-64, wherein the PS is administered orally at a dosage of from about 150 mg to about 200 mg twice a day.
66. The method of any one of embodiments 55-65, wherein the PS is administered orally at a daily dosage of about 300 mg to about 400 mg.
67. The method of any one of embodiments 55-66, wherein the PS is administered orally at a daily dosage of about 250 mg to about 300 mg (for example, about 250 mg) two or three times a day.
68. The method of any one of embodiments 55-67, wherein the PS is administered orally at a daily dosage of about 500 mg to up to about 900 mg a day.
69. The method of any one of embodiments 55-68, wherein the PS is administered orally in a pharmaceutical composition.
70. PS for use in treating and/or preventing neuropathic pain associated with PTPN.
71. PS for use according to embodiment 70, wherein PS is administered by the method of any one of embodiments 1 to 65.
72. Use of PS for the manufacture of a medicament for treating and/or preventing neuropathic pain associated with PTPN.
73. The use of PS according to embodiment 72, wherein PS is administered by the method of any one of embodiments 1 to 65.

## Claims

1. Phosphosulindac (PS) for use in treating and/or preventing migraine pain in a subject.

2. PS for use of claim 1, wherein:
(i) treating the pain comprises reducing the pain, for example pulsating head pain;
(ii) preventing the pain comprises decreasing the incidence of the pain, for example pulsating head pain;
(iii) treating the pain includes reducing one or more of the symptoms associated with migraine; and/or
(iv) preventing the pain includes decreasing the incidence of one or more of the symptoms associated with migraine,
optionally wherein the one or more symptom is selected from aura, nausea, vomiting, photophobia, phonophobia and/or cranial autonomic symptoms, such as wherein:
(a) the aura includes one or more sensory disturbances, for example visual symptoms, pins and needles (tingling), and/or numbness; and/or
(b) the cranial autonomic symptom is eye redness and/or tearing.

3. PS for use of claim 1 or claim 2, wherein:
(i) the subject experiences cutaneous allodynia;
(ii) PS reduces the neuronal signalling involved in the sensation of pain;
(iii) PS reduces pain generated via peripheral sensitisation;
(iv) PS reduces pain generated via central sensitisation;
(v) PS reduces pain signalling occurring centrally;
(vi) PS reduces pain signalling occurring in the trigeminal nerve;
(vii) PS reduces pain signalling occurring in the trigeminal ganglion;
(viii) PS reduces pain signalling occurring in the trigeminal nucleus caudalis; and/or
(ix) PS reduces pain signalling occurring in higher order neurons and/or pain sensing regions of the brain, for example trigeminothalamic neurons.

4. PS for use of any one of claims 1-3, wherein:
(i) the pain is allodynia, for example cutaneous allodynia, optionally wherein the allodynia is mechanical allodynia and/or thermal allodynia;
(ii) the pain is hyperalgesia;
(iii) the migraine is episodic migraine or chronic migraine; and/or
(iv) the migraine is migraine with aura or migraine without aura.

5. PS for use of any one of claims 1-4, wherein the subject is human.

6. PS for use of any one of claims 1-5, wherein:
(i) PS has the formula I (PS-I): 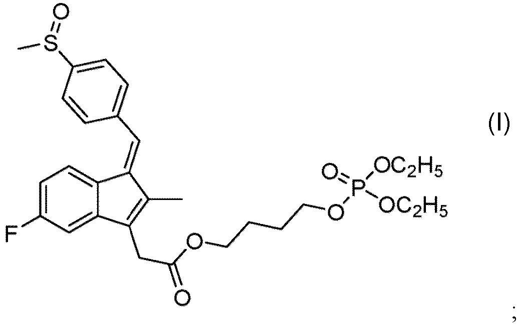 or
(ii) PS has the formula II (PS-II):

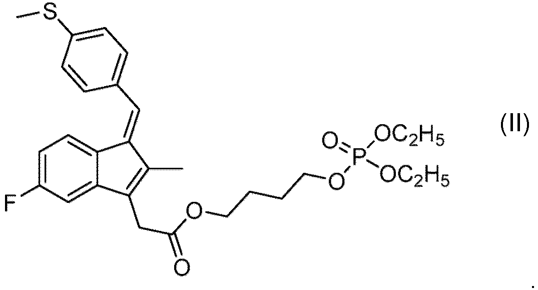

7. PS for use of any one of claims 1-6, wherein the therapeutically effective amount of PS is administered as a pharmaceutical composition further comprising a pharmaceutically acceptable excipient.

8. PS for use of claim 7, wherein the pharmaceutical composition comprising PS is formulated for topical administration, optionally wherein:
(i) the pharmaceutical composition comprising PS is formulated as a semi-solid or a liquid, optionally wherein the pharmaceutical composition comprising PS is:
(a) a cream;
(b) a gel, for example wherein the gel is a hydrogel;
(c) a lotion;
(d) an ointment; or
(e) a spray;
or
(ii) the pharmaceutical composition comprising PS is formulated as a patch.

9. PS for use of claim 7 or claim 8, wherein the pharmaceutical composition comprises PS at a concentration of about 0.5% to about 15% w/w of the pharmaceutical composition, optionally wherein the pharmaceutical composition comprises PS at a concentration of about 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% w/w of the pharmaceutical composition, such as wherein the pharmaceutical composition comprises PS at a concentration of:
(i) less than or equal to 8% w/w of the pharmaceutical composition, for example about 5% or about 3% w/w of the pharmaceutical composition; or
(ii) less than or about equal to 3% w/w of the pharmaceutical composition, for example about 2% or about 1% w/w of the pharmaceutical composition.

10. PS for use of any one of claims 7-9, wherein the PS is administered at about 0.005 g/10cm² to about 0.25 g/10cm² of affected area, optionally wherein the PS is administered at:
(i) about 0.005 g/10cm² of affected area;
(ii) about 0.01 g/10cm² of affected area;
(iii) about 0.05 g/10cm² of affected area;
(iv) about 0.1 g/10cm² of affected area;
(v) about 0.15 g/10cm² of affected area;
(vi) about 0.2 g/10cm² of affected area; or
(vii) about 0.25 g/10cm² of affected area.

11. PS for use of any one of claims 7-10, wherein the PS is applied to the affected area and left on the affected area for between about 1 hour and about 5 hours, optionally wherein:
(i) the PS is applied to the affected area and left on the affected area for about 0.5 hours, for about 1 hour, for about 2 hours, for about 3 hours, for about 4 hours, or for about 5 hours;
(ii) the PS is removed from the affected area after the dosing period, for example by washing off, or a second or further application of PS is applied to the affected area after the dosing period; and/or
(iii) the PS is applied:
(a) once a day;
(b) twice a day;
(c) three times a day; or
(d) four times a day.

12. PS for use of claim 7, wherein the pharmaceutical composition comprising PS is formulated for transdermal administration or injection, for example subcutaneous injection.

13. PS for use of any one of claims 1-7, wherein the PS is administered orally.

14. PS for use of claim 13, wherein the PS is formulated as a liquid or solid dosage form, optionally wherein:
(i) the liquid dosage form is a pharmaceutically acceptable emulsion, microemulsion, solution, suspension, syrup or and elixir; or
(ii) the solid dosage form is a capsule, tablet, pill, powder, or granule.

15. PS for use of claim 13 or claim 14, wherein:
(i) the PS is administered orally at dosage levels of about 0.01 mg/kg to about 100 mg/kg, from about 0.05 mg/kg to about 50 mg/kg, or from about 0.1 mg/kg to about 10 mg/kg of subject body weight, for example about 1 mg/kg to about 5 mg/kg, for example about 3 mg/kg of subject body weight;
(ii) the PS is administered orally at a dosage of about 1 mg to about 2000 mg, of about 100 mg to 1500 mg, of about 200 mg to about 100 mg, of about 50 mg to about 400 mg, for example about 100 mg to about 350 mg, for example about 150 mg to about 300 mg, for example about 150mg to about 250 mg;
(iii) the PS is administered orally at a dosage of about 250 mg to about 300 mg, preferably about 250 mg;
(iv) the PS is administered orally once a day;
(v) the PS is administered orally at least twice a day, at least three times a day, or at least four times a day;
(vi) the PS is administered orally two or three times a day;
(vii) the PS is administered orally at a dosage of from about 150 mg to about 200 mg twice a day;
(viii) the PS is administered orally at a daily dosage of about 300 mg to about 400 mg;
(ix) the PS is administered orally at a daily dosage of about 250 mg to about 300 mg (for example, about 250 mg) two or three times a day; and/or
(x) the PS is administered orally at a daily dosage of about 500 mg to up to about 900 mg a day.
